# EUROPEAN PATENT APPLICATION

(11) **EP 1 557 670 A1**
(43) Date of publication of application: **27.07.2005**
(21) Application number: 03809869.5
(22) Date of filing: 30.10.2003
(51) Int. Cl.: G01N 33/50, G01N 33/15, G01N 33/566, A01K 67/027, A61K 31/44, A61K 45/00, A61K 38/17, A61K 48/00, A61P 29/00, A61P 35/00, A61P 37/02, A61P 37/06, C07K 16/18, C12N 15/00

(54) **REGULATION OF INTERACTION BETWEEN RAPL AND Rap1**

(30) Priority: 30.10.2002 JP 2002316892
(71) Applicant: ISHIHARA SANGYO KAISHA, LTD., Nishi-ku, Osaka 550-0002 (JP)
(72) Inventor: KINASHI, Tatsuo, Kashihara-shi, Nara 634-0073 (JP); SHIKAMA, Hiroshi c/o Ishihara Sangyo Kaisha, Ltd., Kusatsu-shi, Shiga 525-0025 (JP)
(74) Representative: von Kreisler Selting Werner
(86) International application number: PCT/JP2003/013937
(87) International publication number: WO 2004/040302

(57) **Abstract**

A functional failure of Rap1 as a molecule that regulates integrin adhesion is believed to be related to pathology of inflammation, allergy, autoimmune diseases, cancer immunity, transplantation immunity, and the like that are immune diseases, and therefore elucidation of the mechanism of regulation of integrin adhesion by Rap1 leads to an understanding of the pathology of these immune diseases and the development of their treatment methods. As a molecule that is involved in the regulation of integrin adhesion by Rap1 p30 has been identified, and it was found that the p30 regulates the Rap1 function by binding to it. The use of this knowledge can be used for developing a p30 and Rap1 binding inhibitor and the like, developing drugs for inflammation, allergy, autoimmune diseases, cancer immunity, transplantation immunity, and the like, and further elucidating their regulation mechanisms.

## Description

### FIELD OF THE INVENTION

The present invention relates to regulation of bioactivities induced by interaction with p30 (that is, RAPL). The present invention relates to techniques that allow regulation, e.g. inhibition or promotion, of Rap1-p30 binding and application techniques thereof.

### BACKGROUND OF THE INVENTION

Although a low molecular weight G protein Rap1 has been reported so far to have a function as an antagonist of H-Ras, it has recently become apparent that the protein is an intracellular regulatory molecule of the adhesion molecule integrin. In the immune system, Rap1 positively regulates adhesiveness of b2 integrin such as LFA-1 expressed in immune cells and exerts important effects on adhesion between white blood cells and vascular endothelial cells, migration and localization of these cells in tissues, and adhesion to antigen presenting cells. Functional failure of Rap1 as a molecule of integrin adhesive regulation is presumed to be closely connected to immune disease states such as inflammation, allergy, autoimmune disease, tumor immunity, and transplantation immunity.

In addition, there is a report that p30 has been identified as a molecule to participate in integrin adhesive regulation by Rap1 (Non-patent Document 1).
[Non-patent document No. 1] The 31st Annual Meeting of the Japanese Society for Immunology (Dec. 11-13, 2001, Osaka International Convention Center), and its proceedings (issued Oct. 31, 2001))

### SUMMARY OF THE INVENTION

Elucidation of regulatory mechanisms of integrin-mediated adhesion by Rap1 can lead to understanding of these immune disease states and development of their treatment methods.

The present inventors have conducted an extensive research. As a result, the present inventors have succeeded in uncovering that p30 (i.e. RAPL) functions as an effector molecule for Rap1 and that p30 is a molecule to positively regulate adhesion and migration by LAF-1. The present invention has been achieved as a result of further study on the basis of this knowledge. Hereinafter, p30 indicates RAPL.

The present invention provides the following:
[1] A screening method for a compound, or a salt thereof, that promotes or inhibits the interaction and/or the binding between Rap1 and p30 which comprises:
   (1) a process to allow
      (a) a polypeptide selected from the group consisting of active-form polypeptides each containing an amino acid sequence identical or essentially identical to the amino acid sequence of SEQ ID NO:2, active-form polypeptides each containing a point-mutated SEQ ID NO:2 amino acid sequence in which the 12th glycine thereof is replaced with valine or active-form polypeptides each containing an amino acid sequence essentially identical to said point-mutated amino acid sequence, partial peptides thereof, and salts thereof,
      (b) a polypeptide selected from the group consisting of polypeptides each containing an amino acid sequence identical or essentially identical to the amino acid sequence represented by SEQ ID NO:4, partial peptides thereof, and salts thereof, and
      (c) a test sample to come in contact with one another;
   (2) a process to detect the interaction and/or binding between the polypeptide selected from the group (a) and the polypeptide selected from the group (b).
[2] The screening method according to the above [1] comprising
   (1) the process to allow a polypeptide selected from the group (a), a polypeptide selected from the group (b), and a test sample to come in contact with one another,
   (2) the process to detect the interaction and/or binding between the polypeptide selected from the group (a) and the polypeptide selected from the group (b), and
   (3) a process to select a compound that promotes or inhibits the interaction and/or binding between these polypeptides.
[3] The screening method according to the above [1] or [2] in which another peptide is fused to a polypeptide selected from the group (a) and/or a polypeptide selected from the group (b).
[4] The screening method according to any one of the above [1] to [3] in which the polypeptide selected from the group (a) and/or the polypeptide selected from the group (b) is labeled and the label is detected or measured to detect the binding and/or interaction between the polypeptides.
[5] The screening method according to any one of the above [1] to [3] in which the polypeptide of the group (b) bound to the polypeptide of the group (a) is assayed (detected or measured) with a primary antibody against the polypeptide of the group (b) or a primary antibody against another peptide fused to the polypeptide of the group (b) to detect the binding and/or interaction between the polypeptide selected from the group (a) and the polypeptide selected from the group (b).
[6] The screening method according to any one of the above [1] to [3] in which the polypeptide selected from the group (a) bound to the polypeptide selected from the group (b) is assayed (detected or measured) with a primary antibody against the polypeptide of the group (a) or a primary antibody against another peptide fused to the polypeptide of the group (a) to detect the binding and/or interaction between the polypeptide selected from the group (a) and the polypeptide selected from the group (b).
[7] The screening method according to any one of the above [1] to [3] in which the polypeptide selected from the group (b) bound to the polypeptide selected from the group (a) is assayed (detected or measured) with a primary antibody against the polypeptide of the group (b) or a primary antibody against another peptide fused to the polypeptide of the group (b), and a secondary antibody against the primary antibody to detect the binding and/or interaction between the polypeptide selected from the group (a) and the polypeptide selected from the group (b).
[8] The screening method according to any one of the above [1] to [3] in which
   the polypeptide of the group (a) is an active fusion polypeptide, or a salt thereof, wherein a polypeptide having the amino acid sequence of SEQ ID NO:2 is fused with glutathione-S-transferase at the N-terminal side thereof, or an acitive fusion polypeptide, or a salt thereof, wherein glutathione-S-transferase is fused with the N-terminal side of a polypeptide having a point-mutated SEQ ID NO:2 amino acid sequence in which the 12th glycine thereof is replaced with valine and
   the polypeptide of the group (b) is a polypeptide, or a salt thereof, wherein an Myc epitope is fused with the N-terminal side of a polypeptide having the amino acid sequence of SEQ ID NO:4.
[9] A screening kit for a compound, or a salt thereof, which promotes or inhibits the interaction and/or binding between Rap1 and p30 which comprises an effective amount of
   (a) a polypeptide selected from the group consisting of polypeptides each containing an amino acid sequence identical or essentially identical to the amino acid sequence of SEQ ID NO:2, polypeptides each containing a point-mutated SEQ ID NO:2 amino acid sequence in which the 12th glycine thereof is replaced with valine, or an amino acid sequence essentially identical to said point-mutated amino acid sequence, partial peptides thereof, and salts thereof, and
   (b) a polypeptide selected from the group consisting of polypeptides each containing an amino acid sequence identical or essentially identical to the amino acid sequence of SEQ ID NO:4, partial peptides thereof, and salts thereof.
[10] The screening kit according to the above [9] in which another peptide is fused to the polypeptide selected from the group (a) and/or the polypeptide selected from the group (b).
[11] The screening kit according to the above [9] in which the polypeptide selected from the group (a) and/or the polypeptide selected from the group (b) is labeled.
[12] The screening kit according to the above [9] in which
   the polypeptide of the group (a) is a fusion polypeptide, or a salt thereof, wherein glutathione-S-transferase is fused with the N-terminal side of a polypeptide having the amino acid sequence of SEQ ID NO:2 or a fusion polypeptide, or a salt thereof, wherein glutathione-S-transferase is fused with the N-terminal side of a polypeptide having a point-mutated SEQ ID NO:2 amino acid sequence in which the 12th glycine thereof is replaced with valine and
   the polypeptide of the group (b) is a fusion polypeptide, or a salt thereof, wherein an Myc epitope is fused with the N-terminal side of a polypeptide having the amino acid sequence of SEQ ID NO:4;
[13] A compound, or a salt thereof, which promotes or inhibits the interaction and/or binding between Rap1 and p30 and is obtained using the screening method according to the above [1] or the screening kit according to the above [9] ;
[14] The compound, or the salt thereof, which inhibits the interaction and/or binding between Rap1 and p30 according to the above [13] ;
[15] A pharmaceutical composition comprising an effective amount of the compound, or a salt thereof, according to the above [13] ;
[16] A pharmaceutical composition comprising an effective amount of the compound, or a salt thereof, according to the above [14] ;
[17] The pharmaceutical composition according to the above [15] or [16] in which a target to be treated or prevented is selected from the group consisting of:
   (a) inflammatory diseases;
   (b) immune diseases;
   (c) graft versus host reaction upon organ transplantation; and
   (d) cancers;
[18] A monoclonal antibody that recognizes a polypeptide containing an amino acid sequence identical or essentially identical to the amino acid sequence of SEQ ID NO:4;
[19] A diagnostic method which comprises using the monoclonal antibody according to the above [18] ;
[20] A diagnostic kit which comprises an effective amount of the monoclonal antibody according to the above [18] ;
[21] A polypeptide, or a salt thereof, that intracellularly functions against a polypeptide containing an amino acid sequence identical or essentially identical to the amino acid sequence of SEQ ID NO:4 in a dominantly negative fashion;
[22] A composition comprising an effective amount of the polypeptide, or a salt thereof, according to the above [21] for treatment or prevention of a disease selected from the group consisting of:
   (a) inflammatory diseases;
   (b) immune diseases;
   (c) graft rejection in organ transplantation (or graft versus host reaction upon organ transplantation); and
   (d) cancers;
[23] A polynucleotide encoding the polypeptide according to the above [21] ;
[24] A composition comprising an effective amount of the polynucleotide according to the above [23] for treatment or prevention of a disease selected from the group consisting of:
   (a) inflammatory diseases;
   (b) immune diseases;
   (c) graft versus host reaction upon organ transplantation; and
   (d) cancers;
[25] A transgenic animal having a regulated expression of a polypeptide containing an amino acid sequence identical or essentially identical to the amino acid sequence of SEQ ID NO:10;
[26] The transgenic animal according to the above [25] wherein a polypeptide containing an amino acid sequence identical or essentially identical to the amino acid sequence of SEQ ID NO:10 is overexpressed;
[27] The transgenic animal according to the above [25] or [26] which is a mouse;
[28] A Rap1-p30 binding inhibitor which comprises an effective amount of a compound, or a salt thereof, of the formula (1): 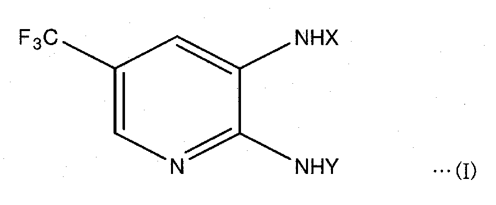 wherein X is a group: -CW¹R¹ or -C(=W¹)W²R²
   in which
   R¹ is alkyl, haloalkyl, alkoxycarbonylalkyl, alkenyl, haloalkenyl, alkenyl substituted with thienyl, cycloalkyl, cycloalkyl substituted with a halogen atom, phenyl, phenyl substituted with a halogen atom, phenyl substituted with alkyl or haloalkyl, phenyl substituted with alkoxy or haloalkoxy, tetrahydronaphthyl, indanyl, furanyl, or thienyl,
   R² is alkyl or haloalkyl, and
   W¹ and W² each independently represents an oxygen or sulfur atom, and
   Y is -SO₂R⁹
   in which
   R⁹ is alkyl, haloalkyl, phenyl, phenyl substituted with a halogen atom, phenyl substituted with alkyl or haloalkyl, or phenyl substituted with alkoxy or haloalkoxy].
[29] The Rap1-p30 binding inhibitor according to the above [28] wherein X is alkoxycarbonylalkylcarbonyl, alkenylcarbonyl, alkenylcarbonyl substituted with thienyl, cycloalkylcarbonyl, indanylcarbonyl, furancarbonyl, thiophenecarbonyl, tetrahydronaphthylcarbonyl, or benzoyl unsubstituted or optionally substituted with a halogen atom or haloalkyl, and Y is alkylsulfonyl;
[30] The Rap1-p30 binding inhibitor according to the above [28] wherein X is cycloalkylcarbonyl, furancarbonyl or benzoyl unsubstituted or optionally substituted with halogen, and Y is alkylsulfonyl;
[31] The Rap1-p30 binding inhibitor according to the above [28] wherein the compound is selected from the group consisting of
   N-(2-ethylsulfonylamino-5-trifluoromethyl-3-pyridyl)cyclohexanecarboxamide,
   N-(2-methylsulfonylamino-5-trifluoromethyl-3-pyridyl)-4-fluorobenzamide,
   N-(2-isopropylsulfonylamino-5-trifluoromethyl-3-pyridyl)-3-fluorobenzamide,
   N-(2-methylsulfonylamino-5-trifluoromethyl-3-pyridyl)-2-furancarboxamide, and
   N-(2-isopropylsulfonylamino-5-trifluoromethyl-3-pyridyl)cyclopentanecarboxamide; and
[32] A Rap1-p30 binding inhibitor which comprises an effective amount of N-(2-ethylsulfonylamino-5-trifluoromethyl-3-pyridyl)cyclohexanecarboxamide or a salt thereof.

In another aspect, the present invention provides the following:
[33] A screening method and a reagent for screening for a compound having an activity of inhibiting the interaction between p30 and Rap1;
[34] A screening method and reagent for a compound having an activity of regulating a bioactivity regulated by the expression of p30;
[35] The screening method and reagent according to the above [34] characterized in that the biological activity regulated by the expression of p30 is an activity selected from the group consisting of:
   (a) development of microtubule or induction of microtubule development,
   (b) induction of formation of leading edge and/or uropod,
   (c) enhancement of adhesive activity of LFA-1,
   (d) enhancement of migration activity of T cell by stimulation with a chemokine,
   (e) enhancement of cell adhesion,
   (f) induction of cell migration,
   (g) induction of redistribution of CXCR4 and/or CD44,
   (h) polarization by TCR or a chemokine,
   (i) clustering of LFA-1 at leading edge,
   (j) polarizing of LFA-1,
   (k) localization of p30 to microtubule,
   (1) colocalization of clustering of LFA-1 at leading edge and p30,
   (m) co-accumulation of LFA-1 and p30 on adhesion surface formed by antigen-dependent conjugation between T cell and APC,
   (n) activation of integrin-dependent migration,
   (o) polarization of T cell, and
   (p) SMAC formation of T cell;
[36] The screening method according to the above [33] or [34] characterized in that, in the presence of cells expressing p30 or p30-containing materials derived from the cells,
   (i) a screening target is allowed to make contact in the presence of a Rap1 activator,
   (ii) a screening target is allowed to make contact in the absence of a Rap1 activator, and
   (iii) the test result from the above (i) and the test result from the above (ii) are compared to each other;
[37] The screening method according to the above [36] characterized in that Rap1 is coexpressed with p30;
[38] The screening method according to the above [36] or [37] characterized in that screening is carried out in the presence of active Rap1;
[39] A pharmaceutical drug which comprises an effective amount of a compound having an activity to inhibit the interaction between p30 and Rap1;
[40] The pharmaceutical drug according to the above [39] which comprises an effective amount of a compound having an activity to inhibit a bioactivity selected from the group consisting of:
   (a) development of microtubule or induction of microtubule development,
   (b) induction of formation of leading edge and/or uropod,
   (c) enhancement of adhesive activity of LFA-1,
   (d) enhancement of migration activity of T cell by stimulation with a chemokine,
   (e) enhancement of cell adhesion,
   (f) induction of cell migration,
   (g) induction of redistribution of CXCR4 and/or CD44,
   (h) polarization by TCR or a chemokine,
   (i) clustering of LFA-1 at leading edge,
   (j) polarizing of LFA-1,
   (k) localization of p30 to microtubule,
   (1) clustering of LFA-1 at leading edge and colocalization with p30,
   (m) co-accumulation of LFA-1 and p30 on adhesion surface formed by antigen-dependent conjugation between T cell and APC,
   (n) activation of integrin-dependent migration,
   (o) polarization of T cell, and
   (p) SMAC formation of T cell;
[41] A p30 regulator which controls a bioactivity of p30;
[42] The p30 regulator according to the above [41] wherein the activity through the interaction between p30 and Rap1 is controled;
[43] A pharmaceutical drug which comprises an effective amount of the p30 regulator according to the above [41] or [42] ;
[44] A p30 inhibitor which inhibits a bioactivity of p30;
[45] The p30 inhibitor according to the above [44] wherein the activity through the interaction between p30 and Rap1 is inhibited ;
[46] A pharmaceutical drug which comprises an effective amount of the p30 inhibitor according to the above [44] or [45] ;
[47] A p30 activator which promotes a bioactivity of p30;
[48] The p30 activator according to the above [47] wherein the activity through the interaction between p30 and Rap1 is promoted;
[49] A pharmaceutical drug which comprises an effective amount of the p30 activator according to the above [47] or [48] ;
[50] A screening method and reagent characterized in that p30 is used; and
[51] The screening method and reagent according to the above [50] wherein screening for a pharmaceutical agent is performed.

### ADVANTAGEOUS EFFECTS OF THE PRESENT INVENTION

In the present invention, it becomes possible to find that cell activation and control of signal transduction are effected via the interaction between p30 and Rap1 to develop technologies with use of the findings, to perform screening of a substance that regulates the interaction between p30 and Rap1 such as a compound to inhibit the binding between p30 and Rap1 and develop reagents to be used for such an object, and to develop p30-Rap1 binding regulatory agents such as identified inhibitors, serving as pharmaceutical drugs including anti-inflammatory agents, immunosuppressive agents, immunosuppressive drugs for transplantation, anticancer agents, and the like with use of the present technologies. Further, in the present invention, it is possible to provide, for example, substances that function in dominantly negative fashion in cells or exert an inhibitory activity against binding between p30 and Rap1 such as a modified p30-related peptide or nucleic acid and further an anti-p30 antibody and to make use of these substances. The development of reagents, assay methods, and the like for analysis of biofunction can also be developed with use of the technologies and knowledge of the present invention.

The above objectives and other objectives, features, advantages, and aspects of the present invention will be readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents listed herein for illustrative purposes are hereby incorporated by reference into the present disclosure and should be interpreted as part of this specification.

### BRIEF DESCRIPTION OF THE DRAWINGS

Fig. 1 depicts the amino acid sequence of p30 and its gene structure, where the amino acid sequence of Nore1 is also shown;
Fig. 2 represents an analysis result of association of p30 with Rap1 which is a photograph taken by exposing to chemiluminescent film by an ECL chemiluminescence method;
Fig. 3A represents electrophoretograms showing a result of tissue distribution of p30 expression obtained by an RT-PCR method;
Fig. 3B represents electrophoretograms showing a result of Western blot for various cells by using a specific anti-p30 antibody;
Fig. 4 represents a result obtained by studying an effect of coexpression of the wild-type p30 or a RBD domain mutant of p30 and Rap1 on cell migration, and electrophoretograms representing results of Western blot are shown on the right;
Fig. 5 represents an effect of p30 on cell migration induced by a chemokine;
Fig. 6A represents a regulatory effect of p30 on LFA-1 adhesion;
Fig. 6B represents regulatory effects of p30 mutants on the LFA-1 adhesion, in which an enhancement of the adhesion of LFA-1 to ICAM-1 was observed only by E37G that retains p30 binding activity;
Fig. 6C illustrates a result showing that deltaNp30 suppresses the adhesion of LFA-1 to ICAM-1 by TCR stimulation in an expression-dependent manner, and the lower photograph is an electrophoretogram showing deltaNp30 expression;
Fig. 7 represents a test result of an effect of compound I on Rap1-p30 binding;
Fig. 8 represents microphotographs showing results of observation of microtubule development by p30 with use of a confocal laser scanning microscope; and
Fig. 9 represents microphotographs showing results obtained by observation of localization of p30 and LFA-1 at a contact site of a T cell and an antigen presenting cell with use of a confocal laser scanning microscope.

### BEST MODES OF CARRYING OUT THE INVENTION

In the present invention, the interaction and/or binding between the polypeptide p30 represented by SEQ ID NO:4 and the polypeptide Rap1 represented by SEQ ID NO:2, particularly the active form of Rap1, has been found to play a key role in expression of bioactivities such as cell adhesion, for example, in the downstream of Rap1, and thus a technology that makes use of such a phenomenon is provided. The bioactivities in which p30 participates include development of microtubule or induction of microtubule development, induction of formation of leading edge and/or uropod, enhancement of adhesive activity of LFA-1, enhancement of migration activity of T cell by stimulation with a chemokine, enhancement of cell adhesion, induction of cell migration, induction of redistribution of CXCR4 and/or CD44, polarization of cell by stimulation with cross-linking of T cell receptor (TCR) complex by an antibody or a chemokine and the like, clustering of LFA-1 at leading edge, polarization of LFA-1, localization of p30 to microtubule, colocalization of clustering of LFA-1 at leading edge and p30, co-accumulation of LFA-1 and p30 on adhesion surface formed by antigen-dependent conjugation between T cell and APC (antigen-presenting cell), activation of integrin-dependent migration, polarization of T cell, SMAC formation of T cell, and the like. Therefore, p30 was termed RAPL (regulator for cell adhesion and polarization enriched in lymphoid tissues) (Koko Katagiri et al., Nature Immunology, Aug. 4, 2003, (8): 741-748). In the present invention, p30 and RAPL represent the same polypeptide having the amino acid sequence shown by SEQ ID NO:4. In the following description, p30 indicates RAPL.

It is possible to detect or measure an interaction between p30 and active Rap1 by detection or measurement of any one of the above bioactivities in which p30 participates. The use of the technology allows not only screening of an inhibitor or promotor for the interaction and/or the binding of p30 to active Rap1 but also development of a drug using the identified inhibitor or promotor. According to the present technology, it becomes possible not only to regulate the binding between p30 and active Rap1 but also to regulate their interaction. Thus, a compound that participates in the regulation of the binding of p30 to active Rap1 the regulation of the interaction thereof, and the like can be screened, thereby making it possible to study various physiological phenomena and bioactive phenomena as well as develop a pharmaceutical agent related to these phenomena.

In the present invention, a method for screening a compound that inhibits or promotes the interaction and/or the binding between the active form of polypeptide of the polypeptide Rap1 represented by SEQ ID NO:2 and the polypeptide p30 represented by SEQ ID NO:4 is provided by the technology. The screening method is carried out by allowing a test sample for screening to be present with a polypeptide selected from the group (a) consisting of an active form of polypeptide, a partial peptide thereof, and a salt thereof containing an amino acid sequence identical or essentially identical to the amino acid sequence represented by SEQ ID NO:2 and an active form of polypeptide, a partial peptide thereof, and a salt thereof containing an amino acid sequence represented by SEQ ID NO:2 in which glycine of the twelfth amino acid is replaced with valine or an amino acid sequence essentially identical to the point-mutated amino acid sequence and a polypeptide selected from the group (b) consisting of a polypeptide, a partial peptide thereof, and a salt thereof containing an amino acid sequence identical or essentially identical to the amino acid sequence represented by SEQ ID NO:4. Typically, the polypeptide of the group (a) or the polypeptide of the group (b) to be used is a polypeptide obtained by gene recombination technology as a recombinant protein, while cells coexpressing these polypeptides or extracts therefrom may also be used.

The active form of polypeptide of the group (a) in the present invention refers to a polypeptide capable of binding to p30. When the polypeptide is prepared by extraction from cells, such an active polypeptide of the group (a) can be obtained by activation by treating with GTP or a GTP analog such as GTPγS or GppNHp for 10 min to one hour at 4 to 37°C in a buffer solution. The activation by a GTP analog such as GTPγS or GppNHp is preferred, and the activation by GTPγS is more desirable. When a polypeptide of the group (a) is expressed in a cell, the polypeptide can be converted into the active form by stimulating the cell by cross-linking of T cell receptor (TCR) complex using an antibody, or a chemokine. An example of the stimulation by cross-linking of TCR complex using an antibody is a treatment, for example, with an anti-CD3 monoclonal antibody, and the polypeptide can be activated by incubating the cell for 10 min to one hour at 37°C in the presence of an antibody 2C11 (10 µg/ml concentration). The stimulation by a chemokine includes treatment with SLC (secondary lymphoid tissue chemokine), CCL21 (chemokine cc motif ligand 21), SDF-1 (stromal cell-derived factor-1), and the like, and the polypeptide can be activated by incubation treatment of the cell for 10 min to one hour at 37°C in the presence of these substances (100 nM) .

In the screening method, screening of test samples can be performed by various techniques. For example, screening may include the processes of (1) allowing a polypeptide selected from the group (a) consisting of an active form of polypeptide, a partial peptide thereof, and a salt thereof containing an amino acid sequence identical or essentially identical to the amino acid sequence represented by SEQ ID NO:2 and an active form of polypeptide containing an amino acid sequence represented by SEQ ID NO:2 in which glycine of the twelfth amino acid is replaced with valine or an amino acid sequence essentially identical to the point-mutated amino acid sequence, a polypeptide selected from the group (b) consisting of a polypeptide, a partial peptide thereof, and a salt thereof containing an amino acid sequence identical or essentially identical to the amino acid sequence represented by SEQ ID NO:4, and a test sample to come in contact with one another and (2) detecting the interaction and/or the binding between the polypeptide selected from the group (a) and the polypeptide selected from the group (b). Conceivable methods for allowing the polypeptide selected from the group (a), the polypeptide selected from the group (b), and the test sample to be coexisted in the present invention include a method to allow the three ingredients to come in contact with one another at the same time, a method in which the test sample is allowed to make contact with the polypeptide of the group (a) or the polypeptide of the group (b) in advance, and the like. When cells coexpressing these polypeptides are used, selection of those methods is possible by manipulating expression time of each polypeptide and adjusting contact time of the test sample with the cells.

In the present invention, "essentially identical amino acid sequence" indicates an amino acid sequence in which the homology to the original amino acid sequence is higher than ca. 70%, preferably ca. 80%, more preferably ca. 90%, and most preferably ca. 95%. For example, in the case of "an amino acid sequence essentially identical to the amino acid sequence represented by SEQ ID NO:4," the original amino acid sequence is the amino acid sequence represented by SEQ ID NO:4. "A polypeptide containing an amino acid sequence essentially identical to the amino acid sequence" contains the above-described "essentially identical amino acid," and a polypeptide and the like essentially identical to the polypeptide having the original amino acid sequence are desirable. In the present invention, "essentially identical" means that protein activities, for example, binding activity, physiological activity, and bioactivity, are essentially the same. Furthermore, the meaning of the term may include a case having an activity essentially of the same quality, and the activity essentially of the same quality includes regulation of cell adhesion, cell migration, cell polarization, and the like. The activity essentially of the same quality indicates that their activities are of the same quality in nature. For example, activities such as binding activities are desirably comparable (for example, from ca. 0.001 to ca. 1,000 fold, preferably from ca. 0.01 to ca. 100 fold, more preferably from ca. 0.1 to ca. 20 fold, still more preferably from ca. 0.5 to ca. 2 fold); however quantitative measures such as degrees of these activities and molecular weights of proteins may differ.

The polypeptides of the group (a) and the group (b) of the present invention may be polypeptides having a favorable alteration such as substitution, deletion, insertion, and addition of amino acid and may also be altered in physiological properties or chemical properties. The polypeptide subjected to the substitution, deletion, insertion, or addition may be regarded as essentially identical to the one not subjected to such substitution, deletion, insertion, or addition. An essentially identical amino acid substitute in the amino acid sequence can be selected from other amino acids of the class to which the amino acid of concern belongs. For example, nonpolar (hydrophobic) amino acids include alanine, phenylalanine, leucine, isoleucine, valine, proline, tryptophan, methionine, and the like. Polar (neutral) amino acids include glycine, serine, threonine, cysteine, tyrosine, asparagine, glutamine, and the like. Amino acids having a positive charge (basic amino acids) include arginine, lysine, histidine, and the like, and amino acids having a negative charge (acidic amino acid) include aspartic acid, glutamic acid, and the like. In some cases, cysteine may be replaced by serine, glycine by alanine or leucine, or leucine by alanine, isoleucine, valine, and the like. The polypeptide of the present invention can be modified at a contained amino acid residue by a chemical method or can be modified or partially degraded by an enzyme peptidase such as pepsin, chymotrypsin, papain, bromelain, endopeptidase, or exopeptidase, followed by its derivatization.

The target peptide or polypeptide (or protein) may be the one in which one or more amino acid residues are different from natural amino acids in view of their identity or the positions of one or more amino acid residues are different from those of natural ones. The polypeptide of the present invention includes a deletion analogue in which one or more amino acid residues (for example, from one to 80, preferably from one to 60, more preferably from one to 40, still more preferably from one to 20, and particularly from one to 10) peculiar to Rap1 or p30 are lacking, a substitution analogue in which one or more peculiar amino acid residues (for example, from one to 80, preferably from one to 60, more preferably from one to 40, still more preferably from one to 20, and particularly from one to 10) are substituted by other amino acid residues, an addition analogue in which one or more amino acid residues (for example, from one to 80, preferably from one to 60, more preferably from one to 40, still more preferably from one to 20, and particularly from one to 10) are added, and an insertion analogue in which one or more amino acid residues (for example, from one to 80, preferably from one to 60, more preferably from one to 40, still more preferably from one to 20, and particularly from one to 10) are inserted. When an amino acid sequence is inserted, deleted, or substituted as described above, the position of the insertion, deletion, or substitution is not particularly limited.

When domain structure or substrate binding ability characteristic of natural proteins is maintained, the above mutants are all included in the present invention. The mutants that have primary structural conformation or part thereof essentially identical to the natural protein may be regarded as being included. Further, the mutants having a biological activity essentially identical to the natural protein may also be regarded as being included. Furthermore, the mutant may be one of naturally occurring mutants.

In the present invention, the test sample can also be screened by Rap1 and p30 binding inhibition besides a bioactivity resulting from the interaction between Rap1 and p30. A polypeptide of the group (a) used for such a case is not particularly limited as long as it has an activity to bind to the wild type p30. The polypeptide may be one that has lost a biological activity of Rap 1, and may also be one that has an amino acid sequence modified by substitution, deletion and/or addition of an amino acid residue as described above in the amino acid sequence represented by SEQ ID NO:2 as long as it has an activity to bind to wild type p30. Further, a polypeptide of the group (b) used for such a case is not particularly limited as long as it has an activity to bind the wild type Rap 1. It may also be one that has lost a biological activity of p30.

The test sample includes, for example, proteins, peptides, non-peptide compounds, synthetic compounds, fermented products, plant extracts, and tissue extracts and cell extracts from e.g. animal. Examples of test compounds used for the test sample may contain preferably anti-p30 antibody, enzyme inhibitor, cytokine, compounds having various inhibitory activities, and particularly synthetic compounds. These compounds may be novel ones or known ones. Representative compounds include, for example, various diaminotrifluoromethylpyridine derivatives that are disclosed in Japanese Published Unexamined Patent Application No. H06-263735. The screening can be carried out in accordance with a general method for measurement of binding activity or bioactivity. [Screening method]

One mode of the screening method of the present invention is carried out by allowing any active form of polypeptide of the group (a) to come in contact with any one polypeptide of the group (b) and a test sample, and then detecting or measuring a bioactivity in which p30 participates, thereby detecting an interaction between the polypeptides of both of the groups and selecting a compound to promote or inhibit the interaction. When a purified compound or the like is screened, the selection process may be omitted.

The bioactivity in which p30 participates includes development of microtubule or induction of microtubule development, induction of formation of leading edge and/or uropod, enhancement of adhesive activity of LFA-1, enhancement of migration activity of T cell by stimulation with a chemokine, enhancement of cell adhesion, induction of cell migration, induction of redistribution of CXCR4 and/or CD44, polarization of cell stimulated by cross-linking of T cell receptor (TCR) complex by antibody or a chemokine and the like, clustering of LFA-1 at leading edge, polarization of LFA-1, colocalization of p30 to microtubule, colocalization of clustering of LFA-1 at leading edge and p30, co-accumulation of LFA-1 and p30 on an adhesion surface formed by antigen-dependent conjugation between T cell and APC (atigen-presenting cell), activation of integrin-dependent migration, polarization of T cell, and SMA formation of T cell (Koko Katagiri et al., Nature Immunology, (8): 741-748).

Another mode of the screening method of the present invention is carried out by allowing an active polypeptide of the group (a) to come in contact with a polypeptide of the group (b) and a test sample, and then detecting the occurrence of binding between the polypeptides of both of the groups and selecting a compound that promotes or inhibits the occurrence of binding. When a purified compound or the like is screened, the selection process may be omitted. When activation of the polypeptide of the group (a) is required, it is activated with use of a GTP derivative as described above. When the polypeptide is immobilized, the activation may be performed before the immobilization or after the immobilization, and preferably after the immobilization.

The polypeptides used in the present invention can be used via binding to a support. First, either one of the purified or crudely purified polypeptides of the group (a) or the group (b) in a combination is allowed to bind to the support. In order to allow the polypeptide to bind to the support, the polypeptide may be immobilized to the support by a standard method. The support to which a polypeptide is bound includes, for example, insoluble polysaccharides, for example, agarose, dextran, cellulose, synthetic resin, e.g. polystyrene, polyacrylamide, and silicone. More specifically, commercially available beads and plates produced using the above as a material are used. In the case of beads, a column packed with them and the like may be used. In the case of plates, multiwell plate (96-well multiwell plate and the like) and biosensor chip are listed.

In order to allow a polypeptide to bind to a support, a generally used method that makes use of chemical binding, physical adsorption and the like may be used. Further, it is also possible that an antibody recognizing a polypeptide specifically is allowed to bind to a support in advance, and that this antibody and a polypeptide are allowed to bind to each other. Furthermore, they can be bound to each other via avidin/biotin.

The binding between a polypeptide of the group (a) and a polypeptide of the group (b) is generally performed in a buffer. As the buffer, for example, phosphate buffer or Tris buffer is used. Conditions for incubation include a condition that has already been frequently employed, for example, incubation for 1 second to 3 hours at from 4°C to room temperature, preferably for 3 seconds to 2 hours, and more preferably for 10 seconds to 30 minutes. For washing after incubation, any washing solution may be used as long as it does not prevent binding of protein, and for example, a buffer solution containing a surface active agent is used. As the surface active agent, for example, 0.05% Tween20 is used.

For selecting a target compound, a polypeptide of the group (a), a polypeptide of the group (b) and a test sample are incubated under an appropriate condition and then washed, whereby specific binding can be separated from non-specific binding. Then, a binding state between the polypeptide of the group (a) and the polypeptide of the group (b) may be evaluated.

On selecting a target compound, either of a polypeptide of the group (a) and a polypeptide of the group (b) is available for binding to a support. That is, when a polypeptide of the group (a) is allowed to bind to a support, the polypeptide of the group (a) is immobilized, and then a mixture in which the polypeptide of the group (b) and a test sample are premixed may be added, or the polypeptide of the group (b) may be added after the test sample is added. In a similar way, when the polypeptide of the group (b) is immobilized to the support, a mixture in which the polypeptide of the group (a) and the test sample are mixed in advance may be added, or the polypeptide of the group (a) may be added after the test sample is added. The polypeptide of the group (a), the polypeptide of the group (b), and the test sample that are added in the order described above are incubated under an appropriate condition, and a binding state between the polypeptide of the group (a) and the polypeptide of the group (b) can be evaluated.

In the screening method of the present invention, not only a group in which a polypeptide is allowed to come in contact with a test sample but also a control group may be prepared. For the control group, a negative control group that does not contain the test sample or a positive control group, or both groups may be prepared.

On detecting or measuring a bound polypeptide in the present invention, the bound polypeptide may be merely detected, or the bound polypeptide may be quantitatively measured. In these cases, a target compound can be detected by comparing the results obtained from a negative control group that does not contain the test sample and a group containing the test sample and/or a positive control group.

Further, these results are obtained as numerical values, and an activity of the target compound can be quantitatively measured by comparing these numerical values. When the measurement is quantitatively carried out, a target compound can be detected by comparing the numerical values obtained from the negative control group that does not contain a test sample and the group to which a test sample is applied. When the obtained numerical value is compared with that from the negative control group and the obtained value increases or decreases, it is possible to judge that the test sample contains a target compound.

Furthermore, when measurement is quantitatively carried out, quantification can be performed based on a standard curve prepared from the numerical values obtained from the positive control group containing known amounts of a compound that is known to inhibit binding between a polypeptide of the group (a) and a polypeptide of the group (b). When the amount of a bound polypeptide is large, it is assumed that the activity of the compound to inhibit binding between these polypeptides is low. On the other hand, when the amount of a bound polypeptide is small, it is assumed that the activity of the compound to inhibit binding between these polypeptides is strong.

In the present invention, it is possible to use a biosensor that makes use of a surface plasmon resonance phenomenon as a means to detect or measure the bound polypeptide. The biosensor that makes use of a surface plasmon resonance phenomenon allows an interaction between these polypeptides to be observed as a surface plasmon resonance signal (for example, BIAcore, product of Pharmacia) in real time with use of a minute amount of polypeptide and without labeling. Accordingly, the use of a biosensor such as BIA core makes it possible to evaluate binding between polypeptides used in the present invention. In other words, a sensor chip immobilized with one polypeptide in a combination of a polypeptide of the group (a) and a polypeptide of the group (b) is allowed to come in contact with the other polypeptide of the combination, and the other polypeptide that binds to the one polypeptide immobilized is detected as a change in resonance signal.

Specifically, the following is carried out. First, a sensor chip CM5 (product of Biosensor) is activated, and one polypeptide in a combination of a polypeptide of the group (a) and a polypeptide of the group (b) is immobilized on the sensor chip. That is, after the sensor chip is activated with an aqueous solution of EDC/NHS (200 mM EDC (N-ethyl-N'-(3-dimethylaminopropyl)carbonate hydrochloride), 50 mM NHS (N-hydorxysuccinimide)), the sensor chip is washed with an HBS buffer (10 mM HEPES, pH 7.4, 150 mM NaCl, 3.4 mM EDTA, 0.05% Tween 20). Then, an appropriate amount of a polypeptide having an interaction activity that is dissolved in the HBS buffer is allowed to come in contact with the sensor chip to be immobilized. After the sensor chip is washed with the HBS buffer, residual active groups on the sensor chip are blocked with an ethanolamine solution (1 M ethanolamine hydrochloride, pH 8.5). Then, the sensor chip is washed again with the HBS buffer to be used for evaluation of binding. Next, an appropriate amount of the other polypeptide dissolved in the HBS buffer is added. At this time, the amount of the polypeptide having an interaction activity to bind to the polypeptide immobilized to the sensor chip is observed as an increase in resonance signal value.

Still further, in the above evaluation system of binding, a test sample is added following the other polypeptide having the interaction activity to the one polypeptide. In addition to a group in which the test sample is added, a control group may be prepared. As the control group, a negative control group that does not contain the test sample or a positive control group, or both groups may be prepared.

The bound polypeptide can be quantitatively measured as a change in the resonance signal value. In this case, it is possible to detect and determine a target compound by comparing results obtained from the negative control group that does not contain the test sample, the group containing the test sample and/or the positive control group. In the present invention, a means to detect or measure the bound polypeptide can include labeling of either one of the polypeptides and utilizing the label of the bound polypeptide. For example, in the foregoing screening method, the other polypeptide with which the one polypeptide is allowed to come in contact is labeled in advance, incubated with a test sample, and then washed, followed by detecting or measuring the bound polypeptides by the label. That is, preferably, the one polypeptide bound to the support is allowed to come in contact with the test sample and the other labeled polypeptide. After the incubation and washing, the label of the bound polypeptide can be detected or measured.

The polypeptides used in the present invention can be labeled by a generally known method. Labeling materials include, for example, radioisotope, enzyme, fluorescent substance, and biotin/avidin. Commercially available labeling materials can be used for these labeling materials. The radioisotopes include, for example, ³²p, ³³p, ¹³¹I, ¹²⁵I, ³H, ¹⁴C, and ³⁵S. The enzymes include, for example, alkaline phosphatase, horse radish peroxidase (HRP), β-galactosidase, and β-glucosidase. The fluorescent substances include, for example, fluoroscein isothiocyanate (FITC) and rhodamine. These are commercially available and labeling is carried out by a known method. Specifically, the labeling can be performed as follows. That is, a solution containing either polypeptide is added to a plate and left standing overnight. After washing the plate, the plate is blocked with, for example, BSA in order to prevent non-specific binding of polypeptides. The plate is washed again, and a test sample and the other polypeptide that is labeled are added to the plate. At the same time, a negative control group that does not contain the test sample and/or a positive control group are prepared, followed by incubation. After the incubation, the plate is washed and the bound polypeptide is detected or measured. For the detection or the measurement, the polypeptide is detected or measured by liquid scintillation in the case of the radioisotope. In the case of the enzyme, its substrate is added, and the polypeptide is detected or measured by an enzymatic change of the substrate, for example, coloring by means of an absorption spectrometer. In the case of the fluorescent substance, the polypeptide is detected or measured by a fluorometer. These results are compared to the numerical values obtained from the control groups, thereby making it possible to determine a target compound.

In the present invention, a primary antibody that specifically recognizes one polypeptide in a combination of a polypeptide of the group (a) and a polypeptide of the group (b) can be used as a means to detect or measure a bound polypeptide. For example, the one polypeptide is allowed to come in contact with the other polypeptide and a test sample. After the incubation with the test sample and subsequent washing, the bound polypeptide is detected or measured by the primary antibody that recognizes the polypeptide specifically. That is, preferably, the one polypeptide bound to the support is allowed to come in contact with the test sample and the other polypeptide. After incubation and subsequent washing, the bound polypeptide may be detected or measured by the antibody that recognizes the polypeptide specifically. The primary antibody is preferably labeled with a labeling material. Specifically, the labeling can be performed as follows. That is, a solution containing either polypeptide is added to a plate and left standing overnight. After the plate is washed, it is blocked, for example, with BSA in order to prevent non-specific binding of polypeptide. It is washed again, and the test sample and the other polypeptide are added to the plate. At the same time, a negative control group that does not contain the test sample and/or a positive control group are prepared, and they are incubated. After the incubation, they are washed, and the antibody against the polypeptide that is added with the test sample is added. After appropriate incubation, the plate is washed, followed by detecting or measuring the polypeptide by the primary antibody that recognizes the polypeptide specifically. For the detection or the measurement, in the case of the radioisotope, the polypeptide is detected or measured by liquid scintillation. In the case of the enzyme, its substrate is added and the polypeptide is detected or measured by an enzymatic change of the substrate, for example, coloring by means of an absorption spectrometer. In the case of the fluorescent substance, the polypeptide is detected or measured by a fluorometer. These results are compared to the numerical values obtained from the control groups, thereby making it possible to determine a target compound.

In the present invention, a means to detect or measure a bound polypeptide can make use of a primary antibody that specifically recognizes still another peptide that is fused with the polypeptide used in the present invention. For example, in the aforementioned screening method, one polypeptide is allowed to come in contact with a test sample and the other polypeptide, and incubated. After washing, the bound polypeptide is detected or measured by the primary antibody that specifically recognizes said still another peptide fused with the polypeptide. That is, preferably, the one polypeptide bound to the support is allowed to come in contact with the test sample and the other polypeptide. After incubation and subsequent washing, the bound polypeptide may be detected or measured by the primary antibody that specifically recognized said still another peptide fused with the polypeptide. The primary antibody is preferably labeled with a labeling material. Specifically, the labeling can be carried out as follows. That is, a solution containing either polypeptide is added to a plate and left standing overnight. After washing the plate, it is blocked with, for example, BSA to prevent non-specific binding of the polypeptide. The plate is again washed, and the test sample and the other polypeptide fused with said still another peptide are added to the plate. At the same time, a negative control group that does not contain the test sample and/or a positive control is prepared and incubated. After incubation and subsequent washing, the antibody against said still another peptide fused with the polypeptides that is added with the test sample is added. After appropriate incubation, the plate is washed, and the polypeptide is detected or measured by the primary antibody that recognizes specifically said still another peptide fused with the polypeptide. For the detection or the measurement, the polypeptide is detected or measured by liquid scintillation in the case of the radioisotope. In the case of the enzyme, its substrate is added and the polypeptide is detected or measured by an enzymatic change of the substrate, for example, coloring, by means of an absorption spectrometer. In the case of the fluorescent substance, it is detected or measured by a fluorometer. These results are compared with the numerical values obtained from the control groups, thereby making it possible to determine a target compound.

In the present invention, a means to detect or measure a bound polypeptide can make use of a primary antibody that specifically recognizes the polypeptide used in the present invention and a secondary antibody that specifically recognizes the primary antibody. For example, the test sample and one polypeptide are allowed to make contact with the other polypeptide. After incubation and subsequent washing, the bound polypeptide is detected or measured by the primary antibody that specifically recognizes the polypeptide and a secondary antibody that specifically recognizes the primary antibody. That is, preferably, the one polypeptide bound to the support is allowed to come in contact with the test sample and the other polypeptide. After incubation and subsequent washing, the bound polypeptide is detected or measured by the primary antibody that specifically recognizes the polypeptide and the secondary antibody that recognizes specifically the primary antibody. The secondary antibody is preferably labeled with a labeling material. Specifically, the labeling can be performed as follows. That is, a solution containing one polypeptide is added to a plate and left standing overnight. After washing the plate, the plate is blocked with, for example, BSA to prevent non-specific binding of polypeptide. The plate is washed again, and the test sample and the other polypeptide are added to the plate. At the same time, a negative control group that does not contain the test sample and/or a positive control group is prepared and incubated. After the incubation and subsequent washing, the primary antibody against still another peptide fused with the polypeptide that is added with the test sample is added. After appropriate incubation, the plate is washed, followed by adding the secondary antibody that specifically recognizes the primary antibody. After appropriate incubation, the plate is washed, and the polypeptide is detected or measured by the secondary antibody that specifically recognizes the primary antibody capable of recognizing specifically said still another polypeptide. For the detection or the measurement, in the case of the radioisotope, the polypeptide is detected or measured by liquid scintillation. In the case of the enzyme, its substrate is added and the polypeptide is detected or measured by an enzymatic change of the substrate, for example, coloring, by means of an absorption spectrometer. In the case of the fluorescent substance, the polypeptide is detected or measured by a fluorometer. These results are compared with the numerical values obtained from the control groups, thereby making it possible to select a target compound.

In the present invention, a means to detect or measure a bound polypeptide can make use of a primary antibody that specifically recognizes still another peptide that is fused with the polypeptide and a secondary antibody that specifically recognizes the primary antibody. For example, in the aforementioned screening method, one polypeptide is allowed to come in contact with the test sample and the other polypeptide. After incubation and subsequent washing, the bound polypeptide is detected or measured by the primary antibody that specifically recognizes said still another peptide fused with the polypeptide and the secondary antibody that specifically recognizes the primary antibody. In other words, preferably, the one polypeptide bound to the support is allowed to come in contact with the test sample and the other polypeptide. After incubation and subsequent washing, the bound polypeptide may be detected or measured by the primary antibody that specifically recognizes the said still another peptide fused with the polypeptide and the secondary antibody that specifically recognizes the primary antibody. The secondary antibody is preferably labeled with a labeling material.

Specifically, the labeling can be carried out as follows. That is, a solution containing either polypeptide is added to a plate and left standing overnight. After the plate is washed, the plate is blocked with, for example, BSA to prevent non-specific binding of polypeptide. The plate is washed again, and a test sample and the other polypeptide fused with said still another peptide are added to the plate. At the same time, a negative control group that does not contain the test sample and/or a positive control group is prepared and incubated. After the incubation and subsequent washing, the primary antibody against said still another peptide fused with the polypeptide that is added with the test sample is added. After appropriate incubation, the plate is washed, followed by the addition of the secondary antibody that specifically recognizes the primary antibody. After appropriate incubation and subsequent washing, the polypeptide is detected or measured by the secondary antibody that specifically recognizes the primary antibody that specifically recognizes said still another peptide fused with the polypeptide. For detection or measurement in the case of the radioisotope, the polypeptide is detected or measured by liquid scintillation. In the case of the enzyme, its substrate is added, and the polypeptide is detected or measured by an enzymatic change of the substrate, for example, coloring, by means of an absorption spectrometer. In the case of the fluorescent substance, the polypeptide is detected or measured by a fluorometer. These results are compared with the numerical values obtained from the control groups, thereby making it possible to determine a target compound.

More specifically, the present invention is preferably carried out by ELISA (Enzyme-linked Immunosorbent Assay) as follows. That is, one peptide, for example, a polypeptide of the group (a) fused with 6xHis is diluted with a solid phased buffer (0.1 M NaHCO₃, 0.02% NaN₃, pH 9.6). An appropriate volume of this diluted aqueous solution is added to each well of a 96-well immuno plate (product of Nunc), which is incubated overnight at 4°C. After each well is washed with a washing buffer (prepared by adding Tween 20 to PBS at 0.05%) three times, and 200 µl of a solution of 5% BSA (product of SIGMA) dissolved in PBS is added, followed by blocking overnight at 4°C. Next, each well is washed with the washing buffer three times, and an appropriate amount of the other peptide, e.g. a polypeptide of the group (b) fused with FLAG, that is diluted with a dilution buffer (1% BSA, 0.5% Tween 20, PBS) and a test sample are added, followed by incubation for from 1 second to 3 hours at from 4°C to room temperature, preferably from 3 second to 2 hours, and more preferably from 10 seconds to 30 minutes. Each well is washed with the washing buffer three times, and 100 µl of a mouse anti-FLAG M2 antibody (product of IBI) diluted to 3 µg/ml with the dilution buffer is added to each well, followed by incubation for 1 hour at room temperature. Each well is washed three times with the washing buffer, and 100 µl of an alkaline phosphatase-labeled goat anti-mouse IgG antibody (product of ZYMED) diluted 1000-fold with the dilution buffer is added to each well, followed by incubation for 1 hour at room temperature. Each well is washed five times with the washing buffer, and 100 µl of a coloring solution (p-phenylphosphate dissolved in a substrate buffer; 50 mM NaHCO₃, 10 mM MgCl₂, pH 9.8, at a concentration of 1 mg/ml; product of SIGMA) is added to each well, allowed to react at room temperature, followed by measuring absorbance at 405 nm with use of a microplate reader (Model 3550, product of BIO-RAD). When these results are compared to the numerical values obtained for the negative control group and/or the positive control group, a target compound can be determined. Note that protein G or protein A in place of the secondary antibody can also be used in the detection or measurement with the antibody of the present invention.

The screening method of the present invention may make use of high throughput screening (HTS). Specifically, up to blocking is carried out by manual operation, and high throughput screening can be realized by allowing subsequent reaction to be performed by a robot for automation. Namely, one peptide, e.g. a polypeptide of the group (a) fused with 6xHis is diluted with a solid phased buffer (0.1 M NaHCO₃, 0.02% NaN₃, pH 9.6). An appropriate volume of this diluted aqueous solution is added to each well of a 96-well immuno plate (product of Nunc) and incubated overnight at 4°C. After each well is washed with a washing buffer (prepared by adding Tween 20 at 0.05% into PBS) three times, 200 µl of 5% BSA (product of Sigma) dissolved in PBS is added and subjected to blocking overnight at 4°C. Then, the immuno plate after blocking is set on, for example, a Biomek 2000 HTS system (product of Beckman), and the control program of the system is executed. At this time, the use of a Biomek 2000 dispenser (product of Beckman) or a Multipipette 96-well simultaneous dispenser (product of Sagian) makes it possible to dispense a solution into each well of the immuno plate and remove the solution. In addition, each well of the immuno plate can be washed using an EL 404 microplate washer (product of Bio Tek). The measurement of absorbance can be performed with a SPECTRA max 250 plate reader (product of Molecular Devices). The program is set to execute the following operations. That is, each well is washed three times with the washing buffer, and appropriate amounts of a test sample and the other peptide, e.g. a polypeptide of the group (b) fused with MBP (maltose-binding polypeptide), diluted with a dilution buffer (1% BSA, 0.5% Tween 20, PBS) are added. At the same time, a negative control group that does not contain a test sample and a positive control group are prepared, and these are incubated at from 4°C to room temperature for one sec to 3 hours, preferably for 3 sec to 2 hours, and more preferably for 10 sec to 30 min. After each well is washed three times with the washing buffer, 100 µl of a rabbit anti-MBP antiserum (product of New England Biolabs) diluted 5,000-fold with the dilution buffer is added to each well and incubated for one hour at room temperature. Each well is washed three times with the washing buffer, and 100 µl of an alkaline phosphatase-labeled goat anti-rabbit IgG antibody (product of TAGO) diluted 5,000-fold with the dilution buffer is added to each well and incubated for one hour at room temperature. Each well is washed five times with the washing buffer, 100 µl of a color developing solution (p-nitrophenylphosphate from Sigma dissolved in a substrate buffer; 50 mM NaHCO₃, 10 mM MgCl₂, pH 9.8, at a concentration of 1 mg/ml) is added to each well and allowed to react at room temperature, followed by measurement of absorbance at 405 nm using a microplate reader, Biomek plate reader (product of Beckman/Molecular Devices). A target compound can be identified by comparing these results with those obtained for the control groups.

The antibodies used in the present invention may be commercially available antibodies or those contained in a commercially available kit. Monoclonal antibodies or polyclonal antibodies prepared by known methods can also be used. Monoclonal antibodies can be prepared by immunization with a desired sensitized antigen according to a conventional immunization method, fusion of the obtained immunocytes with a known parent cell according to a conventional cell fusion method, and screening of monoclonal antibody-producing cells according to a conventional screening method.

As a method of detection or measurement of bound polypeptide in the present invention, FRET (fluorescent resonance energy transfer) between fluorescent proteins can be used by allowing both of a polypeptide of the group (a) and a polypeptide of the group (b) in combination to be fused with specific fluorescent proteins, respectively, to give each fused polypeptide (A. Miyawaki et al. Nature vol. 388:882-887, 1997; N. Mochizuki et al. Nature vol. 411:1065-1068; GFP and bioimaging, A. Miyawaki, ed., Yodosha Co.(Tokyo)). The binding between the polypeptides of the group (a) and the group (b) is detected by measuring FRET that takes place between adjacent fluorescent proteins. For example, an active form of a polypeptide of the group (a) fused with a yellow fluorescent protein (YFP) and a polypeptide of the group (b) fused with a cyano fluorescent protein (CFP) are allowed to come in contact with each other. When both polypeptides are bound to each other, YFP and CFP become in a close state. When excited at 433 nm that is an excitation wavelength of CFP under such a condition, its energy is transferred to YFP due to FRET, thereby allowing to measure emission at 527 nm that is an emission wavelength of YFP. Accordingly, when these fused polypeptides are allowed to come in contact with each other in the presence of a test sample containing a compound that inhibits the binding between the polypeptides of the group (a) and the group (b), a decrease in FRET occurs, resulting in a decrease of emission at 527 nm. Therefore, screening of a compound or the like that regulates the binding between the polypeptides of the group (a) and the group (b) can be performed by comparing the emission intensity in the presence of the test sample with that in its absence. In addition to the bimolecular system described above, FRET takes place even within a single molecule in which a polypeptide of the group (a) fused with the yellow fluorescent protein (YFP) and a polypeptide of the group (b) fused with the cyano fluorescent protein (CFP) are both present in a molecule via a spacer. In this case, the sensitivity of detection can be adjusted by changing the length of the spacer. In addition, even when either one or both of the polypeptides of the both groups are not polypeptides fused with these fluorescent proteins but the antibodies against the polypeptide of each group are labeled with YFP and CFP, respectively, FRET is observed. Therefore, this form can also be used in the screening method of the present invention.

Furthermore, cells expressing the polypeptide fused with the fluorescent proteins (coexpression in the case of a bimolecular system) can be used in the screening method of the present invention. In this instance, detection or measurement of the binding between the polypeptides of the group (a) and the group (b) and detection or measurement of a bioactivity in which p30 plays a role can be carried out at the same time. This is possible, for example, according to the method disclosed in Nature vol. 411:1065-1068 by N. Mochizuki et al., or GFP and bioimaging, A. Miyawaki, ed., Yodosha Co. (Tokyo), and the above detection or measurement is made possible by culturing cells to be used for screening in the presence or absence of a test sample.

In the present invention, the use of "gene recombination technology" makes it possible to isolate and sequence a specific nucleotide, prepare a recombinant, and obtain the specific peptide. The gene recombination technology usable in the present specification includes methods disclosed in, for example, J. Sambrook, E. F. Fritsch & T. Maniatis, "Molecular Cloning: A Laboratory Manual (2nd edition)", Cold Spring Harbor Laboratory Press, Cold Spring Harbor, New York (1989); D. M. Glover et al. ed., "DNA Cloning", 2nd ed., Vol. 1 to 4, (The Practical Approach Series), IRL Press, Oxford University Press (1995); "zoku Seikagaku Jikkenkouza 1, Idensi Kenkyuhou II," the Japanese Biochemical Society ed., Tokyo Kagaku Dozin, Tokyo (in Japanese) (1986), "Shin Seikagaku Jikkenkouza 2, Kakusan III (Recombinant DNA technology)," the Japanese Biochemical Society ed., Tokyo Kagaku Dozin, Tokyo (in Japanese) (1992); "Methods in Enzymology" series, Academic Press, New York, for example, R. Wu ed., "Methods in Enzymology", Vol. 68 (Recombinant DNA), Academic Press, New York (1980); R. Wu et al. ed., "Methods in Enzymology", Vol. 100 (Recombinant DNA, Part B) & 101 (Recombinant DNA, Part C), Academic Press, New York (1983); R. Wu et al. ed., "Methods in Enzymology", Vol. 153 (Recombinant DNA, Part D), 154 (Recombinant DNA, Part E) & 155 (Recombinant DNA, Part F), Academic Press, New York (1987); J. H. Miller ed., "Methods in Enzymology", Vol. 204, Academic Press, New York (1991); R. Wu ed., "Methods in Enzymology", Vol. 216 (Recombinant DNA, Part G), Academic Press, New York (1992); R. Wu ed., "Methods in Enzymology", Vol. 217 (Recombinant DNA, Part H) & 218 (Recombinant DNA, Part I), Academic Press, New York (1993); G. M. Attardi et al. ed., "Methods in Enzymology", Vol. 260 (Mitochondrial Biogenesis and Genetics, Part A), Academic Press, New York (1995); J. L. Campbell ed., "Methods in Enzymology", Vol. 262 (DNA Replication), Academic Press, New York (1995); G. M. Attardi et al. ed., "Methods in Enzymology", Vol. 264 (Mitochondrial Biogenesis and Genetics, Part B), Academic Press, New York (1996); P. M. Conn ed., "Methods in Enzymology", Vol. 302 (Green Fluorescent Protein), Academic Press, New York (1999); S. Weissman ed., "Methods in Enzymology", Vol. 303 (cDNA Preparation and Characterization), Academic Press, New York (1999); J. C. Glorioso et al. ed., "Methods in Enzymology", Vol. 306 (Expression of Recombinant Genes in Eukaryotic Systems), Academic Press, New York (1999); M. lan Phillips ed., "Methods in Enzymology", Vol. 313 (Antisense Technology, Part A: General Methods, Methods of Delivery and RNA Studies) & 314 (Antisense Technology, Part B: Applications), Academic Press, New York (1999); J. Thorner et al. ed., "Methods in Enzymology", Vol. 326 (Applications of Chimeric Genes and Hybrid Proteins, Part A: Gene Expression and Protein Purification), 327 (Applications of Chimeric Genes and Hybrid Proteins, Part B: Cell Biology and Physiology) & 328 (Applications of Chimeric Genes and Hybrid Proteins, Part C: Protein-Protein Interactions and Genomics), Academic Press, New York (2000) the methods disclosed in the references cited therein, or methods essentially identical to or modified from these methods (the descriptions therein are hereby incorporated into the present specification by reference).

The term "polypeptide" used in the present specification may indicate any polypeptide described below. The basic structure of polypeptides is known, and there are descriptions in a large number of reference books and other publications in the technology field. In view of this fact, the term "polypeptide" used in the present specification indicates an arbitrary peptide or an arbitrary protein containing two or more amino acids that are bound to each other via peptide bond or modified peptide bond. In the field, the term "polypeptide" used in the present specification may generally mean both of short-chain polypeptide called, for example, peptide, oligopeptide, or peptide oligomer, and long-chain polypeptide generally called protein for which various forms are known, and the term may mean both. The polypeptide may contain amino acids other than the amino acids that are generally called naturally occurring amino acids (amino acids present in nature: or amino acids encoded by gene). It is understood that the polypeptide can also be altered (modified) not only by natural processes such as processing and other alterations (or modifications) that occur at many amino acid residues including the terminal amino acid residue after translation but also by chemical modification technology known to a person skilled in the art. Many forms are known for alteration (modification) applicable to the polypeptide, and these are described in detail in basic reference books in this field, detailed articles, and a large number of research papers, all of which are known to a person skilled in the art. Some of the particularly conventional alterations and modifications include, for example, alkylation, acylation, esterification, amidation, glycosylation, lipid binding, sulfation, phosphorylation, γ-carboxylation of glutamic acid residue, hydroxylation, ADP-ribosylation, and the like. Available references for these are, for example, T. E. Creighton, Proteins-Structure and Molecular Properties, Second Edition, W. H. Freeman and Company, New York, (1993); B. C. Johnson (Ed.), Posttranslational Covalent Modification of Proteins, Academic Press, New York, (1983) (Wold, F., "Posttranslational Protein Modifications: Perspective and Prospects," pp. 1-12); Seifter et al., "Analysis for Protein Modifications and nonprotein cofactors," Methods in Enzymology, 182: 626-646 (1990); Rattan et al., "Protein Synthesis: Posttranslational Modification and Aging," Ann. N. Y. Acad. Sci., 663: p. 48-62 (1992)

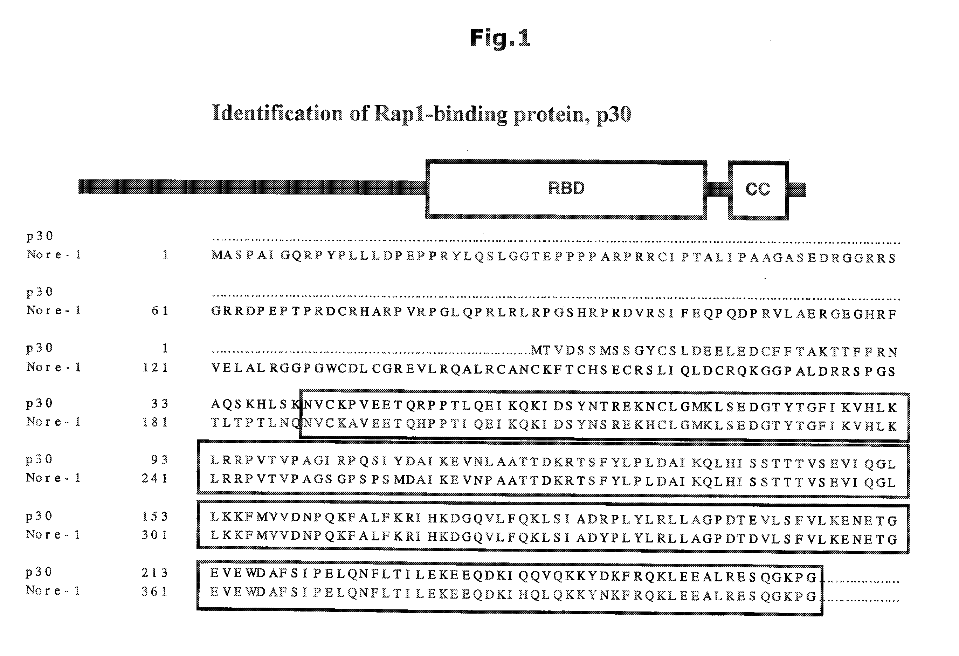

A representative p30 protein of the present invention includes a polypeptide having an amino acid sequence identical or essentially identical to the amino acid sequence of the polypeptide shown in Fig. 1, for example, a polypeptide having at least from 5 to 213 consecutive amino acid residues of the amino acid sequence and a biological activity including an essentially identical biological activity such as Rap1 binding activity, dominantly negative function, or equivalent antigenicity, and a novel polypeptide having not only these properties but also at least 50% or higher homology, or at least 60% or higher homology, or at least 70% or higher homology, or at least 80% or higher homology, or at least 90% or higher homology, or at least 95% or higher homology, or at least 98% or higher homology to any one of the respective domains in Fig 1.

A human p30-related polypeptide of the present invention includes a polypeptide having consecutive amino acid residues containing the whole or part of the amino acid sequence shown in Fig. 1 or consecutive amino acid residues of the amino acid sequence shown in Fig. 1 that are not less than 5, preferably not less than 10, more preferably not less than 20, still more preferably not less than 30, still more preferably not less than 40, still more preferably not less than 50, still more preferably not less than 60, still more preferably not less than 70, still more preferably not less than 80, still more preferably not less than 90, still more preferably not less than 100, and still more preferably not less than 110. The p30-related polypeptide of the present invention may have the whole or part of the amino acid sequence shown in Fig. 1 (Met corresponding to the initiation codon may be lacking). All that have these sequences are included.

The nucleic acid that encodes what is dealt with in the present invention or the p30 protein or polypeptide may be one of those containing a nucleotide sequence encoding the peptide represented by Fig. 1 or part of its consecutive amino acid sequence, a nucleotide sequence constituting at least peptide-coding region in the above nucleotide sequence (including a nucleotide sequence encoding only each characteristic domain), a nucleotide sequence in which an initiation codon (the codon encoding Met) and a termination codon are attached to a code sequence, and a nucleotide sequence containing an equally effective nucleotide sequence encoding a peptide that has an amino acid sequence with 50% or higher homology to the protein encoded by the nucleotide sequence, at least characteristically consecutive amino acid residues of the amino acid sequence in Fig. 1 and the like, and further a biological activity including an essentially identical biological activity such as Rap1 binding activity, dominantly negative function, or equivalent antigenicity.

The nucleic acid encoding the protein is one such as single stranded DNA, double stranded DNA, RNA, DNA-RNA hybrid, or synthetic DNA, or may be any one of human genomic DNA, human genomic DNA library, cDNA derived from human tissues or cells, and synthetic DNA. The nucleotide sequence of the nucleic acid encoding the protein may also be modified (for example, addition, deletion, substitution, etc.), and those modified may also be included. Further, as described below, the nucleic acid of the present invention may be one that encodes the peptide of the present invention or part thereof, and a preferred polynucleotide is DNA. The above "equally effective nucleotide sequence" is a nucleotide sequence that hybridizes, for example, under a stringent condition, to a consecutive nucleotide sequence of the nucleotide sequence that is not less than 5, preferably not less than 10, more preferably not less than 15, and still more preferably not less than 20 and encodes an amino acid sequence essentially identical to the protein, or the like.

One mode of the method for obtaining and isolating a functionally equivalent protein that is well known to one of ordinary skill in the art is a method for introducing a mutation into an amino acid of a protein. That is, the person skilled in the art is able to prepare a modified protein having a function equivalent to a natural protein (for example, p30 protein illustrated in Fig. 1) by appropriate substitution, deletion, addition, and the like of an amino acid for a natural protein by a known method. In addition, mutation of an amino acid can sometimes occur naturally. The protein of the present invention includes a protein having an amino acid sequence in which one or a plurality of amino acids are substituted, deleted, or added in the amino acid sequence of the natural protein and having a function equivalent to that of a natural protein. Alteration of amino acid in the protein is generally within 50 amino acids of the total amino acids, preferably within 30 amino acids, more preferably within 10 amino acids, and still more preferably within 3 amino acids. Alteration of amino acid is made possible, for example, by using a "Transformer Site-directed Mutagenesis Kit" or "ExSite PCR-Based Site-directed Mutagenesis Kit" (product of Clontech) in the case of mutation or substitution, and "Quantum leap Nested Deletion Kit" (product of Clontech) and the like in the case of deletion.

Examples of the methods of mutation, transformation, and modification are those described in, for example, "Zoku Seikagaku Jikkenkouza 1, Idensi Kenkyuhou II," p. 105 (by S. Hirose), the Japanese Biochemical Society ed., Tokyo Kagaku Dozin, Tokyo (in Japanese) (1986), "Shin Seikagaku Jikkenkouza 2, Kakusan III (Recombinant DNA technology)," p 233 (by S. Hirose), the Japanese Biochemical Society ed., Tokyo Kagaku Dozin, Tokyo (in Japanese) (1992); R. Wu, L. Grossman, ed., "Methods in Enzymology," Vol. 154, p. 350 & p. 367, Academic Press, New York (1987); R. Wu, L. Grossman, ed., "Methods in Enzymology," Vol. 100, p. 457 & p. 468, Academic Press, New York (1983); J. A. Wells et al., Gene, 34: 315, 1985; T. Grundstroem et al., Nucleic Acids Res., 13: 3305, 1985; J. Taylor et al., Nucleic Acids Res., 13: 8765, 1985; R. Wu ed., "Methods in Enzymology," Vol. 155, p. 568, Academic Press, New York (1987); A. R. Oliphant et al., Gene, 44: 177, 1986. The methods include, for example, site-directed mutagenesis (site-specific mutagenesis) using a synthetic oligonucleotide or the like (Zoller et al., Nucl. Acids Res., 10: 6487, 1987; Carter et al., Nucl. Acids Res., 13: 4331, 1986), cassette mutagenesis (Wells et al., Gene, 34: 315, 1985), restriction selection mutagenesis (Wells et al., Philos. Trans. R. Soc. London Ser A., 317: 415, 1986), alanine scanning mutagenesis (Cunningham & Wells, Science, 244: 1081-1085, 1989), PCR mutagenesis, Kunkel mutagenesis method, dNTP[αS] mutagenesis method (Eckstein), region-specific mutagenesis using sulfurous acid or nitrous acid, and the like.

In the present specification, "essentially equivalent" or "essentially identical" mean that activities of proteins, for example, binding activities, physiological activities, and biological activities are essentially the same. Furthermore, the meaning of the terms may include the case having activities essentially of the same quality, and the activity essentially of the same quality is exemplified by regulation of cell adhesion and migration. The activity essentially of the same quality indicates that those activities are of the same quality in property, and for example, of the same quality physiologically, pharmacologically, or biologically. For example, it is preferred that an activity such as binding activity is equivalent (for example, from ca. 0.001 to ca. 1,000 fold, preferably from ca. 0.01 to ca. 100 fold, more preferably from ca. 0.1 to ca. 20 fold, and still more preferably from ca. 0.5 to ca. 2 fold), while quantitative measures such as degrees of these activities and molecular weights of proteins may differ.

When the peptides (or polypeptides) are obtained as a free form, these can be converted into a salt form by a method known per se or its comparable method. When obtained as a salt form, these can be converted into a free form or another salt form by a method known per se or its comparable method.

Salts of the peptides (or polypeptides) are preferred to be physiologically acceptable salts or pharmaceutically acceptable salts, but are not limited to these. These salts include, for example, salts with inorganic acids such as hydrochloric acid, hydrobromic acid, sulfuric acid, nitric acid, and phosphoric acid, and salts with organic acids such as acetic acid, formic acid, maleic acid, fumaric acid, succinic acid, citric acid, tartaric acid, malic acid, benzoic acid, methanesulfonic acid, p-toluenesulfonic acid, and benzenesulfonic acid. Further, the salts include ammonium salt, salts with organic bases such as ethylamine, dimethylamine, trimethylamine, and hydroxyethylamine, and the like.

Furthermore, the polypeptide may be expressed as a fusion protein when prepared by a gene recombination technology, followed by conversion and processing in vivo or ex vivo into a polypeptide having a biological activity essentially equivalent to the specific natural protein of the present invention. A method for preparing fusion protein that is conventionally used in gene engineering can be used, and such a fusion protein can also be purified by affinity chromatography and the like by taking advantage of the fusion portion. The fusion protein includes those fused to a histidine tag or an amino acid sequence of β-galactosidase (β-gal), maltose-binding protein (MBP), glutathione-S-transferase (GST), thioredoxin (TRX), or Cre recombinase. Similarly, the polypeptide can be added with a heterogeneous epitope tag and purified by immunoaffinity chromatography using an antibody that binds specifically to the epitope. In a more appropriate embodiment, examples of the epitope tag include AU5, c-Myc, CruzTag 09, CruzTag 22, CruzTag 41, Glu-Glu, HA, Ha. 11, KT3, FLAG (registered trademark, Sigma-Aldrich), Omni-probe, S-probe, T7, Lex A, V5, VP16, GAL4, VSV-G, and the like. (Field et al., Molecular and Cellular Biology, 8: pp. 2159-2165 (1988); Evan et al., Molecular and Cellular Biology, 5: pp. 3610-3616 (1985); Paborsky et al., Protein Engineering, 3(6): pp. 547-553 (1990); Hopp et al., Bio/Technology, 6: pp. 1204-1210 (1988); Martin et al., Science, 255: pp. 192-194 (1992); Skinner et al., J. Biol. Chem., 266: pp. 15163-15166 (1991); Lutz-Freyermuth et al., Proc. Natl. Acad. Sci. USA, 87: pp. 6393-6397 (1990). The yeast two-hybrid method is also available.

Furthermore, the fusion protein may be one labeled so as to become a detectable protein. In a more preferred embodiment, the detectable label may be Biotin Avi Tag of biotin/streptavidin system, a fluorescent substance, or the like. The fluorescent substance includes green fluorescent protein (GFP) derived from the luminescent jellyfish such as Aequorea victorea and its modified mutants (GFP variants), for example, enhanced humanized GFP (EGFP), red-shift GFP (rsGFP), yellow fluorescent protein (YFP), green fluorescent protein (GFP), cyan fluorescent protein (CFP), blue fluorescent protein (BFP), and GFP derived from Renilla reniformis (Supplementary volume of Jikkenigaku, Post genome jidai no jikkenkouza 3-GFP and Bio-imaging, A. Miyawaki, ed., Yodosha Co., Tokyo, (2000) (in Japanese). In addition, detection is made possible by the use of an antibody (including a monoclonal antibody and its fragment) that specifically recognizes the above fusion tag.

For the synthesis of a protein or its partial peptide, methods known in the field of peptide synthesis, for example, chemical synthesis methods such as liquid phase synthesis method and solid phase synthesis method can be used. In these methods, an appropriately protected amino acid is bound one after another to a desired amino acid sequence on and using, for example, a resin for protein or peptide synthesis by various condensation methods known per se. A variety of activation reagents known per se are preferably used, and for example, carbodiimide derivatives such as dicyclohexylcarbodiimide are preferably used for these reagents. When a product has a protective group, the objective product can be obtained by removing the protective group as appropriate.

A protein can be prepared by a method known to a person skilled in the art not only as a natural protein but also as a recombinant protein prepared by the use of gene recombination technology. The natural protein can be prepared, for example, by purifying protein extracts from cells using a column obtained by coupling an antibody, that is acquired by immunizing a small animal such as a mouse or rabbit with a prepared recombinant protein, to an appropriate adsorbent (CNBr activated agarose or tosyl activated agarose). On the other hand, the recombinant protein can be prepared by a conventional method, for example, by inserting DNA encoding the protein into an appropriate expression vector, introducing the vector into an appropriate cell, and purifying it from the transformed cells.

Cells used for producing a recombinant protein include, for example, plant cells, microbial cells such as E. coli and yeast, animal cells, and insect cells. Further, vectors for expressing the recombinant protein in the cells include, for example, a plasmid "pBI121" or "pBI101" for plant and yeast cells (product of Clontech), a plasmid "pET Expression system" (product of Stratagene) or "GST gene fusion Vectors" (product of Pharmacia) for E. coli, a pasmid "pMAM" for mammalian cells (product of Clontech), and a plasmid "pBacPAK8.9" for insect cells (product of Clontech). Insertion of DNA into a vector can be carried out by a conventional method described, for example, in Molecular Cloning (Maniatis et al., Cold Spring harbor Laboratory Press). Introduction of the vector into a host cell can be performed by a conventional method such as electroporation method, microinjection method, and particle gun method depending on the host cell.

The purification of a desired recombinant protein from the obtained transformed cells can be carried out by an appropriate combination of precipitation by salting out or with an organic solvent, ion exchange chromatography, affinity chromatography, column chromatography with immunoabsorbent, gel filtration, SDS gel electrophoresis, isoelectric focusing, and the like. Further, when the recombinant protein is expressed as a fusion protein with a label such as glutathione S-transferase, purification can also be effected by affinity chromatography utilizing an affinity for the label and the like.

In addition, the present invention provides DNA that codes for a protein utilized in relation to analysis of regulation and binding of p30-Rap1 binding and the like. The DNA is not particularly limited as long as it is capable of encoding a specific protein according to the present invention, and includes genomic DNA, cDNA, chemically synthesized DNA, and the like. The genomic DNA can be prepared by carrying out a polymerase chain reaction (PCR) using a genomic DNA prepared by a method known in the field as a template as well as primers prepared based on a specific nucleotide sequence of DNA as a template (for example, the nucleotide sequence shown in Fig. 1). In the case of cDNA, mRNA is prepared from cells by a conventional method (Molecular Cloning, Maniatis et al., Cold Spring harbor Laboratory Press) and subjected to reverse transcriptase reaction, followed by PCR using the same primers as above to prepare cDNA. In addition, genomic DNA or cDNA can also be prepared by constructing a genomic DNA library or cDNA library by a conventional method and then performing screening of this library with use of a probe synthesized based on, for example, the nucleotide sequence of the DNA (for example, the nucleotide sequence shown in Fig.1).

In the present specification, PCR generally indicates the method disclosed in Science, 239: 487 (1988), Saiki et al., the specification of U.S. Patent No. 4,683,195, etc., and for example, the method to amplify enzymically a desired nucleotide sequence in vitro. In general, PCR includes repetition of a cycle to perform primer extension synthesis using a pair of oligonucleotide primers that can hybridize preferentially to a template nucleic acid. Typically, the primers that can be used for PCR are complementary to the nucleotide sequence to be amplified in the template. For example, the primers that can be preferably used are complementary to the nucleotide sequence to be amplified at its both ends or adjacent to the nucleotide sequence to be amplified. In a typical case, it is preferred that the 5'-terminal side primer is selected so as to contain at least an initiation codon or so as to be amplified including the initiation codon and that the 3'-terminal side primer is selected so as to contain at least a stop codon or so as to be amplified including the stop codon. Examples of the primer include an oligonucleotide having preferably 5 or more nucleotides, more preferably 10 or more nucleotides, and still more preferably from 18 to 35 nucleotides.

PCR can be performed by a method known in this field or a method essentially equivalent to it or altered from it. In addition to the above references, for example, the methods described in R. Saiki, et al., Science, 230: 1350, 1985; H. A. Erlich ed., PCR Technology, Stockton Press, 1989; D. M. Glover et al. ed., "DNA Cloning," 2nd ed., Vol. 1, (The Practical Approach Series), IRL Press, Oxford University Press (1995); M. A. Innis et al. ed., "PCR Protocols: a guide to methods and applications," Academic Press, New York (1990); M. J. McPherson, P. Quirke and G. R. Taylor (Ed.), PCR: a practical approach, IRL Press, Oxford (1991); M. A. Frohman et al., Proc. Natl. Acad. Sci. USA, 85, 8998-9002 (1988), their modified or altered methods can be employed. Further, PCR can be performed using a commercially available kit suitable for it by following a protocol supplied from the kit manufacturer or its dealer.

In a typical case of PCR, for example, a template (for example, DNA synthesized from mRNA as a template; 1st strand DNA, etc.) and primers designed from the gene are mixed with a 10X reaction buffer (supplied with Tag DNA polymerase), dNTPs (a mixture of deoxynucleotide triphosphates, dATP, dGTP, dCTP, and dTTP), Tag DNA polymerase, and deionized water. The mixture is subjected to a PCR reaction cycle of from 25 to 60 times using an automated thermal cycler such as a GeneAmp 2400 PCR system, Perkin-Elmer/Cetus, under ordinary conditions for PCR cycle. The number of cycles for amplification may be adjusted to an adequate number as appropriate depending on each purpose. The conditions for PCR cycle are, for example, denaturation at 90 to 95°C for 5 to 100 sec, annealing at 40 to 60°C for 5 to 150 sec, and extension at 65 to 75°C for 30 to 300 sec, and preferably denaturation at 94°C for 15 sec, annealing at 58°C for 15 sec, and extension at 72°C for 45 sec. However, the reaction temperature and time for annealing can be adjusted to appropriate values depending on each experiment, and the reaction times for denaturation and extension can be adjusted each to an appropriate value depending on the chain length of the expected PCR product. In general, the reaction temperature for annealing is preferably changed according to the Tm value of the hybrid between the primer and the template DNA. Although an approximate guide for the reaction time for extension is generally about one minute per chain length of 1,000 bp, it is possible to select a shorter time in certain cases. The nucleotide sequence of the obtained DNA can be readily determined by using, for example, "Sequencer Model 310" (product of ABI).

In the present specification, "oligonucleotide" indicates relatively short single stranded or double stranded polynucleotides, preferably polydeoxynucleotides, and can be synthesized by a known method described in Angew. Chem. Int. Ed. Engl., Vol. 28, p. 716-734 (1989), for example, phosphotriester method, phosphodiester method, phosphite method, phosphoramidite method, and phosphonate method. Its synthesis in general is known to be advantageously performable on a modified solid support, and can be performed using, for example, an automated synthesizer, and the apparatus is commercially available. The oligonucleotide may contain one or more modified nucleotides, for example, a naturally unusual nucleotide such as inosine or tritylated nucleotide, and in some cases, a nucleotide labeled with a marker.

The identification of a specific nucleic acid can make use of hybridization technology. The hybridization can be carried out by a method described in the references that disclose the above "gene recombination technology," its essentially equivalent method, or its modified method. For example, hybridization is performed by transferring a sample containing a nucleic acid such as DNA to a carrier including a membrane such as a nylon filter, and subjecting it to denaturation treatment, immobilization treatment, washing treatment, and the like, as necessary, followed by allowing the materials having been transferred to the carrier (for example, membrane) to react with a labeled probe DNA fragment having been denatured as necessary in a buffer for hybridization.

Although the hybridization treatment is performed generally at ca. 35 to ca. 80°C, more preferably at ca. 50 to ca. 65°C for ca. 15 min to ca. 36 hours, and more preferably for ca. one to ca. 24 hours, it can be carried out by selecting an optimal condition as appropriate. For example, the hybridization treatment is carried out at ca. 55°C for ca. 18 hours. The buffer for hybridization can be used by selecting from the buffers conventionally used in this field; for example, Rapid hybridization buffer (product of Amersham) or the like is available. An example of the denaturation treatment of the transferred carrier (for example, membrane) is a method that uses an alkali denaturation solution, and after the treatment, it is preferred to treat with a neutralizing solution or a buffer. The treatment for immobilization on the carrier (for example, membrane) is carried out by baking usually at ca. 40 to ca. 100°C, and more preferably at ca. 70 to ca. 90°C for ca. 15 min to ca. 24 hours, and more preferably for ca. one hour to ca. four hours but can be carried out by selecting a desirable condition as appropriate. For example, the carrier such as filter is baked at ca. 80°C for ca. 2 hours for immobilization. The washing treatment of the transferred carrier (for example, membrane) can be conducted by washing with a washing solution generally used in this field, for example, 50 mM tris-HCl buffer, pH 8.0, containing 1 M NaCl, 1mM EDTA, and 0.1% sodium dodecyl sulfate (SDS). As the carrier including membrane such as a nylon filter, a carrier selected from among carriers conventionally used in this field can be used.

The above alkali denaturation solution, neutralization solution, and buffer selected from among those conventionally used in this field can be used. An example of the alkali denaturation solution is a solution containing 0.5 M NaOH and 1.5 M NaCl. An example of the neutralization solution is 0.5 M Tris-HCl buffer, pH 8.0, containing 1.5 M NaCl and an example of the buffer solution is 2xSSPE (0.36 M NaCl, 20 mM NaH₂PO₄ and 2 mM EDTA). Prior to the hybridization treatment, it is preferred that the transferred carrier (for example, membrane) is subjected to pre-hybridization treatment as needed in order to prevent non-specific hybridization reaction. This pre-hybridization treatment can be performed, for example, by immersing in a pre-hybridization solution [50% formamide, 5xDenhardt's solution (0.2% bovine serum albumin, 0.2% polyvinyl pyrrolidone), 5xSSPE, 0.1% SDS, 100 µg/ml heat denatured salmon sperm DNA] or the like and allowing to react at ca. 35 to ca. 50°C and preferably at ca. 42°C for ca. 4 to ca. 24 hours and preferably for ca. 6 to ca. 8 hours. One of ordinary skill in the art is able to determine more desirable conditions for such conditions by appropriately repeating the experiments. The denaturation of labeled probe DNA fragment that is used for hybridization can be performed, for example, by heating at ca. 70 to ca. 100°C and preferably at ca. 100°C for ca. one to ca. 60 min and preferably for ca. 5 min. Although the hybridization can be performed by a method known per se or its comparable method, a stringent condition in this specification implies that the sodium concentration is, for example, from ca. 15 to ca. 50 mM, preferably from ca. 19 to ca. 40 mM, and more preferably from ca. 19 to ca. 20 mM, and the temperature is, for example, from ca. 35 to ca. 85°C, preferably from ca. 50 to ca. 70°C, and more preferably from ca. 60 to ca. 65°C.

After completion of hybridization, labeled probe other than the labeled probe DNA fragment that underwent specific hybridization reaction is removed by washing the carrier such as a filter thoroughly, which then allows to proceed to detection treatment. The washing treatment of the carrier such as a filter can be performed with a solution selected from among what is generally used in this field, and for example, can be performed by washing with a solution of 0.5xSSC (0.15 M NaCl, 15 mM citric acid) containing 0.1% SDS, and the like.

The hybridized nucleic acid can be typically detected by autoradiography, while a method appropriately chosen from among popular methods in this field may also be used for detection. A nucleic acid band corresponding to the detected signal is suspended in a suitable buffer, for example, SM solution (50 mM Tris-HCl buffer, pH 7.5, containing 100 mM NaCl and 10 mM MgSO₄). Then this suspension is appropriately diluted, and the specific nucleic acid is isolated, purified, and subjected to further amplification treatment.

The process for screening of a target nucleic acid from a nucleic acid sample including a gene library, cDNA library, and the like by hybridization can be carried out repeatedly. Human-derived cDNA libraries that are cloned, for example, various cDNA libraries from tissues and cultured cells of human origin (tissues and cells such as , in particular, human kidney, brain, pineal body, pituitary posterior lobe, nerve cells, retina, retinal vascular cells, retinal nerve cells, thymus, blood vessel, endothelial cells, vascular smooth muscle cells, blood cells, macrophage, lymphocytes, testis, ovary, uterus, intestine, heart, liver, pancreas, small intestine, large intestine, gingiva-related cells, skin-related cells, glomerular cells, renal tubular cells, and connective tissue cells, and further various tumor tissues and cancer cells) can be used. Commercially available cDNA libraries derived from various tissues may also be directly utilized for a cDNA library that is used as a template; for example, cDNA libraries marketed from Stratagene Corp., Invitrogene Corp., Clontech Laboratories Inc. can be used. In a typical example, a gene library prepared from human tissues and cells such as a human P1 artificial chromosome genomic library (Human Genome Mapping Resource Center) and human tissue cDNA library (for example, available from Clontech, etc.) can be used. Human genomic DNA libraries or human derived cDNA libraries constructed from various human tissues or cultured cells can be screened by using a probe. Labeling a probe or the like with a radioisotope or the like can be conducted by using a commercially available labeling kit, for example, a random prime DNA labeling kit (Boehringer Mannheim). For example, DNA for a probe can be labeled with [α-³²P]dCTP (Amersham) and the like using the random-priming kit (Pharmacia LKB, Uppsala) and the like to obtain a radioactive probe.

Phage particle, recombinant plasmid, recombinant vector, and the like containing a specific nucleic acid can be purified and separated by a conventional method in the field, for example, by glycerol gradient ultracentrifugation (Molecular Cloning, a laboratory manual, ed. T. Maniatis, Cold Spring Harbor Laboratory, 2nd ed. 78, 1989) and electrophoresis. From phage particle and the like, DNA can be purified and separated by a conventional method in the field. For example, obtained phage or the like is suspended in a TM solution (50 mM Tris-HCl buffer, pH 7.8, containing 10 mM MgSO₄) or the like, treated with DNase I, RNase A, and the like, then added with a mixture of 20 mM EDTA, 50 µg/ml Proteinase K, and 0.5% SDS, and the like, and incubated for ca. one hour at ca. 65°C. After this is extracted with phenol and with diethyl ether, DNA is precipitated by ethanol precipitation. Subsequently, the obtained DNA is washed with 70% ethanol, then dried, and dissolved in a TE solution (10 mM Tris-HCl buffer, pH 8.0, containing 10 mM EDTA) to yield DNA solution. Further a target DNA can be obtained in a large amount by subcloning and the like, and for example, the subcloning can be carried out with use of a plasmid vector and the like using E. coli as a host. The DNA obtained by such subcloning can also be purified and separated by the methods such as centrifugation, phenol extraction, and ethanol precipitation as described above.

In the present specification, when the term "high homology" is used, though it depends on the length of the target sequence, it may represent, for example, 50% or higher, further 60% or higher, preferably 70% or higher, more preferably 80% or higher, in a particular case 95% or higher, and most preferably 97% or higher homology. The "equally effective nucleotide sequence" may be a nucleotide sequence that hybridizes, for example, under a stringent condition, to a nucleotide sequence having the sequence of concern. For example, this includes nucleotide sequences that hybridize to a 5 or more consecutive nucleotide sequence, preferably a 10 or more consecutive nucleotide sequence, more preferably a 15 or more consecutive nucleotide sequence, and still more preferably 20 or more consecutive nucleotide sequence, and encode an amino acid sequence essentially equivalent to the polypeptide, and the like among the nucleotide sequences. The nucleic acid may also be obtained by chemical synthesis. In this case, its fragments are chemically synthesized, and these may be ligated by an enzyme.

In the present specification, the obtained nucleic acid (including DNA) such as the PCR product is usually subjected to electrophoresis on one to 2% agarose gel, cut out from the gel as a specific band, and extracted using a commercially available kit such as gene clean kit (Bio 101). The extracted DNA is cleaved by an appropriate restriction enzyme, purified as needed, and further phosphorylated at the 5' terminus using T4 polynucleotide kinase and the like as necessary. Then, this is ligated to an appropriate plasmid vector such as a pUC vector like pUC18 and used to transform an appropriate competent cell. The cloned PCR product is analyzed for its nucleotide sequence. For the cloning of the PCR product, commercially available plasmid vectors such as p-Direct (product of Clontech), pCR-Script™ SK(+) (product of Stratagene), pGEM-T (product of Promega), and pAmp™ (product of Gibco-BRL) can be used. The transformation of a host cell can be carried out by, for example, the use of a phage vector, a method known in this field such as a calcium method, rubidium/calcium method, calcium/manganese method, TFB highly efficient method, FSB frozen competent cell method, rapid colony method, or electroporation, or a method practically equivalent to these methods (D. Hanahan, J. Mol. Biol., 166: 557, 1983, etc.). For the isolation of the target DNA, polymerase chain reaction coupled reverse transcription (RT-PCR) and rapid amplification of cDNA ends (RACE) can be applied. RACE can be performed according to a method described in, for example, "PCR Protocols" M. A. Innis et al. (eds.) (M. A. Frohman, "A guide to methods and applications"), pp. 28-38, Academic Press, New York (1990).

DNA can be cloned as needed, and for example, a plasmid, λ phage, cosmid, P1 phage, F factor, and YAC can be utilized. A preferred example that can be used is a vector derived from λ phage such as Charon 4A, Charon 21A, λgt10, λ gt11, λDASHII, λFIXII, λEMBL3, or λZAPTT™ (product of Stratagene). Further, the obtained DNA can be inserted into an appropriate vector described below in detail such as plasmid pEX, pMAMneo, or pKG5, and expressed in an appropriate host cell described below in detail such as E. coli, yeast, CHO cell, or COS cell. The DNA fragment can also be inserted into an appropriate vector as it is or as a DNA fragment added with an appropriate regulatory sequence, introduced into an animal, thereby producing a transgenic animal that expresses the specific gene. Examples of the animal are mammals such as a mouse, rat, rabbit, guinea pig, and cattle. Preferably, a transgenic animal can be produced by introducing the DNA fragment into a fertilized egg of an animal such as a mouse. Confirmation of the specific gene product can be performed with an animal cell suitable for it such as 293T cell or COS-1 cell transfected with the foreign gene.

The introduction of a foreign gene into an animal cell such as a mammalian cell can be carried out by a method known in the field or a method essentially the same as that. For example, this includes a calcium phosphate method (for example, F. L. Graham et al., Virology, 52: 456, 1973), DEAE-dextran method (for example, D. Warden et al., J. Gen. Virol., 3: 371, 1968), electroporation method (for example, E. Neumann et al., EMBO J. 1: 841, 1982), microinjection method, liposome method, virus infection method, and phage particle method. The gene product produced by an animal cell transfected with a specific gene can also be analyzed for it.

The plasmid into which a specific gene (DNA obtained in the present invention, etc.) is inserted may be any plasmid as long as it is one that allows the DNA to be expressed in a host cell that is used conventionally in gene engineering (for example, a prokaryotic cell host such as E. coli or Bacillus subtilis, a eukaryotic cell host such as yeast, 293T cell, CHO cell, or COS cell, and an insect cell such as Sf21). Of course, it is also possible to use a plasmid selected from those attached to a commercially available kit or reagent. In the sequence of these plasmids, for example, a modified codon that is suitable for expression in the selected host cell may be contained, restriction enzyme sites may also be provided, and a regulatory sequence, a promotion sequence, and the like that facilitate the expression of the target gene, linker, adapter, and the like that are useful for binding of the target gene, and further, sequences useful for regulating antibiotic resistance and the like, regulating metabolism, selection and the like (sequences coding for a hybrid protein and a fusion protein), and the like may be contained. Preferably, an adequate promoter, for example, a tryptophan promoter (trp), lactose promoter (lac), tryptophan-lactose promoter (tac), lipoprotein promoter (lpp), and λ phage P_{L} promoter for a plasmid hosted in E. coli, SV40 late promoter, MMTV LTR promoter, RSV LTR promoter, CMV promoter, and SRα promoter for a plasmid hosted in an animal cell, and GAL1 and GAL10 promoters for a plasmid hosted in yeast can be used. Furthermore, a regulatory system such as CYC1, HIS3, ADH1, PGK, PHO5, GAPDH, ADC1, TRP1, URA3, LEU2, ENO, TP1, or AOX1 can also be used.

In order to promote transcription of DNA encoding a desired polypeptide, an enhancer can be inserted into a vector, and the enhancer listed is an element generally consisting of ca. 10 to 100 bp and having cis effect that acts on a promoter to promote transcription. Many enhancers are known to originate in mammalian genes such as globin, elastase, albumin, α-fetoprotein, and insulin genes. A representative enhancer obtained from a virus infectious to eukaryotic cells is suitably used, and examples of enhancers are, for example, SV40 enhancer present in the late region of replication origin (100 to 270 bp), the enhancer of early promoter of cytomegalovirus, the enhancer present in the late region of the replication origin of polyoma virus, and the enhancer of adenovirus. Furthermore, a signal sequence adequate for a host can be added as needed, and a signal sequence known to one of ordinary skill in the art can be used.

Plasmids hosted in E. coli include, for example, pBR322, pUC18, pUC19, pUC118, pUC119, pSP64, pSP65, pTZ-18R/-18U, pTZ-19R/-19U, pGEM-3, pGEM-4, pGEM-3Z, pZEM-4Z, pZEM-5Zf(-), and pBluescript KS™ (obtained from Stratagene). The plasmid vectors suitable for expression in E. coli are, for example, pAS, pKK223 (obtained from Pharmacia), pMC1403, pMC931, pKC30, and pRSET-B (obtained from Invitrogen). The plasmids hosted in animal cells are, for example, SV40 vector, polyoma virus vector, vaccinia virus vector, and retrovirus vector, and specifically, pcD, pcD-SRα, CDM8, pCEV4, pME18S, pBC12BI, and pSG5 (obtained from Stratagene). The plasmids hosted in yeast are YIp type vector, YEp type vector, YRp type vector, and YCp type vector, and for example, pGPD-2 is listed. When the host cell is E. coli, cells originating from the E. coli K12 strain are named; for example, NM533, XL1-Blue, C600, DH1, DH5, DH11S, DH12S, DH5α, DH10B, HB101, MC1061, JM109, and STBL2. Cells originating from B834 include BL21(DE3)pLysS and the like. When the host cell is yeast, yeast strains such as Saccharomyces cerevisiae, Shizosaccharomyces prombe, Pichia pastoris, Kluyveromyces strains, Candida, Trichoderma reesia, and other yeast strains are included.

When the host cells are animal cells, cells include, for example, COS-7 cell originating from fibroblast of an African green monkey, COS-1 cell, CV-1 cell, 293 cell originating from a human kidney cell, A431 cell from a human epidermal cell, 205 cell originating from a human colon, COP cell originating from a mouse fibroblast, MOP cell, WOP cell, CHO cell originating from a Chinese hamster cell, CHO DHFR⁻ cell, human Hela cell, C127 cell originating from a mouse cell, NIH 3T3 cell originating from a mouse cell, mouse L cell, 9BHK, HL-60, U937, HaK, Jurkat cell, other cell lines obtained by transformation, normal diploid cells, and cell lines derived from primary culture of tissues in vitro. Insect cells to be used include Spodoptera frugiperda (caterpillar), Aedes aegypti (mosquito), Aedes albopictus (mosquito), Drosophila melanogaster (fruitfly), and larva or cultured cell of Bombyx mori, e.g. BM-N cell, in combination with Bombyx mori nuclear polyhedrosis virus, viruses derived from it or other suitable viruses as a vector (for example, Luckow et al., Bio/Technology, 6. 47-55 (1988); Setlow, J. K. et al. (eds.), Genetic Engineering, Vol 8, pp. 277-279, Plenum Publishing, 1986; Maeda et al., Nature, 315, pp. 592-594 (1985)). It is also possible to use plant cells as host cells by making use of Agrobacterium tumefaciens, etc., and these are widely known in the field in conjunction with vectors suitable for them. In the gene engineering method of the present invention, a restriction enzyme, reverse transcriptase, DNA-modifying enzyme and DNA-cleaving enzyme for modification or conversion to a structure suitable for cloning of a DNA fragment, DNA polymerase, terminal nucleotidyltransferase, DNA ligase, and the like known in this field or used conventionally can be used. The restriction enzyme includes those described in the following literature: R. J. Roberts, Nucleic Acids Res., 13: r165, 1985; S. Linn et al. ed. Nucleases, p. 109, Cold Spring Harbor Lab., Cold Spring Harbor, New York, 1982; R. J. Roberts, D. Macelis, Nucleic Acids Res., 19: Suppl. 2077, 1991

The transformant transformed by an expression vector containing a nucleic acid encoding the polypeptide (or protein) according to the present invention is subjected to repeated cloning using an appropriate selection marker as needed, thereby allowing to obtain a cell line stably possessing a high expression ability. When a dhfr gene is used as a selection marker for a transformant that makes use of an animal cell as a host, the DNA encoding the polypeptide of the present invention can be amplified by gradually increasing MTX concentration during cell culture and selecting a resistant cell line, thereby enabling to obtain a cell line with higher expression. It is possible for the transformant of the present invention to produce and accumulate the target substance by being cultured under the conditions that allow the nucleic acid encoding the polypeptide of the present invention to be expressed. The transformant can be cultured in a medium conventionally used in this field. For example, a liquid medium is preferably used for the transformants of prokaryotic hosts such as E. coli and Bacillus subtilis and of yeast host and the like. In the medium, carbon sources, nitrogen sources, inorganic substances, and others which are necessary to culture the transformants are supplied. The carbon sources are, for example, glucose, dextrin, soluble starch, and saccharose, the nitrogen sources are inorganic or organic substances such as ammonium salts, nitrates, corn steep liquor, peptone, casein, meat extracts, malt extracts, soy bean cake, and potato extracts, the inorganic substances are, for example, calcium chloride, sodium dihydrogenphosphate, magnesium chloride, and calcium carbonate. In addition, yeast extracts, vitamins, casamino acid, and growth promoting factors may be added. Further, an agent such as 3-β indolylacrylic acid can be added as needed in order to allow a promoter to work efficiently. It is desirable that pH of the medium is from ca. 5 to ca. 8.

In the case of E. coli, the culture is usually carried out at ca. 15 to ca. 45°C for ca. 3 to ca. 75 hours, and aeration and agitation can also be applied as needed. When a transformant whose host cell is an animal cell is cultured, the medium to be used is, for example, MEM medium, RPMI-1640 medium, or DMEM medium that contains ca. 5 to ca. 20% fetal calf serum. It is desirable that pH of the medium is from ca. 6 to ca. 8. The culture is usually carried out at ca. 30 to ca. 40°C for ca. 15 to ca. 72 hours, and aeration and agitation are applied as needed. Although the transformant expressing a specific gene product can be used not only as it is but also as its cell homogenates, the specific gene product can also be isolated for use. When extracted from the above cultured cells, the bacteria or the cells are collected by a known method after culturing, suspended in an appropriate buffer, and disrupted by sonication, lysozyme and/or freeze thawing, and the like, followed by obtaining crude extracts by centrifugation or filtration. These methods can be appropriately employed. A protein denaturing agent such as urea or guanidine hydrochloride and a surface active agent such as Triton X-100 (trade name) or Tween-20 (trade name) may be added to the buffer. When the target product is secreted into the culture medium, after the culture, the bacteria or the cells and the supernatant are separated by a method known per se, and the supernatant is collected.

The target product contained in the culture supernatant or the extracts obtained in this way can be purified by an appropriate combination of separation and purification methods known per se, and can be obtained by purification by, for example, salting out such as ammonium sulfate precipitation method, gel filtration method using Sephadex and the like, ion exchange chromatography using, for example, a carrier having diethylaminoethyl group or carboxymethyl group, and the like, hydrophobic chromatography using, for example, a carrier having a hydrophobic group such as butyl group, octyl group, or phenyl group, dye-ligand gel chromatography, electrophoresis, dialysis, ultrafiltration, affinity chromatography, and high performance liquid chromatography. Preferably, the purification and separation can be performed by polyacrylamide gel electrophoresis, immobilized ligand affinity chromatography, and the like. For example, gelatin-agarose affinity chromatography, heparin-agarose chromatography and the like are included. The obtained protein (may include peptide or polypeptide) can be immobilized to an appropriate carrier or solid phase by a method known in enzyme immunoassay or the like. The solid phased protein and the solid phased peptide can be advantageously used for binding assay and screening of substances.

Relevant proteins, their fragments, and further, nucleic acids including DNA (include mRNAs and oligonucleotides) that are disclosed in the present specification are used alone or in an organized manner, and further in combination with antisense technology, an antibody including monoclonal antibody, a transgenic animal, and the like as appropriate, thereby allowing them to be applied to genomics and proteomics. In addition, there is an application for an RNAi technology (RNA interference) using a double stranded RNA (dsRNA). Thus, it becomes possible to carry out genetic polymorphism analysis centered around SNP (single nucleotide polymorphism), gene expression analysis with nucleic acid array and protein array, gene function analysis, analysis of protein-protein interaction, analysis of related genes, and agrochemical analysis. For example, conduct of sample analysis by the nucleic acid array technology makes use of hybridization after arraying DNAs using cDNA library or those obtained by PCR technology on a substrate at a high density using a spotter.

The arraying of nucleic acids can be carried out by applying DNAs to each specific position on a substrate such as a slide glass, silicon plate or plastic plate using a needle or a pin or by ink jet printing technology. Signals resulting from hybridization on the nucleic acid array are detected to acquire data. The signals may be those obtainable from a label such as fluorescent dye (for example, Cy3, Cy5, BODIPY, FITC, Alexa Fluor dyes (trade name), Texas red (trade name), etc.). The detection can make use of a laser scanner, and the data obtained may be processed by a computer system provided with a program according to an appropriate algorithm. In a protein array technology, tagged proteins produced by recombinant expression may be utilized, and technologies such as two-dimensional electrophoresis (2-DE), mass spectrometry (MS) including enzyme-digested fragments (this includes techniques such as electrospray ionization (ESI) and matrix-assisted laser desorption/ionization (MALDI), and MALDI-TOF analyzer, ESI-3 linear quadrupole mass spectrometer analyzer, ESI-ion trap analyzer, and the like may be used), dyeing technology, isotope labeling and analysis, and image processing technology can be used. Accordingly, the present invention may include software, database, and the like that are related to the above p30, its related analogues and derivatives, and the like as well as their antibodies.

When a specific DNA (for example, DNA encoding p30 or Rap1) is transferred into a target animal, it is generally advantageous to use the DNA as DNA fragments or by coupling it to the downstream of a promoter capable of promoting its expression in animal cells. For example, when the DNA is introduced into a mouse, a gene construct in which the DNA is bound to the downstream of various promoters capable of promoting the expression of the highly homologous animal-derived DNA in the animal cell is introduced into a fertilized egg of the target animal, e.g. fertilized mouse egg, by microinjection, thereby enabling to produce a transgenic mouse that synthesizes a large amount of its protein. The mouse to be used is not particularly limited to a pure line mouse, and for example, C57BL/6, Balb/C, C3H, and (C57BL/6xDBA/2)F₁(BDF₁) are included. The promoters that can be preferably used are promoters originating from viruses and ubiquitous expression promoters such as metallothionein. Further, when the DNA is introduced, the DNA can also be recombined into a recombinant retrovirus to use for its introduction. Preferably, the fertilized mouse egg introduced with the target DNA can be grown by an adoptive mouse such as ICR.

The transfer of the DNA at the stage of a fertilized egg cell (for example, DNA encoding p30 or Rap1) assures to allow the DNA to exist in all of the embryonic cells and somatic cells of the target animal. The presence of DNA encoding the protein in the embryonic cells of the animal to be produced after the DNA transfer implies that the progenies of the produced animal contain DNA encoding the protein in all of their embryonic cells and somatic cells. The progenies of this kind of animal that inherit the gene have a possibility that the protein can be expressed in all of their embryonic cells and somatic cells.

The animal introduced with the DNA can be bred successively as the DNA-containing animal under conventional farming conditions while confirming that the gene is stably retained after mating. Further, mating of a pair of animals both possessing the target DNA makes it possible to produce a homozygotic animal having the introduced gene both on a homologous chromosome, and mating of a pair of these animals makes it possible to successively breed animals whose progenies all possess the DNA. The animal introduced with the DNA is useful as an animal for screening of an inhibitor of the protein and the like because the protein is expressed at a high level. It is also useful as an animal for screening of an antisense oligonucleotide such as antisense DNA that can inhibit the expression of the gene, and the like.

This transgenic animal can also be used as a source of cells for tissue culture. For example, p30-related proteins can be analyzed, for example, by directly analyzing DNA or RNA in the transgenic mouse tissues or by analyzing proteins and tissues expressed by the gene. The tissue cells producing the protein are cultured according to a standard tissue culture technique, and their use makes it possible to study the functions of p30-related proteins in cells originating from, for example, brain, thymus, vascular cells such as endothelial cells, blood cells, testis, brain, intestine, kidney, and other cells. Further, the use of these cells can contribute, for example, to the development of a pharmaceutical that enhances functions of various tissues. Furthermore, when a cell line with high expression is in hand, the protein can be isolated and purified from the cells. Technology concerned with a transgenic mouse and the like can be pursued by the methods described in references, for example, Brinster, R. L., et al.,; Proc. Natl. Acad. Sci. USA, 82: 4438, 1985; Costantini, F. & Jaenisch, R. (eds.): Genetic manipulation of the early mammalian embryo, Cold Spring Harbor Laboratory, 1985, the methods described in references cited therein, or their modified methods.

As used herein, the term "monoclonal antibody" is used in the broadest sense and may cover a single species of desirable monoclonal antibodies against p30 proteins, p30-constituent polypeptides or p30 protein-related peptide fragments, and monoclonal antibody compositions (or mixtures) having a specificity to various epitopes thereof, further monovalent or polyvalent antibodies, polyclonal antibodies or monoclonal antibodies, and also those which are intact molecules or fragments and derivatives thereof, including F(ab') ₂, Fab', and Fab fragments, and also chimeric antibodies, hybrid antibodies each having at least two antigen- or epitope-binding sites, or bispecific recombinant antibodies (e.g., quadromes, triomes, etc.), interspecies hybrid antibodies, anti-idiotypic antibodies and those which have been chemically modified or processed and must be regarded as derivatives of these antibodies and further which may be produced either by adopting cell fusion or hybridoma techniques or antibody engineering or by using synthetical or semisynthetical techniques in a known manner, which may be prepared either by the known conventional methods in view of antibody production or by recombinant DNA techniques, and which have neutralizing or binding properties with respect to the target antigen substances or target epitopes described and defined herein. Particularly preferred antibodies of the present invention are those capable of specifically recognizing a polypeptide selected from the N-terminal region of p30.

Monoclonal antibodies prepared against antigenic substances are produced by any method capable of providing production of antibody molecules by a series of cell lines in culture. The modifier "monoclonal" indicates the character of the antibody as being obtained from a substantially homogeneous population of antibodies, and is not to be construed as requiring production of the antibody by any particular method. The individual antibodies are those containing a population of identical antibodies except for possible naturally occurring mutations that may be present in minor amounts. Monoclonal antibodies are highly specific, being directed against a single antigenic site. In contrast to conventional (polyclonal) antibody preparations which typically include different antibodies directed against different antigenic determinants (epitopes), each monoclonal antibody is directed against a single determinant on the antigen. In addition to their specificity, the monoclonal antibodies are advantageous in that they are synthesized by the hybridoma culture, uncontaminated or little contaminated by other immunoglobulins. The monoclonal antibodies included within the scope of the invention include hybrid and recombinant antibodies. They are obtainable by substituting a constant domain of an antibody for a variable domain, or a heavy chain for a light chain, by substituting a chain from one species with a chain from another species, or by fusing to heterogeneous proteins, regardless of species of origin or immunoglobulin class or subclass designation, so long as they exhibit the desired biological activity (e.g., U.S. Pat. No. 4,816,567; Monoclonal Antibody Production Techniques and Applications, pp.79 to 97, Marcel Dekker, Inc., New York, 1987; etc.).

Preferable techniques for producing monoclonal antibodies include, for example, the methods using hybridoma cells (G. Kohler and C. Milstein, Nature, 256, pp.495 to 497 (1975)); the methods using human B cell hybridomas (Kozbor et al., Immunology Today, 4, pp.72 to 79 (1983); Kozbor, J. Immunol., 133, pp.3001 (1984); Brodeur et al., Monoclonal Antibody Production Techniques and Applications, pp.51 to 63, Marcel Dekker, Inc., New York (1987); triome methods; EBV-hybridoma methods (Cole et al., Monoclonal Antibodies and Cancer Therapy, Alan R. Liss, Inc., pp.77 to 96 (1985)) (techniques for production of human monoclonal antibodies); U.S. Pat. No. 4,946,778 (techniques for production of single-chain antibodies), as well as the following documents: S. Biocca et al., EMBO J, 9, pp.101 to 108 (1990); R.E. Bird et al., Science, 242, pp.423 to 426 (1988); M.A. Boss et al., Nucl. Acids Res., 12, pp.3791 to 3806 (1984); J. Bukovsky et al., Hybridoma, 6, pp.219 to 228 (1987); M. DAINO et al., Anal. Biochem., 166, pp.223 to 229 (1987); J.S. Huston et al., Proc. Natl. Acad. Sci. USA, 85, pp.5879 to 5883 (1988); P.T. Jones et al., Nature, 321, pp.522 to 525 (1986); J.J. Langone et al. (ed.), "Methods in Enzymology", Vol. 121 (Immunochemical Techniques, Part I: Hybridoma Technology and Monoclonal Antibodies), Academic Press, New York (1986); S. Morrison et al., Proc. Natl. Acad. Sci. USA, 81, pp.6851 to 6855 (1984); V.T. Oi et al., BioTechniques, 4, pp.214 to 221 (1986); L. Riechmann et al., Nature, 332, pp.323 to 327 (1988); A. Tramontano et al., Proc. Natl. Acad. Sci. USA, 83, pp.6736 to 6740 (1986); C. Wood et al., Nature, 314, pp.446 to 449 (1985); Nature, 314, pp.452 to 454 (1985), or documents quoted therein (the disclosures of which are incorporated herein by reference). The antibody of the present invention can be used for analysis and detection of products expressed by the gene as well as for various applications.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they have the desirable biological activity (U.S. Pat. No. 4,816,567; Morrison et al., Proc. Natl. Acad. Sci. USA, 81, pp.6851 to 6855 (1984)).

Described herein below is the production of antibodies, including embodiments of monoclonal antibodies. It goes without saying that the monoclonal antibody to be used in the present invention may be a product obtained by adoptions of cell fusion techniques (e.g., Kohler, G. & Milstein, C., Nature, 256: 495-497 (1975), etc.) with myeloma cells. The monoclonal antibodies to be used in the present invention can be produced by the following processes:
1. Preparation of immunogenic antigens (immunogens)
2. Immunization of animals with immunogenic antigens
3. Preparation of myeloma cells
4. Cell fusion between antibody-producing cells and myeloma cells
5. Selection and cloning of hybridomas (hybrid cells)
6. Production of monoclonal antibodies

1. Preparation of immunogenic antigens
   The antigen as used herein includes not only p30 polypeptides or isolated fragments that are derived therefrom, as disclosed herein above, but also suitable synthetic oligopeptides which are chemically synthesized, based on determined sequence information on the sequenced p30 protein. Representative examples are peptides each having at least 5 consecutive amino acids of the amino acid residues present in Fig. 1. This includes selection of characteristic sequences, for example, selection of appropriate portions from the amino acid sequence on the N-terminal side and the like.
   Although the antigen may be used to immunize animals after being mixed with a suitable adjuvant without any modifications, it can be used after formation of immunogenic conjugates. For example, the antigen for such an immunogen may be selected from fragmented molecules derived from the proteins, synthetic polypeptide fragments which are prepared via selecting characteristic sequence areas based on the amino acid sequences followed by design and chemical synthesis. The fragments may be coupled with various carrier proteins via suitable coupling agents to form immunogenic conjugates such as hapten-proteins. The immunogenic conjugates can be used to design monoclonal antibodies that can react with (or recognize) specific sequences exclusively. A cysteine residue or others can be added beforehand to the polypeptide thus designed so as to facilitate preparation of an immunogenic conjugate. To couple with a carrier protein or the like, the carrier protein is first activated. This activation may include incorporation of an activated coupling group thereinto, etc. The activated coupling groups include (1) active ester or active carboxyl groups such as a nitrophenyl ester group, a pentafluorophenyl ester group, a 1-benzotriazol ester group, and an N-succinimido ester group; (2) active dithio groups such as a 2-pyridyldithio group, etc. The carrier proteins include keyhole limpet haemocyanin (KLH), bovine serum albumin (BSA), ovalbumin, globulin, polypeptides such as polylysine, and bacterial cell components such as BCG.
2. Immunization of animals with immunogenic antigens
   Animals can be immunized by methods known to those skilled in the art and according to techniques as described in, for example, Shigeru Muramatsu et al. ed., "Jikken-Seibutsu-Gaku-Koza 14, Men-eki-Seibutsu-Gaku", Maruzen Co. Ltd., Japan, (1985); The Japanese Biochemical Society (Ed.), "Zoku-Seikagaku-Jikken-Kouza 5, Men-eki-Seikagaku-Kenkyuho", Tokyo Kagaku Dojin Co. Ltd., Japan (1986); The Japanese Biochemical Society (Ed.), "Shin-Seikagaku-Jikken-Kouza 12, Bunshi-Men-eki-Gaku III (Kougen-Koutai-Hotai)", Tokyo Kagaku Dojin Co. Ltd., Japan (1992) (all in Japanese); etc., the disclosures of which are hereby incorporated by reference. Immunization can be performed in a mammal, for example, by one or more injections of an immunizing agent (and, if desired, an adjuvant). Typically, the immunizing agent and/or adjuvant will be injected in the mammal by multiple subcutaneous or intraperitoneal injections. The immunizing agent may include the aforementioned antigen peptides or those containing any of related peptide fragments thereof. It may be useful to conjugate the immunizing agent to a protein known to be immunogenic in the mammal being immunized. Examples of such immunogenic proteins which may be employed include the aforementioned carrier proteins. The adjuvant to be used with the antigen includes Freund's complete adjuvant, Ribi adjuvant, pertussis vaccine, BCG, lipid A, liposome, aluminium hydroxide, silica, etc. The Immunization is carried out with suitable animals, including mice such as BALB/c, hamsters, and others. The antigen dose is, for example, about 1 to 400µg/animal for mice. Generally, the antigen is injected intraperitoneally or subcutaneously into a host animal, followed by additional immunization by repeated courses wherein intraperitoneal, subcutaneous, intravenous or intramuscular administrations are carried out approximately 2 to 10 times at 1- to 4-week intervals, preferably 1- to 2-week intervals. For immunization, BALB/c mice, as well as F1 mice between BALB/c mice and other mice, etc. can be used. As required, the levels of animal immunization can be assessed by constructing an antibody titer measuring system and measuring the titer of an antibody. The antibody of the present invention may include those obtainable from such immunized animals, for example, anti-serum, polyclonal antibodies, etc.
3. Preparation of myeloma cells (plasmacytoma cell lines)
   Immortal cell lines (tumor cell lines) to be used for cell fusion can be selected from non-immunoglobulin-producing cell lines. The cell lines to be used for cell fusion may include, for example, P3-NS-1-Ag4-1 (NS-1, Eur. J. Immunol., 6: 511-519, 1976), SP-2/0-Ag14 (SP-2, Nature, 276: 269 to 270,1978), mouse myeloma MOPC-21 cell line-derived P3-X63-Ag8-U1 (P3U1, Curr. topics Microbiol. Immunol., 81: 1 to 7, 1978), P3-X63-Ag8 (X63, Nature, 256: 495-497, 1975), P3-X63-Ag8-653 (653, J. Immunol., 123: 1548-1550, 1979), etc. 8-Azaguanine resistant mouse myeloma cell lines can be sub-cultured in a cell culture medium, such as Dulbecco's modified Eagle's medium (DMEM) and RPMI-1640, supplemented with antibiotics such as penicillin and amikacin, fatal calf serum (FCS) or others and 8-azaguanine (for example, 5 to 45µg/ml). The specified number of cell lines can be prepared by passage the normal medium two to five days prior to cell fusion. After the frozen and preserved cell lines to be used have been completely thawed at about 37°C and have been washed on the normal medium such as RPMI-1640 three or more times, the cell lines may be cultured on the normal medium to provide a desired number of cell lines.
4. Cell fusion between antibody-producing cells and myeloma cells
   After animals such as mice are immunized according to the above step 2, their spleens are taken out in two to five days from final immunization, and the spleen cell suspension is obtained. In addition to the spleen cells, lymph node cells at various sites of the body can be obtained and used for cell fusion. The spleen cell suspension thus obtained and the myeloma cell lines obtained by the above step 3 are placed in a medium such as minimum essential medium (MEM), DMEM and RPMI-1640 medium, followed by addition of a fusogen, such as polyethylene glycol (PEG). A widely-known fusogen can be used, including inactivated HVJ (Hemagglutinating virus of Japan, "Sendai virus") and the like. Preferably, 0.5 to 2 ml of 30 to 60% PEG can be added. PEG with molecular weights from 1,000 to 8,000 can be employed, more preferably, PEG with molecular weights from 1,000 to 4,000. The preferred concentration of PEG in the fusion medium is from 30 to 60%. As required, a small amount of dimethyl sulfoxide or the like is added to promote fusion. The ratio of spleen cells (lymphocytes) : myeloma cell lines to be used for fusion is preferably 1:1 to 20:1, and preferably falls within 4:1 to 7:1. The fusion reaction is conducted for 1 to 10 min, prior to the addition of a medium such as RPMI-1640 medium. Fusion reaction can be done plural times. After fusion reaction, cells are separated with a centrifuge, etc., then transferred to a selection medium.
5. Selection and cloning of hybridomas (hybrid cells)
   The selection medium includes, for example, FCS-containing MEM, RPMI-1640 medium, etc., supplemented with hypoxanthine, aminopterin, and thymidine; so-called HAT medium. The replacement method for the selection medium is to replenish a volume equivalent to the capacity dispensed into the medium plate on the next day, and thereafter the medium is replaced by half a volume with HAT medium every one to three days. The replacement can be modified depending on situations. Eight to sixteen days after fusion, the medium may be replaced every one to four days with conventionally known "HT medium" wherein aminopterin is excluded. As a feeder cell, for example, mouse thymocyte can be used, which is sometimes effective.
   The supernatant of the culture well with vigorously growing hybridoma is screened, for example, for assaying target antibodies, by using a predetermined peptide fragment as an antigen or by using a labeled anti-mouse antibody with a measuring system such as radioimmunoassay (RIA), enzyme-linked immunosorbent assay (ELISA) and fluoroimmunoassay (FIA), or by a fluorescence activated cell sorter (FACS), etc. The target antibody-producing hybridoma is cloned. Cloning is carried out by picking up colonies in the agar medium or by the limiting dilution. The limiting dilution is preferred. It is desirable that cloning should be performed plural times.
6. Production of monoclonal antibodies
   The obtained hybridoma cells are cultured in a suitable growth medium such as FCS-containing MEM, RPMI-1640 medium or others, and a desired monoclonal antibody can be obtained from the culture supernatant. Mass-procuction of of monoclonal antibodies can be carried out via propagating hybridomas as ascites tumors, etc. In this case, each hybridoma is implanted intraperitoneally and propagated in a histocompatible animal isogenic to an animal from which the myeloma cell is derived. Alternatively, each hybridoma can be inoculated, for example, in nude mice, and propagated to produce the monoclonal antibody in the ascites of the animals. The produced monoclonal antibody can be collected from the ascitic fluid and obtained. Prior to implantation of hybridomas, the animal is pretreated intraperitoneally with mineral oils such as Pristane (2,6,10,14-tetramethyl-pentadecane). After the pretreatment, the hybridoma can be propagated therein and the ascitic fluid can be harvested. The ascitic fluid can be used as a monoclonal antibody without purification or after purification by conventionally known methods, including salting out such as precipitation with ammonium sulfate, gel filtration with Sephadex and the like, ion exchange chromatography, electrophoresis, dialysis, ultrafiltration, affinity chromatography, high-performance liquid chromatography, etc. Preferably, the monoclonal antibody-containing ascitic fluid is fractionated with ammonium sulfate, separated and purified by treatments with anion exchange gel such as DEAE-Sepharose, an affinity column such as protein A column, etc. More preferably, it is treated with affinity chromatography using immobilized antigens or antigen fragments (for example, synthetic peptides, recombinant antigen proteins or peptides, portions which the antibody can specifically recognize, etc.); affinity chromatography with immobilized protein A; hydroxyapatite chromatography; etc.

In addition, it is possible to use transgenic mice and other organisms including other mammals for expressing antibodies such as humanized antibodies against the immunogenic polypeptide products of the present invention. It is also possible to produce antibodies with recombinant DNA techniques wherein the antibody thus obtained in a large amount is sequenced and/or a nucleotide sequence is employed which codes for the antibody obtained from the hybridoma cell line. The nucleic acid encoding the monoclonal antibody can be readily isolated and sequenced using conventional procedures (e.g., by using oligonucleotide probes that are capable of binding specifically to genes encoding the heavy or light chain of murine antibodies), and the like. Once isolated, the DNA may be placed, according to the aforementioned techniques, into expression vectors, which are then transfected into host cells such as CHO cells or COS cells. The DNA may be modified, for example, by substituting the coding sequence for human heavy- and light-chain constant domains for the homologous murine sequences (Morrison et al., Proc. Natl. Acad. Sci. USA, 81: 6581, 1984). Thus, chimeric and hybrid antibodies having a desired binding specificity can be prepared. Further, antibodies can be modified, including preparations of chimeric or hybrid antibodies by adaptations of known methods in synthetic protein chemistry, including those involving coupling agents as listed hereinbelow.

Humanized antibodies are achievable by known techniques in the art (e.g., Joes et al., Nature, 321: pp. 522-525 (1986); Riechmann et al., Nature, 332: pp. 323-327 (1988); Verhoeyen et al., Science, 239: pp. 1534-1536 (1988)). Human monoclonal antibodies can be achieved according to known techniques in the art. For producing human monoclonal antibodies, human myeloma cells and human-mouse hetero-myeloma cells are known in the art (Kozbor, J. Immunol., 133, p. 3001 (1984); Brodeur et al., Monoclonal Antibody Production Techniques and Applications, pp. 51-63, Marcel Dekker, Inc., New York (1987)). Methods for making bispecific antibodies are known in the art (Millstein et al., Nature, 305: pp. 537-539 (1983); WO93/08829; Traunecker et al., EMBO J., 10: pp. 3655-3659 (1991); Suresh et al., "Methods in Enzymology," Vol. 121, pp. 210 (1986)).

These antibodies may be treated with enzymes such as trypsin, papain, pepsin and others, and occasionally be subjected to reduction, to produce antibody fragments including Fab, Fab', and F(ab') ₂. These antibody fragments may be occasionally used.

The antibodies may be employed in any known assay method, such as competitive binding assays, direct and indirect sandwich assays, and immunoprecipitation assays (Zola, Monoclonal Antibodies: A Manual of Techniques, pp. 147-158 (CRC Press, Inc., 1987).

Any method known in the art may be employed for separately conjugating the antibody to a detectable moiety. Such methods include those methods described in David et al., Biochemistry, 13: 1014-1021 (1974), Pain et al., J. Immunol. Meth., 40: pp. 219-231 (1981); and "Methods in Enzymology," Vol. 184, pp. 138-163 (1990). The antibody to be labeled with a marker may include IgG fractions, and specific binding fragments Fab' obtainable by reduction after pepsin digestion. The labels include enzymes (e.g., peroxidase, alkaline phosphatase, beta-D-galactosidase, etc.), chemical substances, fluorescences, radioisotopes, and the like, as disclosed herein below.

In the present invention, detection (including prediction) and measurement (assay) can be carried out by immunostaining including, for example, staining of tissues and cells, immunoassays including, for example, competitive and non-competitive immunoassays, radioimmunoassay, ELISA, etc. The detection and measurement (assay) can also be carried with or without B-F separation. The detection and measurement is carried out preferably by radioimmunoassay and enzyme immunoassay, as well as sandwich assay. In the sandwich-type assay for example, one of the antibodies is set against the protein of the present invention or its related peptide fragment, and the other against the residues on the N-terminal side of the protein wherein one of both the antibodies is detectably labeled. The other antibody capable of recognizing the same antigen is immobilized on a solid phase. In order to allow a test sample to react with the labeled antibody and the immobilized antibody in turn as appropriate, incubation is performed, and the unbound antibody is separated, followed by measurement of the label. The quantity of the measured label is proportional to that of the antigen, that is, the polypeptide fragment antigen. Depending on the order of addition of the insolubilized antibody and the labeled antibody, this assay is called simultaneous sandwich type assay, forward sandwich type assay, or reverse sandwich type assay, and the like. During the process of measurement, for example, washing, stirring, shaking, filtration, or pre-extraction of the antigen is appropriately employed under certain circumstances. Specific reagents, concentration of a buffer and the like, temperature, and other measurement conditions such as incubation time for treatment can be varied depending on factors such as antigen concentration in test samples and properties of test samples. A person skilled in the art can conduct the measurement by selecting appropriately an optimal condition effective for each measurement while using a conventional experimental method.

A number of carriers capable of immobilizing an antigen or an antibody are known, and a carrier appropriately selected from these can be used in the present invention. Various carriers used for antigen-antibody reaction and the like are known, and use in the present invention can be naturally made by selecting from these known carriers. Particularly preferred use includes, for example, an inorganic material such as glass, e.g. activated glass, porous glass, silica gel, silica-alumina, alumina, magnetized iron, and magnetized alloy, an organic polymer such as polyethylene, polypropylene, polyvinyl chloride, polyfluorovinylidene, polyvinyl acetate, polymethacrylate, polystyrene, styrene-butadiene copolymer, polyacrylamide, cross-linked polyacrylamide, styrene-methacrylate copolymer, polyglycidylmethacrylate, acrolein-ethylene glycol dimethacrylate copolymer, cross-linked albumin, collagen, gelatin, dextran, agarose, cross-linked agarose, natural or altered cellulose, e.g. cellulose, microcrystalline cellulose, carboxymethyl cellulose, and cellulose acetate, cross-linked dextran, polyamide e.g. nylon, polyurethane, polyepoxy resin, and further a material obtained by their emulsification polymerization, cell, and red blood cell. Materials introduced with a functional group into these materials using a silane coupling agent or the like as needed are included.

Furthermore, the surface of a solid material (material body), for example, filter paper, bead, inner wall of test vessel such as test tube, titer plate, titer well, glass cell, or cell made of synthetic material, e.g. synthetic resin cell, glass rod, rod made of synthetic material, rod with thickened or narrowed end, rod attached with round or flat protrusion at its end, or rod in a thin plate form is included.

An antibody can be bound to these carriers, and preferably a monoclonal antibody that reacts specifically with the antigen obtained in the present invention can be bound. The binding of a substance involved in antigen-antibody reaction to the carrier can be carried out by a physical method such as adsorption, a chemical method with use of a coupling agent or an activated material, or further a method utilizing a mutual chemical reaction to form a bond between them.

The label includes an enzyme, enzyme substrate, enzyme inhibitor, prosthetic group, coenzyme, enzyme precursor, apoenzyme, fluorescent substance, dye, chemiluminescent compound, luminescent substance, chromogenic substance, magnetic substance, metal particle, eg. gold colloid, radioactive substance, and the like. The enzyme includes an oxidation-reduction enzyme such as dehydrogenase, reductase, or oxidase, a transferase that catalyzes the transfer of, for example, amino group, carboxyl group, methyl group, acyl group, or phosphate group, a hydrolase that hydrolyzes, for example, ester bond, glycoside bond, ether bond, and peptide bond, a lyase, an isomerase, a ligase, and the like. A combination of a plurality of enzymes may also be used for detection. For example, enzymic cycling can also be used.

Examples of representative radioactive isotopes for labeling are [³²P], [¹²⁵I], [¹³¹I], [³H], [¹⁴C], and [³⁵S] .

Representative enzyme labels include peroxidase such as horseradish peroxidase, galactosidase such as β-D-galactosidase from E. coli, maleate dehydrogenase, glucose-6-phosphate dehydrogenase, glucose oxidase, glucoamylase, acetylcholine esterase, catalase, alkaline phosphatase such as alkaline phosphatase from bovine small intestine and alkaline phosphatase from E. coli, and the like.

When an alkaline phosphatase is used, measurement can be performed by generated fluorescence, luminescence, and the like with use of a substrate such as umbelliferone derivative, e.g. 4-methylumbelliferyl phosphate, phosphorylated phenol derivative, e.g. nitrophenyl phosphate, enzymic cycling system using NADP, luciferin derivative, or dioxetane derivative. Luciferin and luciferase system can also be utilized. When a catalase is used, its reaction on hydrogen peroxide produces oxygen, and the oxygen can be detected with an electrode and the like. The electrode can be a glass electrode, ion electrode that employs insoluble salt membrane, liquid membrane electrode, polymer membrane electrode, and the like.

The enzyme label may also be replaced with a biotin label and enzyme labeled avidin (streptavidin). For the label, a plurality of different kinds of labels may also be used. In these cases, it is also made possible that a plurality of measurements are carried out continuously, discontinuously, simultaneously or independently.

In the present invention, a combination of 4-hydroxyphenylacetic acid, 1,2-phenylenediamine, tetramethylbenzidine, or the like with horseradish peroxidase, umbelliferylgalactoside, nitrophenylgalactoside, or the like with β-D-galactosidase, or enzyme reagents for glucose-6-phosphate dehydrogenase and the like can be utilized for formation of signal, and a system making it possible to form quinol compounds from hydroquinone, hydroxybenzoquinone, and hydroxyanthraquinone, thiol compounds from lipoic acid and glutathione, phenol derivatives, ferrocene derivatives, and the like via action of enzymes and the like can be used.

The fluorescent substances and chemiluminescent compounds may include fluorescein isothiocyanate; Rhodamine derivatives such as Rhodamine B isothiocyanate and tetramethyl Rhodamine isothiocyanate, dancyl chloride (5-(dimethylamino)-1-naphtalenesulfonyl chloride), dancyl fluoride, fluorescamine (4-phenylspiro[furan-2(3H),1'-(3'H)-isobenzofuran]-3,3'-dione), phycobiliprotein, acridinium salts; luminol compounds such as lumiferin, luciferase and aequorin; imidazoles, oxalic acid esters, rare earth chelate compounds, cumarin derivatives, etc.

Labeling can be performed using the reaction of a thiol group with a maleimide group, the reaction of a pyridyl disulfide group with thiol group, the reaction of an amino group with an aldehyde group, and the like, and its applicable method is appropriately selected from known methods, methods readily performed by a person skilled in the art, and further their modified methods. In addition, a coupling agent that can be used for preparing the above immunogenic complex, a coupling agent that can be used for binding to the carrier, and the like can be used.

The coupling agent includes, for example, formaldehyde, glutaraldehyde, hexamethylene diisocyanate, hexamethylene diisothiocyanate, N,N'-polymethylene-bis(iodoacetamide), N,N'-ethylene-bis(maleimide), ethylene glycol bis(succinimidylsuccinate), bisdiazobenzidine, 1-ethyl-3-(3-dimethylaminopropyl)carbodiimide, succinimidyl 3-(2-pyridyldithio)propionate (SPDP), N-succinimidyl 4-(N-maleimidomethyl)cyclohexane-1-carboxylate (SMCC), N-sulfosuccinimidyl 4-(N-maleimidomethyl)cyclohexane-1-carboxylate, N-succinimidyl(4-iodoacetyl)aminobenzoate, N-succinimidyl 4-(1-maleimidophenyl)butyrate, N-(ε-maleimidocaproyloxy)succinimide (EMCS), iminothiolane, S-acetylmercaptosuccinic anhydride, methyl-3-(4'-dithiopyridyl)propionimidate, methyl-4-mercaptobutyrylimidate, methyl 3-mercaptopropionimidate, N-succinimidyl-S-acetylmercaptoacetate, and the like.

According to the assay method of the present invention, a substance to be measured is allowed to react with a labeled antibody reagent such as a monoclonal antibody labeled with an enzyme and the like, and an antibody bound to a carrier in succession or simultaneously as well. The order of addition of these reagents varies depending on a type of selected carrier system. When sensitized beads such as plastic are used, a labeled antibody reagent such as a monoclonal antibody labeled with an enzyme and the like and a test sample containing a substance to be measured are first put together in an appropriate test tube, and then sensitized beads such as plastic are added, thereby conducting the measurement.

In the assay method of the present invention, an immunoassay is used. For a solid carrier in this case, an arbitrary selection of a material and a form can be made from among various materials and forms such as ball, microplate, stick, microparticle and test tube made of polystyrene, polycarbonate, polypropylene, and polyvinyl that well adsorb a protein such as antibody.

The measurement can be performed in an appropriate buffer so as to maintain an optimal pH, for example, ca. pH 4 to ca. 9. Particularly suitable buffers include, for example, acetate buffer, citrate buffer, phosphate buffer, Tris buffer, triethanolamine buffer, borate buffer, glycine buffer, carbonate buffer, and Tris-HCl buffer. These buffers can be mutually mixed in an arbitrary proportion for use. It is desirable to carry out the antigen-antibody reaction at ca. 0 to ca. 60°C.

The treatment of incubation of an antibody reagent such as a monoclonal antibody labeled with an enzyme, an antibody reagent bound to a carrier, and further a substance to be measured can be performed until an equilibrium is reached, while the reaction can be stopped after a limited incubation treatment by separating the solid phase and liquid phase at a much earlier time point before an equilibrium of the antigen-antibody reaction is attained, and the degree of the presence of a label on an enzyme or the like in either the liquid phase or the solid phase can be measured. The procedure for the measurement is possible with use of an automated measurement apparatus; a display signal generated from conversion of a substrate by the action of an enzyme can also be detected and measured with a luminescence detector, a photodetector, and the like.

In the antigen-antibody reaction, suitable means such as a stabilizing each reagent, a substance to be measured, and a label, e.g. enzyme or stabilizing the antigen-antibody reaction itself can be taken. Further, it is possible to add a protein, stabilizing agent, surface active agent, chelating agent, and the like to the incubation solution in order to eliminate a non-specific reaction, reduce an effect of inhibitory action, or activate the reaction for measurement. A preferred chelating agent is ethylenediaminetetraacetic acid (EDTA).

A blocking treatment for preventing a non-specific binding reaction that is generally used in the art or known to a person skilled in the art may be applied; for example, treatment with normal serum protein, albumin, skim milk, fermented milk products, collagen, gelatin, and the like from a mammal or the like. As long as the objective is to prevent a non-specific binding reaction, these methods can be used without any particular limitation.

A sample in any form of solution or colloid, non-liquid sample, and the like can be used as the sample to be measured by the assay method of the present invention. Preferably included are samples originating from living organisms, for example, thymus, testis, intestine, kidney, brain, breast cancer, ovarian cancer, cancer of colon and rectum, blood, serum, plasma, synovial fluid, cerebrospinal fluid, pancreatic fluid, bile, saliva, amniotic fluid, urine, other body fluids, cell-cultured medium, tissue-cultured medium, tissue homogenate, biopsy sample, tissue, and cell.

When various analysis and assay methods including these individual immunological assays are applied to the measurement methods of the present invention, setting of any special condition, operation, and the like is unnecessary. A measurement system related to the target substance of the present invention or the substance having an activity essentially equivalent to the target substance may be constructed by a person skilled in the art by adding common technical considerations to a common condition and operational method in each individual method.

The details of these general technical means can be referred to in a review, a book, and the like [for example, H. Irie (ed.), "Radioimmunoassay," Kodansha, Tokyo (1974) (in Japanese); H. Irie (ed.), "Zoku Radioimmunoassay," Kodansha, Tokyo (1979) (in Japanese); E. Ishikawa, et. al. (eds.) "Kouso Meneki Sokuteihou," Igakushoin, Tokyo (1978) (in Japanese); E. Ishikawa, et. al. (eds.), "Kouso Meneki Sokuteihou" (2nd Ed.), Igakushoin, Tokyo (1982) (in Japanese); E. Ishikawa, et. al. (eds.) "Kouso Meneki Sokuteihou" (3rd Ed.), Igakushoin, Tokyo (1987) (in Japanese); H. V. Vunakis et al. (ed.), "Methods in Enzymology", Vol. 70 (Immunochemical Techniques, Part A), Academic Press, New York (1980); J. J. Langone et al. (ed.), "Methods in Enzymology", Vol. 73 (Immunochemical Techniques, Part B), Academic Press, New York (1981); J. J. Langone et al. (ed.), "Methods in Enzymology", Vol. 74 (Immunochemical Techniques, Part C), Academic Press, New York (1981); J. J. Langone et al. (ed.), "Methods in Enzymology", Vol. 84 (Immunochemical Techniques, Part D: Selected Immunoassays), Academic Press, New York (1982); J. J. Langone et al. (ed.), "Methods in Enzymology", Vol. 92 (Immunochemical Techniques, Part E: Monoclonal Antibodies and General Immunoassay Methods), Academic Press, New York (1983); J. J. Langone et al. (ed.), "Methods in Enzymology", Vol. 121 (Immunochemical Techniques, Part I: Hybridoma Technology and Monoclonal Antibodies), Academic Press, New York (1986); J. J. Langone et al. (ed.), "Methods in Enzymology", Vol. 178 (Antibodies, Antigens, and Molecular Mimicry), Academic Press, New York (1989); M. Wilchek et al. (ed.), "Methods in Enzymology", Vol. 184 (Avidin-Biotin Technology), Academic Press, New York (1990); J. J. Langone et al. (ed.), "Methods in Enzymology", Vol. 203 (Molecular Design and Modeling: Concepts and Applications, Part B: Anibodies and Antigens, Nucleic Acids, Polysaccharides, and Drugs), Academic Press, New York (1991), and the like, or the references cited therein (the descriptions therein are hereby incorporated into the present specification by reference)].

It is also possible to perform epitope mapping by using the anti-p30 antibody of the present invention (antihuman p30 antibody, anti-mouse p30 antibody, etc.), in particular, a monoclonal antibody, and the use of an antibody that recognizes each epitope allows detection and measurement of the protein, its related peptide fragment, and the like to be performed.

The antibodies against the protein and its related peptide fragments are useful in detection and/or measurement of cell adhesion by the protein, its binding to the activated form of Rap1 and their resultant various physiological phenomena and phenomena such as regulation, stimulation, and suppression of bioactivities, detection and/or measurement of various physiologically active substances, physiological phenomena, or biophenomena arising from an excess or a decrease of the p30 protein and the like, research and development of factors and mechanisms that regulate the interaction of p30 and Rap1, and the like. The antibody, particularly monoclonal antibody, is useful in (i) detection of disorder, abnormality, and/or disease in tissues and cells caused by the interaction of p30 and Rap1 (ii) detection of cell tumorigenesis, locomotion, invasion, migration and/or metastasis, and possibility thereof that are caused by the interaction of p30 and Rap1 (iii) detection of disorder, abnormality and/or disease, and possibility thereof that are caused in relation to cell adhesion or cell migration involving the interaction of p30 and Rap1 (iv) measurement of expression amount of the p30 protein, (v) detection and/or measurement of a change in the binding of the activated Rap1 and p30, (vi) Search for a compound or the like that regulates the binding of p30 and Rap1 and/or (vii) detection and/or measurement of an activity of a compound that regulates the production of p30 protein, and so forth. It is expected that these can be used to know and understand immune response, inflammation, angiogenesis, blood coagulation, wound healing, regenerative process, malignant transformation, invasion of cancer cell, metastasis, autoimmune process, hematological process, cell abnormality, tissue abnormality and the degree of cancer mobility, invasiveness, chemotaxis and/or metastasis.

According to the present invention, detection and/or measurement of promotion activities or suppression and inhibition activities for phenomena and actions due to various physiological activities or bioactivities resulting from the p30-Rap1 binding makes it possible to use them in judging and monitoring of efficacy of a drug for prevention and treatment of tissue diseases, antiinflammatory drug, anticancer drug, cancer metastasis inhibitor, antiatherosclerotic drug, drug for treatment of joint destruction, antiallergic drug and/or immunosuppressive drug.

Further, the present invention makes it possible to provide a method for detection and/or measurement of disordered phenomena in tissues, cells, or proteins caused by the protein, and a reagent for that.

The compounds discovered by the screening method of the present invention possess effects such as suppression of cell adhesion, suppression of cell migration, immunosuppression, suppression of cytokine production, suppression of apoptosis, and suppression of cell growth. Accordingly, these compounds can be used for an antiinflammatory drug, drug for treatment of immune diseases, and cancer metastasis inhibitor. The efficacy of these compounds can be confirmed by the following methods. These are not to be read as limiting or restricting the scope of the invention disclosed in the present application.
[1] Acute and chronic gastrointestinal ulcer
   Effects on Shy ulcer (pylorus ligation ulcer), water immersion restraint ulcer, indomethacin ulcer, cysteamine duodenal ulcer, and an ulcer caused by a necrotizing substance are studied as acute models. Effects on acetic acid ulcer is studied as chronic model.
   (1) Pylorus ligation ulcer
      A test for a pylorus ligation ulcer can be performed according to the description on P.249 of the reference 3.
      Preparation of model: After a rat (140 to 200g) is fasted for 48 hours or 72 hours (however, on ad libitum water intake), the animal is subjected to an abdominal section under anesthesia with ether, followed by ligation of the pylorus. After its abdomen is closed, the rat is fasted and given no water. Administration of test substance: A test substance is administered into the peritoneal cavity or the duodenum immediately after the pylorus ligation.
      Evaluation method: After 13 to 17 hours, the rat is sacrificed under anesthesia, and the excised stomach is fixed in an aqueous solution of 10% neutrally buffered formalin. The mucous surface of the fixed specimen is washed with water, immersed in an aqueous solution of 3% acetic acid for 5 min and a solution of 1% Alcian blue (dissolved in an aqueous solution of 3% acetic acid) for 20 min for staining, and then washed with an aqueous solution of 3% acetic acid. An area of the dark blue site stained by Alcian blue is measured using an image analyzer (general-purpose image processing software "Win ROOF") to determine the ulcer area. Ulcer areas are compared between the group in which the test substance is administered and the group in which no test substance is administered.
   (2) Water immersion restraint ulcer
      A test for a water immersion restraint ulcer is carried out according to the description on P.249 of the reference 3.
      Preparation of model: Four legs of a rat are tied to a wire net, and the animal is immersed up to the lower edge of the breast bone in a standing position in cold water (23±0.2°C) for 10 to 12 hours.
      Administration of test substance: Ten minutes before the water immersion restraint, a test substance is administered intraperitoneally or orally.
      Evaluation method: After completing the water immersion restraint, the rat is sacrificed under anesthesia, and the same evaluation method as in the above [1] (1) is carried out.
   (3) Indomethacin ulcer
      A test for an indomethacin ulcer can be carried out according to the description on P.250 of the reference 3.
      Preparation of model: A rat (22 to 230 g) is fasted for 24 hours, and then 30 mg/kg of indomethacin (Sigma) is subcutaneously injected.
      Administration of test substance: Ten minutes before administration of indomethacin, a test substance is administered intraperitoneally or orally.
      Evaluation method: Eight hours after the administration of indomethacin, the rat is sacrificed under anesthesia, and the same evaluation method as in the above [1] (1) is carried out.
   (4) Cysteamine duodenal ulcer
      A test for a cysteamine duodenal ulcer can be carried out according to the description on P.250 of the reference 3.
      Preparation of model: A rat (male Wistar) is fasted for 24 hours, and then 300 to 400 mg/kg of cysteamine is subcutaneously injected.
      Administration of test substance: Ten minutes before the administration of indomethacin, a test substance is administered intraperitoneally or orally.
      Evaluation method: 18 hours after the administration of cysteamine, the rat is sacrificed under anesthesia, and the same evaluation method as in the above [1] (1) is carried out.
   (5) Ulcer caused by a necrotizing substance
      A test for an ulcer caused by a necrotizing substance can be carried out according to the method described on P.251 of the reference 3.
      Preparation of model: After a rat is fasted for 24 hours and given no water, pure ethanol, 0.6 N HCl, 25% NaCl, 80 mM acidic taurocholic acid, or hot water is orally administered.
      Administration of test substance: Thirty minutes before the administration of a necrotizing substance, a test substance is administered intraperitoneally or orally.
      Evaluation method: Immediately after the necrotizing substance is administered, the rat is sacrificed under anesthesia, and the same evaluation method as in the above [1] (1) is carried out.
   (6) Acetic acid ulcer
      A test for an acetic acid ulcer can be carried out according to the method of preparation of a model described on P.251 of the reference 3.
      Animal model: A rat is subjected to an abdominal section under anesthesia with ether, and 0.04 to 0.06 ml of 20% acetic acid solution is injected under the mucous membrane of the border of the forestomach and gland stomach.
      Administration of test substance: A test substance is orally administered repeatedly for 14 days from the next day of the acetic acid injection.
      Evaluation method: The rat is sacrificed under anesthesia on the next day of the final administration of the test substance, and the same evaluation method as in the above [1] (1) is carried out.
[2] Ulcerative colitis
   A test for an ulcerative colitis can be performed according to the method described in the reference 5.
   Preparation of model: After a rat is allowed to drink freely an aqueous solution of 3% DSS (dextran sulfate sodium) for 11 days, it is subsequently allowed to drink freely an aqueous solution of 1% DSS for 14 days.
   Administration of test substance: During the period of the administration of the aqueous solution of 1% DSS, a test substance is repeatedly administered orally once a day for 14 days.
   Evaluation method: 13 to 17 hours after the final administration, the rat is sacrificed under anesthesia, and the excised colon is fixed in an aqueous solution of 10% neutrally buffered formalin. The mucous surface of the fixed specimen is washed with water, immersed in an aqueous solution of 3% acetic acid for 5 min and in a solution of 1% Alcian blue (dissolved in an aqueous solution of 3% acetic acid) for 20 min for staining, and then washed with an aqueous solution of 3% acetic acid. An area of the dark blue site stained by Alcian blue is measured using an image analyzer (image processing software "Win ROOF") to determine an erosion area. Erosion areas are compared between the group in which the test substance is administered and the group in which no test substance is administered.
[3] Crohn disease
   A test for Crohn disease can be performed according to the method described in the reference 6.
   Preparation of model: TNBS (160 mg/kg in 50% ethanol) is injected into a rat from the end of the ileum to prepare small intestinal inflammation.
   Administration of test substance: After the preparation of small intestinal inflammation, a test substance is orally administered repeatedly once a day for 7 days.
   Evaluation method: Seven days after the preparation of small intestinal inflammation, the rat is sacrificed under anesthesia, and a pathological anatomical examination (macroscopic observation) is carried out. Further, after fixing in formalin, a tissue specimen stained by HE is prepared (reference 44, P.10), and histopathological examination (microscopic observation) is performed. Degrees of each lesion observed in both examinations are classified into 5 stages: no significant lesion; 0, minimal; 0.5, slight; 1.0, moderate; 2.0, and marked; 3.0. After converting into numeric values, these are summed up for each group, followed by comparing the values between the group in which the test substance is administered and the group in which no test substance is administered.
[4] Chronic obstructive pulmonary disease
   A test for chronic obstructive pulmonary disease (COPD) can be carried out according to the method described in the reference 7.
   Preparation of model: A guinea pig is exposed to mainstream smoke of commercially available cigarettes 30 min a day and 5 days a week for 4 weeks using a cigarette mainstream smoke generator (INH06-CIGR01, product of M.I.P.S. Inc.)
   Administration of test substance: During the 4-week exposure to cigarette mainstream smoke, a test substance is orally administered repeatedly once a day.
   Evaluation method: After completion of the administration period, the guinea pig is sacrificed under anesthesia, and its lung is subjected to a pathological anatomical examination (macroscopic examination). Further, the lung is fixed in formalin, and a tissue specimen stained with HE (reference 19, P.10) is prepared, followed by a histopathological examination (microscopic examination). Degrees of each lesion observed in both examinations are classified into 5 stages: no significant lesion; 0, minimal; 0.5, slight; 1.0, moderate; 2.0, and marked; 3.0. After converting into numeric values, these are summed up for each group, followed by comparing the values between the group in which the test substance is administered and the group in which no test substance is administered.
[5] Cancer metastasis (reference 13)
   A test for cancer metastasis can be carried out according to the description in the reference 13.
   Preparation of model: To the tail vein of a mouse (C57BL/6N, female) is administered a suspension of cultured B16 mouse melanoma cells in 5x10⁴ cells/0.2 ml.
   Administration of test substance: A test substance is repeatedly administered orally or intraperitoneally from the day of the administration of the B16 cell until the 21st day.
   Evaluation method: The mouse is sacrificed under anesthesia 21 days after the administration of the B16 cells, and its excised lung is fixed in an aqueous solution of 10% neutrally buffered formalin. The fixed specimen is sliced and the number of lesions metastasized with the B16 cells is counted by naked eye. The numbers of the metastatic lesions are compared between the group in which the test substance is administered and the group in which no test substance is administered.
[6] Atopic dermatitis
   A test for atopic dermatitis can be carried out according to the description in the reference 14.
   Preparation of model: A mouse (BALB/C) is intraperitoneally administered with DNP-OVA + Alum (dinitrophenol-ovalbumin + aluminium hydroxide gel) for active sensitization. Fourteen days after the sensitization, DNFB (dinitrofluorobenzene) is applied to the auricula to elicit atopic dermatitis.
   Administration of test substance: A test substance is repeatedly administered orally or intraperitoneally during three different periods that are from the sensitization to the elicitation, from the elicitation to an examination, and from the sensitization to the examination.
   Evaluation method: One hour, 24 hours, and 8 days after the elicitation, the thickness of the auricula is measured. The thicknesses of the auricula are compared between the group in which the test substance is administered and the group in which no test substance is administered.
[7] Allergic rhinitis
   A test for allergic rhinitis can be carried out according to the description on P.225 of the reference 2.
   To a guinea pig is injected intramuscularly 0.7 ml of 5% ovalbumin for sensitization. Fourteen days after the sensitization, the trachea is incised under anesthesia, air heated to from 37 to 40°C is allowed to flow from the trachea on the nasal cavity side to the nasal cavity at a flow rate of 500 ml/min using a compressed-air cylinder, and an internal pressure in the nasal cavity is measured. After 0.3 ml of a test substance is flowed in the nasal cavity, 0.3 ml of 1% ovalbumin is flowed inside the nasal cavity from the trachea to elicit allergic rhinitis. The changing internal pressures in the nasal cavity are measured by a pressure transducer, and these internal pressures are compared between the group in which the test substance is administered and the group in which no test substance is administered.
[8] Asthma
   A test for asthma can be carried out according to the description in the reference 16.
   Preparation of model: To the peritoneal cavity of a guinea pig (Hartley strain) is administered 0.5 ml of OVA +Alum (10 µg OVA +10 mg Alum) on day 1 and day 14 for sensitization.
   Administration of test substance: Fourteen days after the final sensitization, a test substance is orally administered.
   Evaluation method: Thirty minutes after the administration of the test substance, 10 mg/kg of pyrilamine (histamine H₁ antagonist) is administered into the peritoneal cavity. Thirty minutes after the administration, 10 mg/ml solution of OVA PBS (-) is allowed to be aspirated for 10 min for elicitation. Four hours after the OVA elicitation, 400 µg/ml of methacholine is allowed to be inhaled for 1 min. After the inhalation, Penh is measured as an airway resistance parameter with use of a no restriction respiratory function analysis system, and the Penh is compared between the group in which the test substance is administered and the group in which no test substance is administered.
[9] Immunological rejection after transplant
   (1) Ectopic heart transplant
      A test for ectopic heart transplant can be carried out according to the method described on P.44 of the reference 17.
      Preparation of model; A donor rat is subjected to thoracotomy under anesthesia, the inferior vena cava is ligated, and the aorta and the pulmonary artery are separated. A lactate Ringer solution (heparinized, 4°C, 5 ml) is injected from the aorta and discharged from the pulmonary artery for perfusion. The superior vena cava and the pulmonary vein are ligated together, and the heart is excised, followed by keeping it in the lactate Ringer solution. The right cervix skin of a recipient rat that serves for a transplant site is incised under anesthesia with ether, the right external vein is separated, a cuff (a vascular anastomic tube, 1.8 mm of body portion, 1.2 mm of support portion, 1.34 mm of O/D) is attached to its cut edge, the right common artery is separated, and a cuff (1.8 mm of body portion, 1.2 mm of support portion, 0.99 mm of O/D) is attached to its cut edge. The cuff attached to the common artery of the recipient is inserted and ligated to the aorta of the donor heart, and the external vein of the recipient and the pulmonary artery of the donor are similarly anastomosed. After the beats become stable, the skin is sutured so as to wrap the transplanted heart, followed by awakening the rat.
      Administration of test substance: A test substance is administered orally, intraperitoneally, or intravenously once a day from after the model preparation to the end of the test.
      Evaluation method: Palpation of the hypodermic transplanted heart and survival rates are compared between the group in which the test substance is administered and the group in which no test substance is administered.
[10] Rheumatoid arthritis
   A test for rheumatoid arthritis can be carried out according to the method described in the reference 18.
   Preparation of model: To the tail head of a mouse is administered intracutaneously 0.1 ml of a homogeneous emulsion of collagen (containing 3 mg/ml of Type II collagen K-41 derived from bovine joints) and an equal volume of FCA (Freund Complete Adjuvant) (final collagen; 1.5 mg/ml).
   Administration of test substance: A test substance is administered once a day from 1 week to 9 weeks after the model preparation.
   Evaluation method: The articulations of all fingers of four legs are scored once a week according to the arthritis score (no change; 13, slight swelling of one finger or multiple fingers; 14, moderate swelling of one finger or multiple fingers; 15, marked swelling of the entire leg or deformation of the articulations (including ankylosis); 16). The scores are summed up to determine an arthritis score for each animal. The onset day is defined as the day when the arthritis score becomes more than one on an observation day. Arthritis scores and days prior to the onset are compared between the group in which the test substance is administered and the group in which no test substance is administered.
[11] Other target diseases and methods for efficacy tests
   Efficacy tests for other target diseases other than the above diseases are shown in Table 1.

**Table 1**

| Classification of diseases, disease names, and their test methods | | |
|---|---|---|
| Classification | Target disease names | Reference |
| Anti-inflammatory drug and cancer metastasis inhibitor (diseases for which efficacy can be expected via suppression of cell adhesion, suppression of cell migration, suppression of cytokine production, and suppression of apoptosis induction) | Subcutaneous edema | 1 |
| | Behcet's disease | 1 |
| | pleuritis | 1 |
| | peritonitis | 1 |
| | emphysema | 3 |
| | pulmonary edema | 3 |
| | cerebral infraction | 8 |
| | polyarteritis nodosa | 4 |
| | septic shock | 9 |
| | acute respiratory distress syndrome | 10 |
| | multiple organ failure (MOF) | 11 |
| | systemic inflammatory reaction syndrome (SIRS) | 12 |
| Drug for treatment of immune diseases (diseases for which efficacy can be expected via immune suppression) | spontaneously systemic progressive skin sclerosis | 2 |
| | allergic conjunctivitis | 15 |
| | autoimmune uveitis | 2 |
| | glomerulonephriits | 3 |
| | stromal nephritis | 3 |
| | acute renal failure | 3 |
| | chronic renal failure | 3 |
| | systemic lupus erythemato'sus | 2 |
| | allergic encephalomyelitis | 2 |
| | multiple sclerosis | 2 |
| | myasthenia gravis | 2 |
| | Sjoegren syndrome | 2 |
| | systemic autoimmune disease | 2 |

### [Reference]

1: "Seibutsuyakurikagaku Jikkenkouza" (Inflammation and Allergy. vol. I to vol. III), Hirokawa Shoten, Tokyo (in Japanese)
2: "(Shikkanbetsu) Moderudoubutsu no Sakusei to Shinyakukaihatsu notameno Shiken-Jikkenhou" Gijutsu Joho Kyokai (Technical Information Institute), Tokyo (in Japanese)
3: "Shinyakukaihatsu notameno Doubutsumoderu Riyoushusei " R & D Planning, Tokyo (in Japanese)
4: "Nanchishikkan no Moderu to Doubutsujikken" (for common understanding of human diseases) Kyogoku Masahisa, ed., Soft Science, Tokyo (in Japanese)
5: Kimura I, Kawasaki M, Nagahama S, Matsuda A, Kataoka M and Kokuba Y., "Determination of the acute moiety of BX661A, a new therapeutic agent for ulcerative colitis, by studying its therapeutic effect on ulcerative colitis induced by dextran sulfate sodium in rats". Arzneim.-Forsch./Drug Res 1998 48 (11) 11, 1091-1096.
6: Tsujikawa T, Ohta N, Nakamura T, Satoh J, Uda K, Ihara T, Okamoto T, Araki Y, Andoh A, Sasaki M, Fujiyama Y, Bamba T., "Medium-chain triglycerides modulate ileitis induced by trinitrobenzene sulfonic acid". J Gastroenterol Hepatol. 1999 Dec; 14(12):1166-72.
7: Wright J L and Chung A., "A model of tobacco smoke-induced airway obstruction in the guinea pig". Chest 2002 121 5 188s-191s.
8: Tamura A, Graham DI, McCulloch J, Teasdale GM., "Focal cerebral ischaemia in the rat: 1. Description of technique and early neuropathological consequences following middle cerebral artery occlusion". J Cereb Blood Flow Metab. 1981;1(1):53-60.
9: Okamoto I, Abe M, Shibata K, Shimizu N, Sakata N, Katsuragi T, Tanaka K., "Evaluating the role of inducible nitric oxide synthase using a novel and selective inducible nitric oxide synthase inhibitor in septic lung injury produced by cecal ligation and puncture". Am J Respir Crit Care Med. 2000 Aug;162(2 Pt 1):716-22.
10: van Helden HP, Kuijpers WC, Langerwerf PE, Langen RC, Haagsman HP, Bruijnzeel PL., "Efficacy of Curosurf in a rat model of acute respiratory distress syndrome". Eur Respir J. 1998 Sep; 12(3):533-9.
11: Gardinali M, Borrelli E, Chiara O, Lundberg C, Padalino P, Conciato L, Cafaro C, Lazzi S, Luzi P, Giomarelli PP, Agostoni A., "Inhibition of CD11- CD18 complex prevents acute lung injury and reduces mortality after peritonitis in rabbits". Am J Respir Crit Care Med. 2000 Mar; 161(3 Pt 1):1022-9.
12: Gloor B, Uhl W, Tcholakov O, Roggo A, Muller CA, Worni M, Buchler MW., "Hydrocortisone treatment of early SIRS in acute experimental pancreatitis". Dig Dis Sci. 2001 Oct;46(10):2154-61.
13: Chirivi RG, Garofalo A, Crimmin MJ, Bawden LJ, Stoppacciaro A, Brown PD, Giavazzi R., "Inhibition of the metastatic spread and growth of B16-BL6 murine melanoma by a synthetic matrix metalloproteinase inhibitor". Int J Cancer. 1994 Aug 1; 58(3):460-4.
14: Satoh T, Tahara E, Yamada T, Watanabe C, Itoh T, Terasawa K, Nagai H, Saiki I., "Differential effect of antiallergic drugs on IgE-mediated cutaneous reaction in passively sensitized mice". Pharmacology. 2000 Feb;60(2):97-104.
15: Magone MT, Chan CC, Rizzo LV, Kozhich AT, Whitcup SM., "A novel murine model of allergic conjunctivitis". Clin Immunol Immunopathol. 1998 Apr;87(1):75-84.
16: Andersson P, Bergstrand H., "Antigen-induced bronchial anaphylaxis in actively sensitized guinea-pigs: effect of long-term treatment with sodium cromoglycate and aminophylline". Br J Pharmacol. 1981 Nov;74(3):601-9.
17: "Zukai Jikkendoubutsu Gijutsushu II" Japanese Association for Experimental Animal Technologist, ed., Adthree, Tokyo (1st issue published June 10, 1998) (in Japanese)
18: Kato F, Nomura M, Nakamura K., "Arthritis in mice induced by a single immunisation with collagen". Ann Rheum Dis. 1996 Aug; 55(8):535-9.
19: "Senshokuhou no Subete," Medical Technology (published monthly), ed., Ishiyaku, Tokyo (1st version of 1st issue published March 15, 1980) (in Japanese)

When the active component of the present invention [for example, (a) a polypeptide related to the p30, a partial peptide thereof, or a salt thereof, a mutant p30 peptide and its related peptide, (b) a nucleic acid such as DNA encoding the p30, Rap1 protein, or the above polypeptide, (c) the antibody of the present invention, its partial fragment (including monoclonal antibody) or its derivative, (d) a compound to regulate the interaction between p30 and Rap1, e.g. the binding (compound that promotes or suppresses and/or inhibits a phenomenon that promotes or suppresses and inhibits the binding, or a biological activity such as a deterioration and excessive production or decomposition phenomenon of tissue or protein) or a salt thereof, or a compound or a salt thereof that regulates the p30 protein production, (e) an antisense oligonucleotide against a nucleic acid such as DNA that is the object of the present invention, and the like, and (f) an active substance found by the use of the present invention] is used for medicine, for example, a binding inhibitor between p30 and Rap1 or a salt thereof is usually administered alone or in combination with various pharmacologically acceptable formulation supplements and can be administered as a medicine component or a pharmaceutical preparation. Preferably, it is administered in the form of a pharmaceutical preparation suitable for use in oral administration, local administration, parenteral administration, or the like, and any mode of administration (including inhalation or rectal administration) may be used according to each objective.

Further, the active component of the present invention can be used by combining with various medicines, for example, antitumor drug (anticancer drug), tumor metastasis inhibitor, thrombus formation inhibitor, agent for treatment of joint destruction, analgesic, antiphlogistic and/or immunosuppressive agent. These can be used without limitation as long as they have an advantageous effect, and the active component can be selected from those known in the field.

The mode of parenteral administration can include local, percutaneous, intravenous, intramuscular, subcutaneous, intracutaneous, and intraperitoneal administrations; however, direct administration to an affected area is also possible, or in some cases, is desirable. Preferably, for mammals including humans, oral administration or parenteral administration (for example, intracellular, intratissular, intravenous, intramuscular, subcutaneous, intracutaneous, intraperitoneal, intrathoracic and intraspinal administrations, drip infusion, intestinal infusion, transrectal infusion, ear dropping, eye dropping, and nasal dropping, and applying to tooth, skin and mucus) is possible. Specific forms of pharmacological preparations include liquid formulation, dispersed formulation, semisolid formulation, powder formulation, molded formulation, slow release formulation, and the like, and for example, tablet, coated tablet, sugar coated agent, pill, troche, hard capsule, soft capsule, microcapsule, implantation tablet, powder, epipastic, granule, microsphere, injection, liquid, elixir, emulsion, irrigations, syrup, mixtura, emulsifiable concentrate, suspension, liniment, lotion, aerosol, spray, inhalant, nebula, ointment, plaster, patch, dermatologic paste, cataplasm, cream, oil solution, suppository (e.g. rectum suppository), tincture, mixtura for skin, eye drops, nasal drops, ear drops, liniment, transfusion, powder for injection solution and the like, lyophilized preparation, and gel preparation.

Compositions for medicine can be formulated according to a general method. For example, if appropriate, a physiologically acceptable carrier, pharmaceutically acceptable carrier, adjuvant, constituents, excipient, diluent, flavoring agent, aromatic, sweetener, vehicle, antiseptic, stabilizer, binder, pH controlling agent, buffer, surface active agent, base, solvent, filler, extender, solubilizing agent, solubilizer, isotonizing agent, emulsifier, suspending agent, dispersant, thickener, gelatinizer, hardening agent, absorbent, adhesive, elastic agent, plasticizer, disintegrating agent, propellant, preservative, antioxidant, shading agent, humectant, demulcent, antistatic agent, soothing agent, or the like is used alone or in combination thereof, and the protein or the like of the present invention is mixed with them, thereby making it possible to produce a medicine in a unit dosage form required for formulation accepted in general.

Formulation suitable for parenteral use is a sterile solution of an active component and water or a pharmaceutically acceptable vehicle other than water, or a suspension, for example, an injection. In general, desirable liquid carriers for injection include water, saline, aqueous solution of dextrose, solutions of other related sugars, ethanol, and glycols such as propylene glycol and polyethylene glycol. When an injection is prepared, an injectable form such as solution, suspension, or emulsion is prepared by a method known in the art using a carrier such as distilled water, Ringer solution, or physiological saline, an appropriate dispersing agent or wetting agent, and a suspending agent.

Examples of an aqueous solution for injection are physiological saline and isotonic solutions containing glucose and other auxiliary agents (e.g. D-sorbitol, D-mannitol, sodium chloride). A combined use with a pharmacologically acceptable suitable solubilizer such as alcohol (e.g. ethanol), polyalcohol (e.g. propylene glycol, polyethylene glycol), nonionic surface active agent (e.g. polysorvate 80™, HCO-50) is allowed. Oily liquids include sesame oil, soybean oil, and the like, and may be used in combination with benzyl benzoate, benzyl alcohol, or the like as a solubilizer. In addition, a buffer (e.g. phosphate buffer, sodium acetate buffer), reagent to adjust osmotic pressure, soothing agent (e.g. benzalkonium chloride, procaine hydrochloride), stabilizer (e.g. human serum albumin, polyethylene glycol), preservative (e.g. benzyl alcohol, phenol), antioxidant such as ascorbic acid, absorbefacient agent, and the like may be added. Prepared injection solution is usually filled in an appropriate ampule.

For parenteral administration, a form of a solution or suspension in a pharmaceutically acceptable sterile liquid such as water, ethanol, or oil with or without addition of a surface active agent and other pharmaceutically acceptable auxiliary agents is formulated. Oily vehicles or solvents used for formulation include glycerides such as natural, synthetic, or semi-synthetic mono-, di-, or triglycerides and natural, semi-synthetic, or synthetic oils and fats or fatty acids, and include, for example, vegetable oils such as peanut oil, corn oil, soybean oil, and sesame oil. For example, the injection can be formulated so as to contain a compound of the present invention generally at approximately 0.1 to 10% by weight.

Formulation suitable for local use, for example, oral or rectal use includes mouth rinsing agent, dentifrice, oral spray, inhalant, ointment, dental filler, dental coating agent, dental paste, suppository, and the like. Mouth rinsing agent and other dental agents are prepared by a conventional method using a pharmacologically acceptable carrier. Oral spray and inhalant can be applied to teeth and the like as a solution dissolving the compound of the present invention or a pharmacologically acceptable inactive carrier in a solution for aerosol or nebulizer or as a fine powder for inhalation. Ointment can be prepared by a conventional method by adding an ointment base (white vaselin, paraffin, olive oil, macrogol 400, macrogol ointment, etc.) and the like.

Drugs for local application to teeth and skin can be prepared as an adequately sterilized solution in water or a nonaqueous excipient or as a suspension. Additive agents include, for example, a buffer such as sodium hydrogen sulfite or edetate disodium, a preservative containing an antibacterial and antifungal agent such as phenylmercuric acetate or nitrate, benzalkonium chloride, or chlorhexidine, and a thickening agent such as hypromellulose.

A suppository is prepared such that the compound of the present invention is usually contained at 0.1 to 95% by weight using a carrier known in the art, preferably an appropriate nonirritating excipient, for example, polyethylene glycols, lanolin, cacao butter, fatty acid triglycerides, and preferably a carrier that is solid at ordinary temperatures but liquid at the temperature of the intestine and releases the drug by melting in the rectum. The drug can be suspended or dissolved in an excipient depending on the excipient and its concentration. Auxiliary agents such as local anesthetic, preservative, and buffer agent are soluble in the excipient.

A formulation suitable for oral use includes, for example, solid compositions such as tablet, pill, capsule, powder, granule, and troche, and liquid compositions such as liquid formulation, syrup, and suspension. When formulation is prepared, an auxiliary agent for formulation known in the art and the like are used. Tablets and pills can also be prepared by further applying enteric coating. When a unit dosage form is a capsule, a liquid carrier such as oil and fat can be included in the above type of materials.

When the active component is a protein or a polypeptide, its conjugation with polyethylene glycol (PEG) is particularly useful because its toxicity is extremely low in mammals. Further, the conjugation with PEG can sometimes reduce immunogenicity and antigenicity of a heterologous compound effectively. The compound may be put in a microcapsule. A polymer such as PEG can be easily attached to α-amino group of the amino-terminal amino acid, ε-amino group of lysine side chain, carboxyl group of aspargine acid or glutamic acid side chain, α-carboxyl group of the carboxyl-terminal amino acid, or activated derivative of glycosyl chain attached to certain asparagine, serine or threonine residue.

Many activated forms of PEG that are suitable for direct reaction with proteins are known. PEG reagents useful for reaction with amino groups of a protein include active esters of carboxylic acids and carbonate derivatives, particularly those having N-hydroxysuccinimide, p-nitrophenol, imidazole, or 1-hydroxy-2-nitrobenzene-4-sufonate as a leaving group. Similarly, PEG reagents having an aminohydrazine or hydrazide group is useful for reaction with aldehyde produced by periodate oxidation of protein.

Furthermore, when a nucleic acid such as DNA of the present invention is used as a therapeutic and/or preventive medicine as described above, the nucleic acid can be used alone or by binding to an appropriate vector that is used in gene recombination technology as described above, for example, a virus-derived vector such as retrovirus-derived vector, and so forth. The nucleic acid such as DNA of the present invention can be administered by a conventionally known method. It can be used alone or by formulating with an appropriate auxiliary agent, physiologically acceptable carrier, and the like so that incorporation into cells may be promoted, for example, and administered as a pharmaceutical composition, a pharmaceutical preparation, or the like as described above. In addition, a method known for gene therapy can also be applied.

The active component of the present invention can be administered by selecting its dose over a wide range, and the dose, dose frequency, and the like are determined depending on sex, age, body weight, and general condition of health of a patient, meal, dosing time, dosing method, excretion rate, drug combination, and severity of disease condition of the patient undergoing a treatment at that time, or by taking these and other factors into consideration.

For the production of the drug product, additives, production methods, and the like can be selected and applied as appropriate by referring to the descriptions in, for example, Editorial committee for guide of Japanese pharmacopoeia (ed.), "Dai 14 Kaisei Nihon Yakkyokuhou Kaisetsusho," Hirokawa Shoten, Tokyo (June 27, 2001); H. Ichibagase et al.(eds.), "Iyakuhin no Kaihatsu Vol. 12 (Seizaisozai [I]), Hirokawa Shoten, Tokyo (Oct. 15, 1990)"; H. Ichibagase et al.(eds.), "Iyakuhin no Kaihatsu Vol. 12 (Seizaisozai [II])," Hirokawa Shoten, Tokyo (Oct. 28, 1990), (all in Japanese).

The active component of the present invention is not particularly limited as long as the component has a biological activity such as regulating (promoting or suppressing and inhibiting) the activity (e.g. bioactivity such as binding activity) of the interaction between p30 and Rap1 but preferably has an advantageous effect. The active component of the present invention includes, for example, (a) a modified p30 protein or its mutant polypeptide, a partial peptide thereof, a salt thereof, and the like, (b) a nucleic acid such as DNA encoding the protein and DNA encoding a mutant polypeptide of the protein, (c) an antibody of the present invention, a partial fragment thereof (including a monoclonal antibody), or a derivatives thereof, (d) a compound, a salt thereof, or the like having an advantageous effect on a biological activity such as regulating (promoting or suppressing and inhibiting) an interaction with a biocomponent that arises from the interaction between p30 and Rap1.

The active component of the present invention is expected to be useful for regulating (promoting or suppressing and inhibiting) changes in various tissues or cells resulting from the interaction between p30 and Rap1. Further, the active component is useful for not only regulating an activity expression of p30 or the activated form of Rap1 but also regulating (promoting or suppressing and inhibiting) the p30-Rap1 binding, and useful for preventing or treating impairments, disorders and/or diseases arising from the interaction. Furthermore, the active component is expected to be useful for regulation, for example, suppression, of for example, locomotion, invasion, migration, and/or metastasis of tumor cells and the like in which the p30-Rap1 binding is involved, for example, useful for their suppression.

The active component of the present invention, for example, the p30-Rap1 binding inhibitor, is useful as a drug for treatment of inflammatory diseases, autoimmune diseases, suppression of immunological rejection after organ transplantation, cancer, and the like, and can be expected to be used, for example, for treatment of gastric ulcer, duodenal ulcer, acute pancreatitis, bronchitis, ARDS (acute respiratory distress syndrome), COPD (chronic obstructive pulmonary disease), septic shock, MOF (multiple organ failure), SIRS (systemic inflammatory response syndrome), systemic lupus erythematosus, mixed type connective tissue disease, chronic rheumatoid arthritis, Sjogren's syndrome, rheumatic fever, Goodpasture's syndrome, Graves' disease, Hashimoto's disease, Addison's disease, autoimmune hemolytic anemia, idiopathic thrombocytopenic purpura, myasthenia gravis, ulcerative colitis, Crohn's disease, sympathetic ophthalmia, multiple sclerosis, psoriasis, allergic rhinitis, asthma, and the like, and for suppression of immunological rejection at the time of organ transplant by treatment before the organ transplant or by administration after the organ transplant, further for suppression of carcinogenesis and metastasis, and the like.

The dosage of the compound or the salt thereof (for example, the compound represented by formula (I)) of the present invention that promotes or inhibits the interaction and/or the binding between Rap1 and p30 varies depending on target diseases, subjects to be treated, routes of administration, and the like. When administered orally, the desired dose is from 10 µg to 10 g/kg, preferably from 100 µg to 1 g/kg, and more preferably from 1 mg to 100 mg/kg. When administered by intravenous drip infusion, the desired dose is from 0.01 µg to 1 g/kg/hrx6hr, preferably from 0.1 µg to 100 mg/kg/hrx6hr, and more preferably from 1 µg to 10 mg/kg/hrx6hr. When administered by a single intravenous injection, the desired dose is from 1 µg to 1 g/kg, preferably from 10 µg to 100 mg/kg, and more preferably from 100 µg to 10 mg/kg. When administered intraperitoneally, the desired dose is from 1 µg to 10 g/kg, preferably from 10 µg to 1 g/kg, and more preferably from 100 µg to 100 mg/kg.

### Example

The present invention is specifically explained by means of the following examples, but these examples are presented merely for explanation of the present invention and for reference of specific modes thereof. Although these examples are for explanation of particularly specific modes, these are in no way intended to limit or restrict the scope of the invention disclosed in the present application. In the present invention, it should be understood that various embodiments can be implemented based on the spirit and scope of the present specification.

It should be noted that methods required for general gene recombination such as cleavage and ligation of DNA, transformation of E.coli, gene sequencing, and hybridization were basically performed according to manuals attached to commercially available reagents, instruments, and the like that were used for each operation, and experimental guidebooks (for example, "Molecular Cloning," Maniatis T. et al., Cold Spring Harbor Laboratory Press). All examples were performed or could be performed by standard technologies except for those otherwise described in detail, and these are conventional and known to a person skilled in the art.

### Example 1

### (Isolation of p30 associated with Rap1)

To identify a molecule that associates with Rap1, the human leukocyte cDNA library was screened by a yeast two-hybrid method using a Rap1-activated mutant Rap1V12 as bait. As a result, a molecule that encodes a protein of a molecular weight of 30,000 having a Ras/Rap binding domain (RBD) was isolated and termed as p30 (Fig.1). Search through a database revealed that the amino acid sequence of p30 was identical to Nore1 at the C-terminal side including RBD domain (Fig. 1). It was found from an analysis of the human genome database that p30 is an alternative splicing product of Nore1 Mouse p30 cDNA was also isolated by a RT-PCR method.

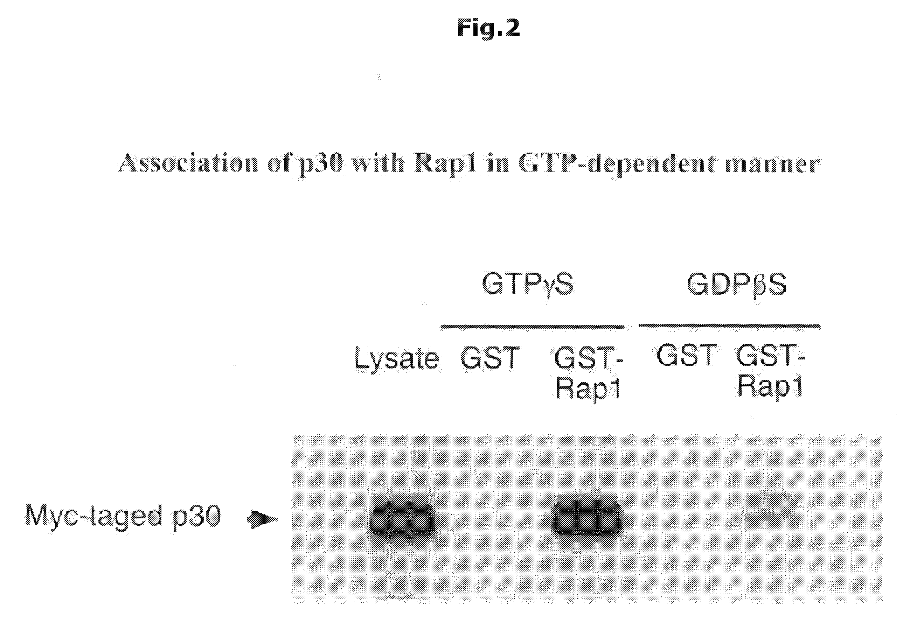

Whether or not p30 binds to Rap1 was studied. For this purpose, a protein (GST-Rap1) in which the N-terminal side of Rap1 was fused to GSH (glutathione-S-transferase) was produced in E. coli and purified with a glutathione affinity column. Further, Myc-p30 gene in which Myc epitope was added to the N-terminal side of p30 cDNA was transfected to COS cells to prepare a lysate expressing p30 in a large amount. Since Rap1 is converted to an active form by binding to GTP and an inactive form by binding to GDP, GST-Rap1 was allowed to bind to GTPγS and GDPβS, respectively, to prepare the active form and the inactive form, followed by mixing with the COS cell lysate containing Myc-p30. Then, after mixing by inversion at 4°C for 2 hours, GST-Rap1 was recovered by adding glutathione-bound beads. The detection of myc-p30 bound to Rap1 was carried out by using an anti-Myc mouse IgG2a antibody (product of Cell Signaling) as a primary antibody and a HRP-labeled anti-mouse IgG (product of Sigma) as a secondary antibody. Then a film for chemiluminescence (product of Amersham) was exposed according to ECL chemiluminescence (Fig. 2). As a result, it was found that p30 was bound specifically to the activated form of Rap1. Thus, p30 was considered to be a molecule that functions depending on the activation of Rap1 from its property of binding to the activated form of Rap1.

### Example 2

### (Tissue distribution of p30 expression by RT-PCR)

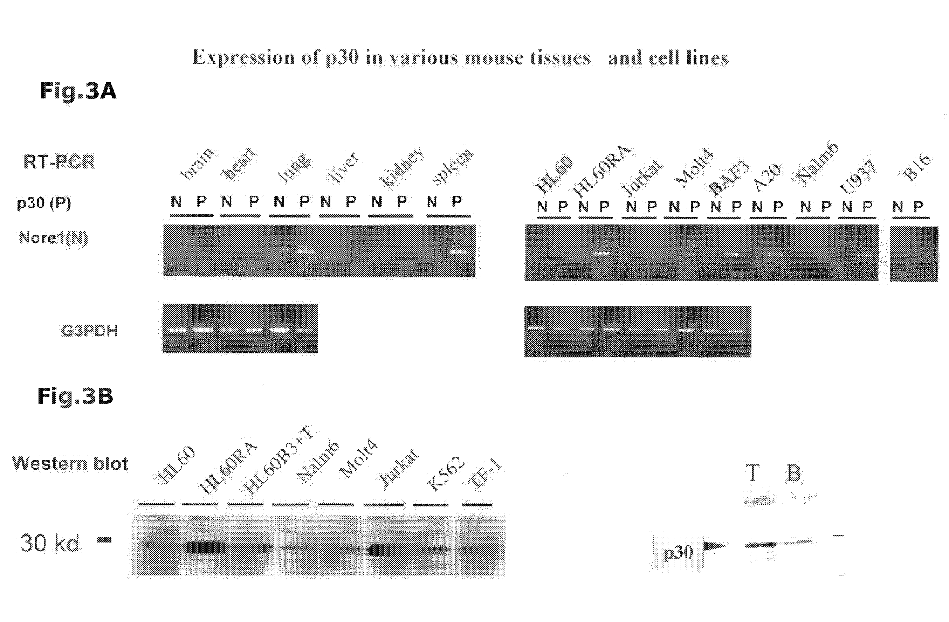

To examine p30 expression in each tissue, the expression of p30 mRNA was studied by RT-PCR. In addition, the expression of the analogous molecule Nore1 was also studied for comparison. RNAs were extracted from the brain, heart, lung, liver, kidney, and spleen and their cDNAs were synthesized, followed by detection of each cDNA of p30 (P) and Nore1 (N) (Fig. 3A). As a control of mRNA amount, glucose-3-phosphate dehydrogenase (G3PDH) was used. As a result, the expression of p30 was strongly observed in the spleen and the lung. In contrast, Nore1 was mainly expressed in the brain. Further, when expressions of p30 and Nore1 in various cell lines derived from blood cells and immune cells were studied by a similar method, p30 was expressed in myelogenic cell lines (HL60, U937), T-lymphoid cell lines (Jurkat, Molt4), and B-lymphoid cell lines (BaF3, A20, Nalm6), whereas the expression of Nore1 was not observed in these cells. In contrast, p30 was not expressed in a melanoma cell line (B16), while the expression of Nore1 was confirmed in the cell line. The undifferentiated myelogenic cell HL60 is known to differentiate into neutrophil by treating with retinoic acid, and it was confirmed by RT-PCR that this treatment enhanced the expression of p30 mRNA (HL60RA).

### Example 3

### (Preparation of monoclonal antibody against p30)

In order to study the expression of p30 protein, monoclonal antibodies were prepared.

A mouse p30 cDNA was subcloned into pGEX vector (product of Amersham Pharmacia) and introduced into E. coli (BL21), thereby producing GST-mp30 in which GST was fused to the N-terminal side of mP30. The transfected BL21 was grown in 400 mL of LB medium at 30°C. When its growth reached a OD₆₀₀ of 0.5, IPTG (0.2 mM, product of Amersham Pharmacia) was supplemented and further cultured for 4 hours. The BL21 cells were precipitated by centrifugation at 6,000 rpm, washed once with PBS, suspended in 5 ml of PBS, and then disrupted with an ultrasonic disintegrator (product of Tomy Digital Biology, UD-200) under a condition of an output 6 for 15 sec four times. After centrifugation at 9,000 rpm, the supernatant was passed through a glutathione column (product of Amersham Pharmacia) for binding, washed with 100 ml of PBS, eluted then with glutathione (5 mM), and dialyzed against PBS to obtain a preparation of a purified antigen.

The purified GST-human p30 and a complete adjuvant (product of Difco) were mixed and injected into the skin of the sole of a 8-week old female rat (WKY/NCrj obtained from Oriental Bio Service) at 0.2 mg/0.1 ml. One week later, GST-mp 30 (0.1 mg/0.1 ml) mixed with an incomplete adjuvant (product of Difco) was again injected into the sole. After 5 days, an ilium lymph node was extracted and subjected to cell fusion with a myeloma cell line Sp2/0 using polyethylene glycol, and the hybridomas were cultured in a HAT medium (product of Gibco BRL) ("Manual for Monoclonal antibody Production," Tada, N., Gakusai Kikaku, Tokyo (1995) (in Japanese)). After 12 days, the culture supernatant was recovered and measured for the antibody against p30 using an ELISA method.

An antigen for use in the ELISA was produced by expressing MBP-hp30 having fused MBP (maltose-binding protein) in E. coli and purified. MBP-hp30 was prepared as a fusion protein of hp30 with maltose binding protein (pML, Protein Fusion and Purification System, New England Biolabs) by subcloning human p30 cDNA into pMAL vector (product of New England Biolabs). The MBP-hp30 (0.2 mg/0.2 ml/well) was immobilized on a 96-well plate, and the antibody in the culture supernatant was detected by a sandwich method ("Manual for Monoclonal antibody Production," Tada, N., Gakusai Kikaku, Tokyo (1995) (in Japanese)). The supernatants that were positive were assayed by a western blot method using COS cell transfected with human p30 cDNA and a immunostaining method. Six hybridomas that were positive in these assays were cloned by limiting dilution. For epitope mapping, an N-terminal deletion mutant of p30 (containing RBD domain and its C terminal side), its C-terminal deletion mutant (containing its N-terminal side and RBD domain), and its RBD domain deletion mutant (containing only its N-terminal and C-terminal sides) were expressed in COS cells. When these were assayed by western blotting, four hybridomas (B1.2, B3.7, E11.2, G7.3) were found to produce antibodies that recognize the N-terminal side, and two hybridomas (B4.1, H10.5) were found to produce antibodies that recognize the C-terminal side. The antibodies that recognize the C-terminal side reacted with Nore1 as well, while all of the four antibodies that recognize the N-terminal side did not react with Nore1 and were found to be p30-specific antibodies. The result of a western blot with use of the specific anti-p30 antibody E11.2 against blood and immune cell lines as well as T-lymphocyte and B-lymphocyte purified from lymph nodes is shown (Fig. 3B). HL60 cell differentiated into neutrophil by treatment with retinoic acid (HL60 RA) and HL60 cell differentiated into macrophage by stimulation with vitamin D3 (1 mM) and TPA (10 ng/ml) (HL60 D3+T) showed an enhanced expression of p30 compared to that in HL60. The expression was also observed in Nalm6 (B-lymphoid), Molt4, Jurkat (T-lymphoid), K562 (erythroblastoid), and TF-1 (myeloid) (Fig. 3B).

### Example 4

### (Effect of p30 on cell migration)

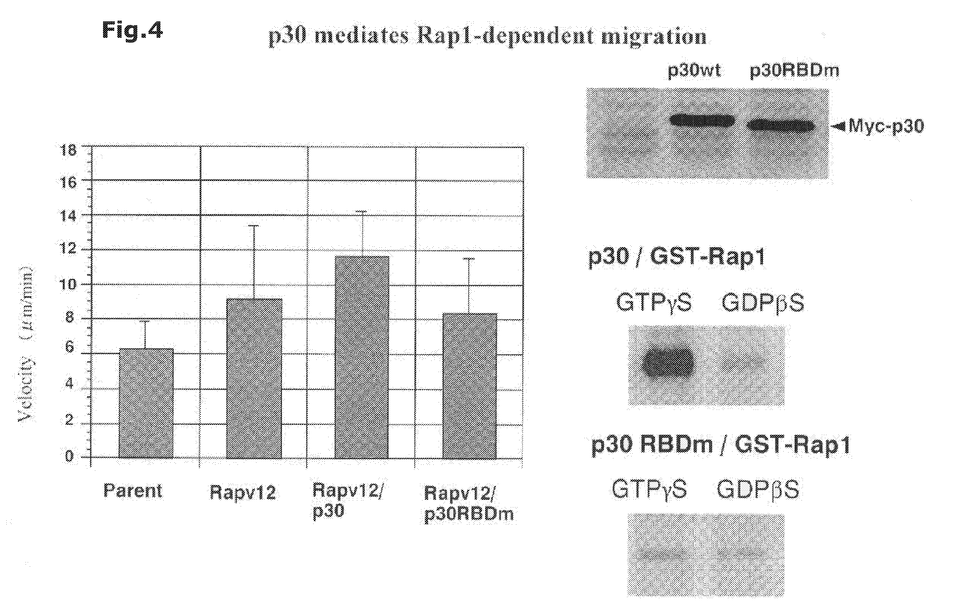

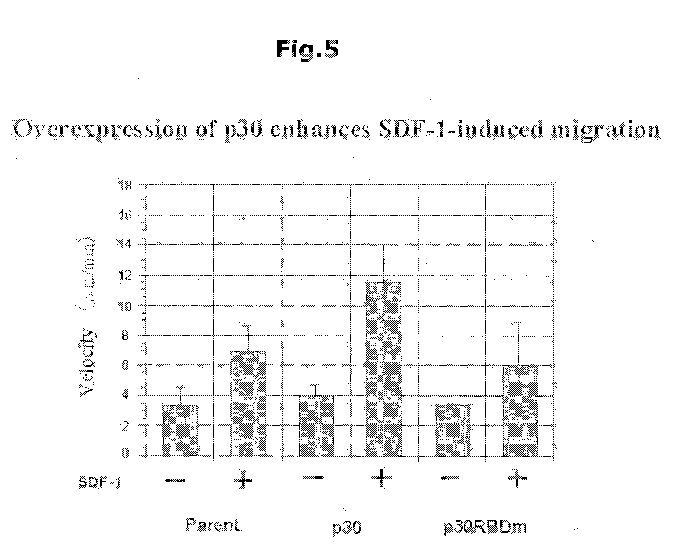

(1) cell migration when wild type p30 and Rap1 were coexpressed
   In order to study what kind of effect p30 exerts on cell migration induced by the activated form of Rap1 (Rap1V12) or a chemokine, BAF cells engineered to express p30, Rap1V12, and both Rap1V12 and p30, respectively, using proB cell line, BAF cells, that were transformed to express human LFA-1 were prepared, and cell migration on ICAM-1 was measured. As the ICAM-1, a protein (hICAM-1-Fc) in which the extracellular region of human ICAM-1 was fused to the Fc region of human immunoglobulin was used. For cell migration, ΔT Culture Dish system (product of Biotechs, Inc.) was used. The prepared BAF cells were added on a temperature controlled plate having a diameter of 6 cm that had been immobilized with ICAM-1 (0.1 mg/ml) and videographed at 37°C for 20 min. In each experiment, distance of migration was measured for 20 cells, and the average rate was calculated. As a result, Rap1V12 increased the rate of cell migration on ICAM-1 and p30 further potentiated its effect (Fig. 4).
(2) Cell migration when RBD-domain mutant of p30 and Rap1 were coexpressed
   An RBD-domain mutant of p30 (p30RBDm) was prepared in order to study whether the potentiation effect of p30 occurs via binding with Rap1 in this system. The p30RBDm is a mutant in which seven amino acids were conserved in RBD domain, that is, 123th lysine, 124th arginine, 135th lysine, 154th lysine, 155th lysine, 160th asprartic acid, and 161th asparagine, were substituted with alanine. Although this p30RBDm was expressed as in the case of the wild type, it was unable to bind to GTP-Rap1, which is different from the wild type. When Rap1V12 and p30RBDm were coexpressed in the BAF cell and the rate of migration on ICAM-1 was measured, no potentiation was observed. Therefore, the promotion effect of p30 on cell migration by Rap1V12 requires its binding to Rap1.
   Next, the effect of p30 on cell migration by a chemokine was studied. Each of p30 and p30RBDm was introduced into the BAF cells transformed to express human LFA-1, and cell migration on ICAM-1 was measured by the above method in the presence of SDF-1 (CXCL12) (20 nM). As a result, p30 enhanced the cell migration stimulated by SDF-1 about two fold, whereas p30RBDm did not show any such effect. From the foregoing, it was found that p30 has an activity to enhance the cell migration by the chemokine via binding to Rap1 (Fig. 5).

### Example 5

### (Regulation of LFA-1 adhesion by p30)

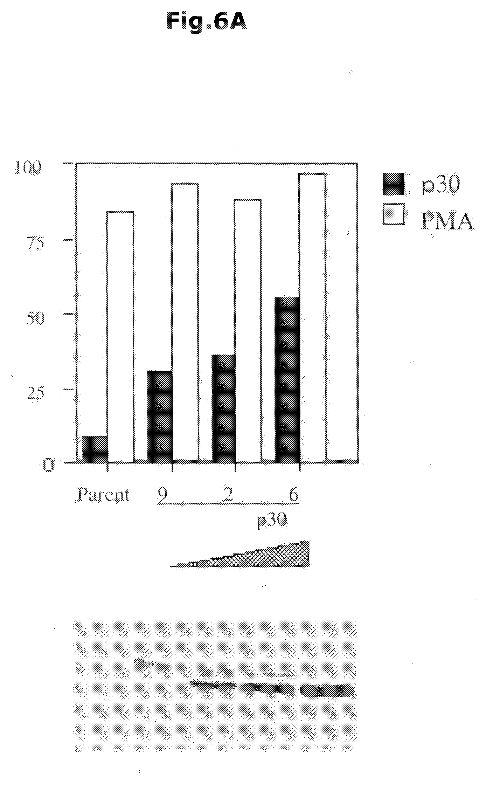

In order to explore the possibility that p30 participates in enhancement of adhesion activity as a downstream effector molecule of Rap1 p30 was overexpressed in 3A9 T cell, and an effect of p30 on the adhesive activity of LFA-1 of 3A9 T cell to ICAM-1 was studied by an adhesion assay. That is, the extracellular region of mouse ICAM-1 was fused to the Fc-region of human immunoglobulin to produce a dimeric chimera protein, which was immobilized on a plate, and adhesive activity of the cell to the ICAM-1 was measured. The cells were labeled with a fluorescent dye using BCECF-AM (product of Molecular Probe) and incubated on a plate immobilized with ICAM-1 (0.1 mg/ml) at 37°C for 30 min. The number of input cells and the number of the cells that remained after washing the plate more than three times (the number of adhering cells) were measured with a fluorescence reader (Cytofluor 4000, product of Persepetive Biosystems), and a proportion of the input cells to the adhering cells was defined as the adhesive activity of the cell. As a result, it was found that the adhesive activity of LFA-1 to ICAM-1 was enhanced depending on the level of p30 expression in the 3A9 T cell (Fig. 6A).

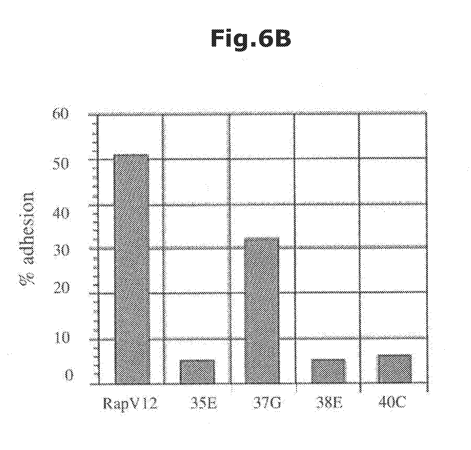

If p30 participates in adhesion in the downstream of Rap1 an enhancement of the adhesion should not be observed in a Rap1 mutant that has lost the binding activity to p30. The amino acid sequence called the effector region of Rap1 has been proved to be the region to which downstream effector molecules beginning with p30 bind. Hence, a point mutation was introduced into this region of the active Rap1V12. Mutants of Rap1 in which 35th threonine was replaced with glutamic acid (T35E), 37th glutamic acid with glycine (E37G), 38th aspartic acid with glutamic acid, (D38E), and 40th tyrosine with cysteine (Y40C) were prepared, respectively. Based on the association with Rap1V12, it was found that E37G retained a binding activity to p30, whereas T35E, D38E, and Y40C could not bind to p30. Subsequently, these mutants were introduced into 3A9 T cells. As a result, an enhancement of adhesion of LFA-1 to ICAM-1 was observed only for E37G that retained a binding activity to p30 (Fig. 6B). This indicated the possibility that p30 participates in enhancement of adhesion in the downstream of Rap1.

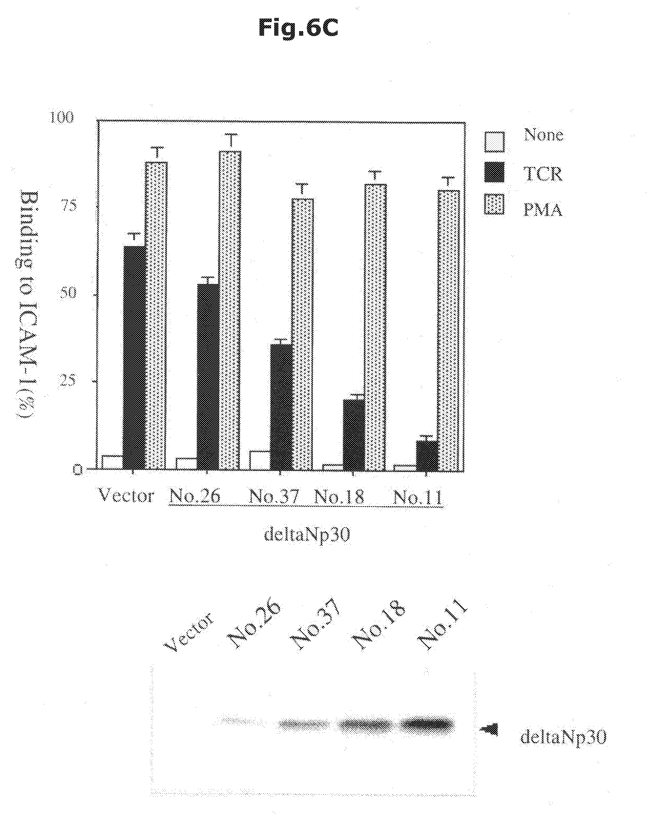

It has been uncovered that LFA-1/ICAM-1 adhesion induced by TCR stimulation is completely suppressed by expressing the Rap1-specific inhibitory molecule Spa-1 and is therefore mediated by Rap1 activation. If p30 participates in adhesion in the downstream of Rap1 it is expected that p30 suppresses LFA-1/ICAM-1 adhesion via TCR. Therefore, a mutant of p30 that functions in a dominantly negative fashion (deltaNp30) was prepared. DeltaNp30 was prepared by deleting 100 amino acids from the N-terminus of p30. After introduction into 3A9 T cell, LFA-1/ICAM-1 adhesion via TCR stimulation was studied. As a result, it was found that deltaNp30 suppressed the adhesion of LFA-1 to ICAM-1 via TCR stimulation depending on the level of deltaNp30 expression as shown in Fig. 6C.

From these results, it has become apparent that p30 plays an important role in cell adhesion via LFA-1/ICAM1 in the downstream of Rap1.

### Example 6

### (Effect of N-(2-ethylsulfonylamino-5-trifluoromethyl-3-pyridyl)cyclohexanecarboxamide monosodium salt monohydrate (compound 1) on the binding of Rap1 and p30)

(1) Preparation of GST-Rap1 beads
   For a plasmid for production of GST-Rap1 fusion protein, pGEX-3X (product of Amersham Pharmacia Biotech) was used. The Rap1 coding sequence represented by SEQ ID N0:1 was inserted between BamHI and EcoRI restriction enzyme sites of the vector. At this time, two nucleotide pair CC were added in front of the 5' terminal ATG of the nucleotide sequence represented by SEQ ID NO:1 for frame adjustment. As a result, the linkage between the BamHI site of the vector and Rap1 resulted in 5'...GGATCCCCATG...3'. The obtained plasmid for production of GST-Rap1 fusion protein was transfected into E. coli. The E. coli with the inserted plasmid was cultured, and GST-Rap1 protein was produced within E. coli cells by inducing the protein production with 0.2 mM IPTG (isopropylthiogalactopyranoside) (30°C, 2 hours). The E. coli cells were recovered by centrifugation, solubilized in a lysis buffer (100 mM NaCl, 50 mM Tris-HCl (pH 7.5), 1% Triton X100, 2 mM MgCl₂, 0.1 TIU/ml Aprotinin), and disrupted by sonication. The solution was centrifuged, and then glutathione Sepharose 4B beads (product of Amersham) was added to the obtained supernatant and reacted at 4°C for one hour to prepare GST-Rap1 beads.
(2) Preparation of p30 lysate
   A plasmid of p30-Myc tag was introduced into 293T cells in 70% confluency by electroporation. The transfected cells were cultured for 2 days and collected by centrifugation. The recovered cells were solubilized in a lysis buffer, and the supernatant was recovered by centrifugation to obtain p30 lysate.
(3) Binding inhibition assay
   GST-Rap1 beads (25 µl) were washed with a loading buffer (25 mM Tris-HCl (pH 7.5), 2 mM EDTA, 2.5 mM MgCl₂, 1 mM DTT) and added with 2 mM GTPγS (product of Sigma), followed by incubation at 37°C for 15 min to activate Rap1. To this was added 250 µl of the p30 lysate and reacted at 4°C for 30 sec. Immediately before this reaction of 30 sec, N-(2-ethylsulfonylamino-5-trifluoromethyl-3-pyridyl)cyclohexanecarboxamide monosodium salt monohydrate (compound 1) was added at a final concentration of 1 µM. After the reaction, the beads were washed four times with the lysis buffer. To the beads precipitated by centrifugation was added 25 µl of a conventional sample buffer for SDS-PAGE to prepare a sample for SDS-PAGE. This sample was subjected to SDS-PAGE, followed by western blotting. Detection of p30 was carried out by using an anti-Myc mouse IgG2a antibody (product of Cell Signaling) as a primary antibody and a HRP-labeled anti-mouse IgG (product of Sigma) as a secondary antibody and then exposing to a film for chemiluminescence (product of Amersham) according to ECL chemiluminescence method.

The bands visualized by exposing to the film for chemiluminescence were quantitated with use of image analyzing software (Win Roof, product of Mitani Corp.). That is, p30 bands to be compared were extracted by binarization processing and compared in terms of the volume defined by brightness x area.

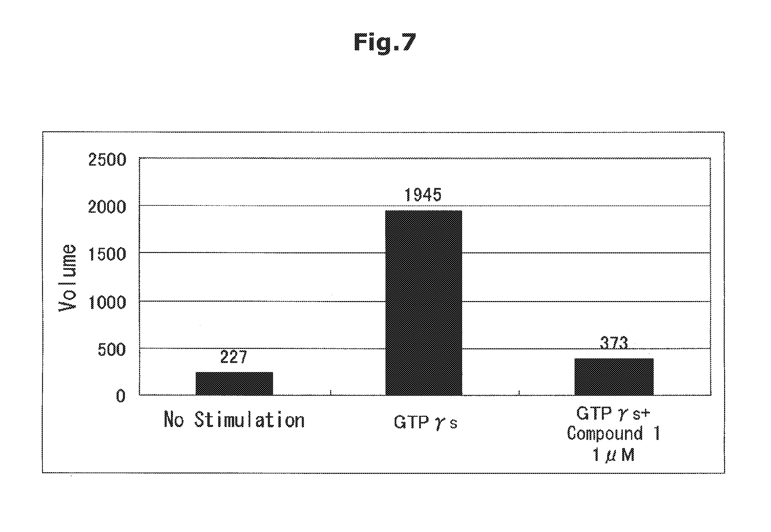

This value for a group treated with 1 µM of the compound 1 was found to be significantly lower compared to that of a group without its addition (Fig. 7). From this, it is considered that the compound 1 inhibits the binding between Rap1 and p30.

According to the above method, it seems that screening of a compound useful for inhibiting the binding between Rap1 and p30 can be carried out.

In a similar way, activities of various diaminotrifluoromethylpyridine derivatives disclosed, for example, in Japanese Published Unexamined Patent Application No. H06-263735 can be screened.

### Example 7

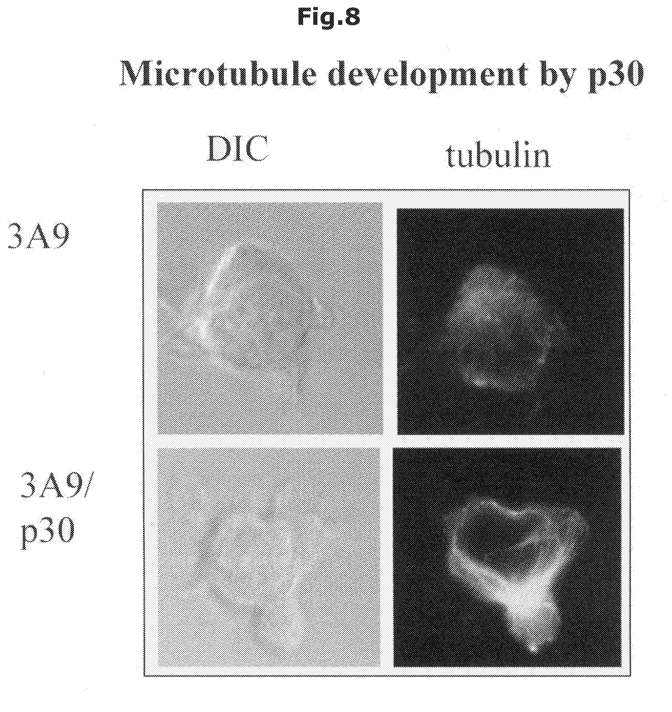

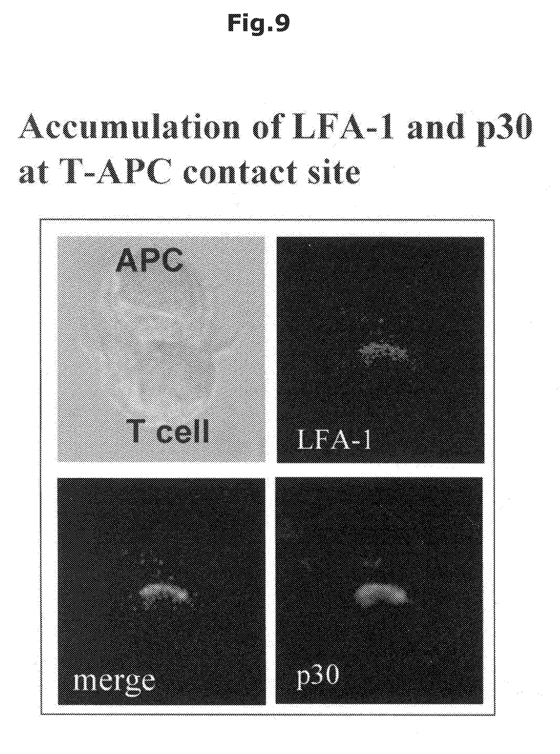

(1) Development of microtubule by p30
   In order to study an effect of p30 on microtubule cytoskeleton, the human p30 gene was introduced into the 3A9 T cell. An untreated 3A9 T cell and the cell introduced with p30 (3A9/p30) were mounted on a slide glass coated with poly-L-lysine, respectively, and fixed with 3.3% paraformaldehyde at room temperature for 15 min. After washing with PBS, PBS/0.2% Tx-100 containing 2 mM MgCl₂ was added and incubated for 5 min. After washing again with PBS, blocking was performed with 5% goat serum for 20 min. Then, mouse anti-tubulin antibody (diluted 500-fold with 1% BSA) was added and incubated for one hour. After washing four times with PBS/0.1% BSA, 400-fold diluted Alexa 488-labeled goat anti-mouse Ig G (product of Molecular Probe) was added, incubated for one hour, and washed four times with PBS/0.1% BSA, followed by observation under a confocal laser scanning microscope (Fig. 8). As a result, it became apparent that introduction of p30 resulted in deformation of the cell and a significant enhancement of development of microtubule. From this, it was found that p30 has a role in development of microtubules.
(2) Localization of p30 and LFA-1 in adhesion between T-cell and antigen presenting cell
   Adhesion formation between a T cell and an antigen presenting cell (APC) is an important adhesion that allows the T cell to recognize the antigen, and this adhesion is mediated by LFA-1/ICAM-1. Hence, the localization of p30 and LFA-1 was studied. For this study, HEL (hen egg lysozyme) specific 3A9 T cell and CH27 cell were used as the T cell and APC cell, respectively. CH27 cells (1x10⁵/ml) were cultured for 16 hours in the presence of an added HEL antigen (100 µg). Then, 3A9 T cells and CH27 cells were mixed in the same number (1x10⁵/ml), respectively, and incubated at 37°C for 30 min. These cells were mounted on slide glass coated with poly-L-lysine, and fixed with 3.3% paraformaldehyde at room temperature for 15 min. After washing with PBS, PBS/0.2% Tx-100 containing 2 mM MgCl₂ was added and incubated for 3 min. After washing again with PBS, blocking was performed with 5% goat serum for 20 min. Then, rat anti-p30 antibody (10 µg/ml) (E11.2) was added and incubated for one hour. After washing four times with PBS/0.1% BSA, Alexa 546-bound rat anti-mouse IgG (diluted 500-fold with 1% BSA, product of Molecular Probe) was added, and incubated for one hour. Further, a biotinylated mouse anti-LFA-1 antibody (diluted 100-fold with 1% BSA, product of Pharmingen) was added and incubated for one hour. After washing four times with PBS/0.1% BSA, an FITC-labeled anti-biotin antibody (product of Jackson Laboratory) diluted 100-fold with 1% BSA was added, incubated for one hour, and washed four times with PBS/0.1% BSA, followed by observation under a confocal laser scanning microscope (Fig. 9: the photograph of p30 in the lower right is hardly recognizable because it is too dark when displayed in black and white, but observable in its center portion as red signals when displayed in color, and LFA is observable as green signals). As a result, LFA-1 and p30 were accumulated at the T-APC adhesion surface and colocalized (merged). This localization is in good agreement with the function of regulation of LFA-1 by p30.

### Example 8

### Production of transgenic mouse with high expression of p30

(1) Preparation of DNA fragment used for production of transgenic mouse
   A DNA fragment having spacer sequences provided with EcoRI site that were formed at both ends of the p30 mouse gene (SEQ ID NO:9) cloned from a library is prepared and inserted into the vector pCNX2 having a CMV enhancer sequence and a CAG promotor sequence (supplied by Prof. Honjo, Kyoto Univ.) that was cleaved by EcoRI (product of Takara Bio). Then the plasmid was cleaved with Sal I-Hind III, thereby preparing a DNA fragment (3,075 bp) for use in microinjection, that is, a DNA in which the p30 gene is expressibly ligated to the downstream of the CMV enhancer sequence and the CAG promotor sequence.
(2) Gene transfer into mouse
   The preparation of a transgenic mouse was carried out by a modification of the method of Gordon et al. (Proc. Natl. Acad. Sci., 77:7380-7384 (1980)).
   First, female mice C57BL/6N Crj (obtained from Charles River Japan) were subjected to superovulation induction (by administration of 5 IU of pregnancy horse serum gonadotropin and subsequent administration of human placental gonadotropin 48 hours later), and then allowed to live with male mice of the same strain for mating. Next morning, an oviduct was extracted from a female mouse showing vaginal plug formation, and pronuclear fertilized ova were collected.
   A solution of the DNA fragment prepared above (prepared at several ng/µl in a TE buffer) was microinjected into each male pronucleus of the fertilized ova using a microcapillary for gene injection, followed by culturing for 2 to 3 hours in a CO₂ incubator.
   After the incubation, morphologically normal embryos were selected and transplanted into the oviduct of a pseudo pregnant female mouse for emryo transplantation (Jcl:MCH(ICR), (obtained from Clea Japan)). For this pseudo pregnant female mouse, estrous female mice were allowed to live together overnight with deferens ligated male mice, and a female mouse showing vaginal plug formation the next morning was used. The transplantation of the embryo into the oviduct was carried out as follows: the back of an anesthetized female mouse was cut open, its ovary was picked up, a portion between the fimbria of the fallopian tube and the ampulla of the fallopian tube was incised, and the culture medium containing the embryo, the air, and the culture medium that were filled in the tip of a Pasteur pipette for embryo transplantation were injected in this order.
   After the transplantation, the female mouse (surrogate mother) that was sutured was put in a vinyl isolator, allowed to deliver naturally, and maintained until weaning. When birth was not observed by the afternoon of the expected date of confinement, neonates were extracted by hysterotomy and the neonates were raised by a foster (specific pathogen free: SPF) prepared beforehand in another isolator. At the time of weaning, a microbiological test was performed using the surrogate mother or the foster mother, and the newborn whose parent was confirmed to be SPF were transferred to an SPF rearing cage, followed by gene analysis. After the gene analysis, the newborn carrying the introduced gene were used to confirm the transmission of the introduced gene to the next generation.
(3) Analysis of introduced gene
   After transferring to the SPF rearing cage (after confirmation of weaning and SPF state), collection of tail tissue was performed by cutting off the tail at a position of ca. 10 to 15 mm from the tip with a pair of special scissors sterilized beforehand by dry heat, and the obtained tissue sample was put in a sterilized Eppendorf tube and stored at -80°C until analysis.

DNA was extracted from the above tail tissue stored frozen by a method of treating with protease K (product of Amersham Biosciences: cord; 27-2537-01). The mouse bearing the above introduced gene fragment was confirmed by PCR using the primers designed from the 5'-side of p30 gene and the 3'-side of the vector portion in the immediate downstream of the former [ATGACCGTGGACAGCAGCATGAGCAGCGGG (SEQ ID NO:11) and TATTTGTGAGCCAGGGCATTGGCCACACCA (SEQ ID NO:12), respectively]
and the extracted DNA as the template. The introduction of the above gene was confirmed by this PCR in which the gene fragment of 1144 bp was amplified. Further, the introduced gene was confirmed by PCR against the CMV promoter present at the 5'-side of the introduced gene 
〔using ATCAATTACGGGGTCATTAG (SEQ ID NO:13) and
TGTACTGCCAAGTAGGAAAG (SEQ ID NO:14) as the primers〕.
 The introduction of the above gene was confirmed by this PCR in which the gene fragment of 320 bp was amplified.

Hereinafter, the DNA nucleotide sequences used in the present specification are explained and disclosed.
(1) Myc-tag (a sequence that allows the protein to be produced in a cell and detected by an antibody against myc and contains an initiation codon) ATGGAACAGAAACTCATATCGGAGGAGGATCTA 〔SEQ ID NO:7〕
(2) Nucleotide sequence of the wild type p30 (when the above myc-tag is attached, it is replaced with the initiation codon ATG) 
ATGACCGTGG ACAGCAGCAT GAGCAGTGGG TACTGCAGCC TGGACGAGGA
ACTGGAAGAC TGCTTCTTCA CTGCTAAGAC TACCTTTTTC AGAAATGCGC
AGAGCAAACA TCTTTCAAAG AATGTCTGTA AACCTGTGGA GGAAACACAG
CGCCCGCCCA CACTGCAGGA GATCAAGCAG AAGATCGACA GCTACAACAC
GCGAGAGAAG AACTGCCTGG GCATGAAACT GAGTGAAGAC GGCACCTACA
CGGGTTTCAT CAAAGTGCAT CTGAAACTCC GGCGGCCTGT GACGGTGCCT
GCTGGGATCC GGCCCCAGTC CATCTATGAT GCCATCAAGG AGGTGAACCT
GGCGGCTACC ACGGACAAGC GGACATCCTT CTACCTGCCC CTAGATGCCA
TCAAGCAGCT GCACATCAGC AGCACCACCA CCGTCAGTGA GGTCATCCAG
GGGCTGCTCA AGAAGTTCAT GGTTGTGGAC AATCCCCAGA AGTTTGCACT
TTTTAAGCGG ATACACAAGG ACGGACAAGT GCTCTTCCAG AAACTCTCCA
TTGCTGACCG CCCCCTCTAC CTGCGCCTGC TTGCTGGGCC TGACACGGAG
GTCCTCAGCT TTGTGCTAAA GGAGAATGAA ACTGGAGAGG TAGAGTGGGA
TGCCTTCTCC ATCCCTGAAC TTCAGAACTT CCTAACAATC CTGGAAAAAG
AGGAGCAGGA CAAAATCCAA CAAGTGCAAA AGAAGTATGA CAAGTTTAGG
CAGAAACTGG AGGAGGCCTT AAGAGAATCC CAGGGCAAAC CTGGGTAA
[SEQ ID NO:3]
(3) Dominant negative type of p30 (an initiation codon present in front of 101th alanine from the N-terminus; note that deltaNp30 used in Fig. 6C is attached with the above myc-tag in place of the initiation codon)

ATGGCTGGGATCC GGCCCCAGTC CATCTATGAT GCCATCAAGG AGGTGAACCT
GGCGGCTACC ACGGACAAGC GGACATCCTT CTACCTGCCC CTAGATGCCA
TCAAGCAGCT GCACATCAGC AGCACCACCA CCGTCAGTGA GGTCATCCAG
GGGCTGCTCA AGAAGTTCAT GGTTGTGGAC AATCCCCAGA AGTTTGCACT
TTTTAAGCGG ATACACAAGG ACGGACAAGT GCTCTTCCAG AAACTCTCCA
TTGCTGACCG CCCCCTCTAC CTGCGCCTGC TTGCTGGGCC TGACACGGAG
GTCCTCAGCT TTGTGCTAAA GGAGAATGAA ACTGGAGAGG TAGAGTGGGA
TGCCTTCTCC ATCCCTGAAC TTCAGAACTT CCTAACAATC CTGGAAAAAG
AGGAGCAGGA CAAAATCCAA CAAGTGCAAA AGAAGTATGA CAAGTTTAGG
CAGAAACTGG AGGAGGCCTT AAGAGAATCC CAGGGCAAAC CTGGGTAA
(corresponding to SEQ ID NO:5)

### Industrial Applicability

The present invention provides a technology making use of knowledge that cell activation and regulation of signal transduction are effected by the interaction between p30 and Rap1 The use of the present technology makes it possible to screen a substance to regulate the interaction between p30 and Rap1 such as a compound to inhibit the binding between p30 and Rap1 as well as to develop reagents used for such a purpose. Further, the p30-Rap1 binding regulatory agent such as an identified inhibitor can be expected to serve as a medicine and used as an antiinflammatory drug, immunosuppressive drug, immunosuppressive drug of transplantation, anticancer drug, and the like. Furthermore, modified p30-related peptides and nucleic acids, anti-p30 antibodies, and the like including, for example, those capable of functioning in a dominantly negative fashion or exerting p30-Rap1 binding inhibition in cells are highly useful. Reagents for analysis of biofunction and its assay methods can also be developed by utilizing the present technology and knowledge.

It is apparent that the present invention can be applied to things other than what has been particularly described in the above explanations and examples. In view of the above teaching, various alterations and modifications of the present invention are possible, and thus those are also included within the scope of the invention in the appended claims.

### [Sequence Listing Free Text]

SEQ ID NO: 1, Human Rap1
SEQ ID NO: 3, Human RAPL (or Human p30)
SEQ ID NO: 5, Dominant-Negative Human RAPL
SEQ ID NO: 7, Nucleotide Sequence for Myc-tag
SEQ ID NO: 8, Peptide Sequence for Myc-tag
SEQ ID NO: 9, House Mouse RAPL (Region 104 to 901 of mRNA)
SEQ ID NO: 11, Description of Artificial Sequence: Oligonucleotide to act as a primer for PCR
SEQ ID NO: 12, Description of Artificial Sequence: Oligonucleotide to act as a primer for PCR
SEQ ID NO: 13, Description of Artificial Sequence: Oligonucleotide to act as a primer for PCR
SEQ ID NO: 14, Description of Artificial Sequence: Oligonucleotide to act as a primer for PCR

## Claims

1. A screening method for a compound, or a salt thereof, that promotes or inhibits the interaction between Rap1 and p30 and/or the binding of Rap1 with p30, which comprises:
(1) a process to allow
(a) a polypeptide selected from the group consisting of an active-form polypeptide containing an amino acid sequence identical or essentially identical to the amino acid sequence of SEQ ID NO:2, an active-form polypeptide containing a point-mutated SEQ ID NO:2 amino acid sequence wherein the 12th glycine thereof is replaced with valine or an amino acid sequence essentially identical to said point-mutated amino acid sequence, a partial peptide thereof, and a salt thereof;
(b) a polypeptide selected from the group consisting of a polypeptide containing an amino acid sequence identical or essentially identical to the amino acid sequence of SEQ ID NO:4, a partial peptide thereof, and a salt thereof; and
(c) a test sample to come in contact with one another; and
(2) a process to detect the interaction and/or binding between the polypeptide selected from the group (a) and the polypeptide selected from the group (b).

2. The screening method according to claim 1, comprising:
(1) the process to allow a polypeptide selected from the group (a), a polypeptide selected from the group (b), and a test sample to come in contact with one another;
(2) the process to detect the occurrence of the interaction and/or binding between the polypeptide selected from the group (a) and the polypeptide selected from the group (b); and
(3) a process to select a compound that promotes and/or inhibits the interaction and/or inding between these polypeptides.

3. The screening method according to claim 1 or 2, wherein another peptide is fused to a polypeptide selected from the group (a) and/or a polypeptide selected from the group (b).

4. The screening method according to any one of claims 1 to 3, wherein the polypeptide selected from the group (a) and/or the polypeptide selected from the group (b) is labeled and the label is detected or measured to detect the binding and/or interaction of the polypeptides.

5. The screening method according to any one of claims 1 to 3, wherein the polypeptide of the group (b) bound to the polypeptide of the group (a) is assayed with a primary antibody against the polypeptide of the group (b) or a primary antibody against another peptide fused to the polypeptide of the group (b) to detect the binding and/or interaction between the polypeptide selected from the group (a) and the polypeptide selected from the group (b).

6. The screening method according to any one of claims 1 to 3, wherein the polypeptide selected from the group (a) bound to the polypeptide selected from the group (b) is assayed with a primary antibody against the polypeptide of the group (a) or a primary antibody against another peptide fused to the polypeptide of the group (a) to detect the binding and/or interaction between the polypeptide selected from the group (a) and the polypeptide selected from the group (b).

7. The screening method according to any one of claims 1 to 3, wherein the polypeptide selected from the group (b) bound to the polypeptide selected from the group (a) is assayed with a primary antibody against the polypeptide of the group (b) or a primary antibody against another peptide fused to the polypeptide of the group (b) and a secondary antibody against the primary antibody to detect the binding and/or interaction between the polypeptide selected from the group (a) and the polypeptide selected from the group (b).

8. The screening method according to any one of claims 1 to 3, wherein the polypeptide of the group (a) is an active-form fusion polypeptide, or a salt thereof, wherein glutathione-S-transferase is fused with the N-terminal side of a polypeptide having the amino acid sequence of SEQ ID NO:2 or an active fusion polypeptide, or a salt thereof, wherein glutathione-S-transferase is fused with the N-terminal side of a polypeptide having a point-mutated SEQ ID NO:2 amino acid sequence in which the 12th glycine thereof is replaced with valine; and the polypeptide of the group (b) is a polypeptide, or a salt thereof, wherein an Myc epitope is fused with the N-terminal side of a polypeptide having the amino acid sequence of SEQ ID NO:4.

9. A screening kit for a compound, or a salt thereof, which promotes or inhibits the interaction and/or binding between Rap1 and p30 which comprises an effective amount of
(a) a polypeptide selected from the group consisting of a polypeptide containing an amino acid sequence identical or essentially identical to the amino acid sequence of SEQ ID NO:2, and a polypeptide containing a point-mutated SEQ ID NO:2 amino acid sequence in which the 12th glycine thereof is replaced with valine, or an amino acid sequence essentially identical to said point-mutated amino acid sequence, a partial peptide thereof and a salt thereof; and
(b) a polypeptide selected from the group consisting of a polypeptide containing an amino acid sequence identical or essentially identical to the amino acid sequence of SEQ ID NO:4, a partial peptide thereof, and a salt thereof.

10. The screening kit according to claim 9, wherein another peptide is fused to the polypeptide selected from the group (a) and/or the polypeptide selected from the group (b).

11. The screening kit according to claim 9, wherein the polypeptide selected from the group (a) and/or the polypeptide selected from the group (b) is labeled.

12. The screening kit according to claim 9, wherein the polypeptide of the group (a) is a fusion polypeptide, or a salt thereof, wherein glutathione-S-transferase is fused with the N-terminal side of a polypeptide having the amino acid sequence of SEQ ID NO:2 or a fusion polypeptide, or a salt thereof, wherein glutathione-S-transferase is fused with the N-terminal side of a polypeptide having a point-mutated SEQ ID NO:2 amino acid sequence in which the 12th glycine thereof is replaced with valine; and the polypeptide of the group (b) is a fusion polypeptide, or a salt thereof, wherein an Myc epitope is fused with the N-terminal side of a polypeptide having the amino acid sequence of SEQ ID NO:4.

13. A compound, or a salt thereof, wich promotes or inhibits the interaction and/or binding between Rap1 and p30 and is obtained using the screening method according to claim 1 or the screening kit according to claim 9.

14. The compound, or a salt thereof, according to claim 13 which inhibits the interaction and/or binding between Rap1 and p30.

15. A pharmaceutical composition containing the compound or the salt thereof according to claim 13.

16. A pharmaceutical composition comprising an effective amount of the compound, or a salt thereof, according to claim 14.

17. The pharmaceutical composition according to claim 15 or 16, wherein a target to be treated or prevented is selected from the group consisting of:
(a) inflammatory diseases;
(b) immune diseases;
(c) graft versus host reaction upon organ transplantation; and
(d) cancers.

18. A monoclonal antibody that recognizes a polypeptide containing an amino acid sequence identical or essentially identical to the amino acid sequence of SEQ ID NO:4.

19. A diagnostic method which comprises using the monoclonal antibody according to claim 18.

20. A diagnostic kit which comprises an effective amount of the monoclonal antibody according to claim 18.

21. A polypeptide, or a salt thereof, that functions intracellularly against a polypeptide containing an amino acid sequence identical or essentially identical to the amino acid sequence of SEQ ID NO:4 in a dominantly negative fashion.

22. A composition comprising an effective amount of the polypeptide, or a salt thereof, according to claim 21 for treatment or prevention of a disease selected from the group consisting of:
(a) inflammatory diseases;
(b) immune diseases;
(c) graft versus host reaction on organ transplantation; and
(d) cancers.

23. A polynucleotide encoding the polypeptide according to claim 21.

24. A composition comprising an effective amount of the polynucleotide according to claim 23 for treatment or prevention of a disease selected from the group consisting of:
(a) inflammatory diseases;
(b) immune diseases;
(c) graft versus host reaction upon organ transplantation; and
(d) cancers.

25. A transgenic animal having a regulated expression of a polypeptide containing an amino acid sequence identical or essentially identical to the amino acid sequence of SEQ ID NO:10.

26. The transgenic animal according to claim 25, wherein a polypeptide containing an amino acid sequence identical or essentially identical to the amino acid sequence of SEQ ID NO:10 is overexpressed.

27. The transgenic animal according to claim 25 or 26, which is a mouse.

28. A Rap1-p30 binding inhibitor which comprises an effective amount of a compound, or a salt thereof, of the formula (I): 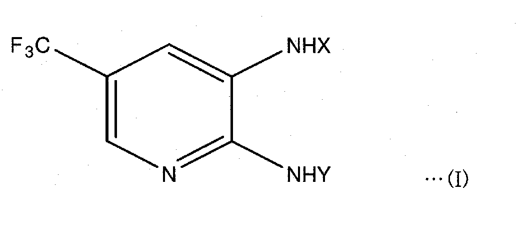 wherein X is a group: -CW¹R¹ or -C(=W¹)W²R²
in which
R¹ is alkyl, haloalkyl, alkoxycarbonylalkyl, alkenyl, haloalkenyl, alkenyl substituted with thienyl, cycloalkyl, cycloalkyl substituted with a halogen atom, phenyl, phenyl substituted with a halogen atom, phenyl substituted with alkyl or haloalkyl, phenyl substituted with alkoxy or haloalkoxy, tetrahydronaphthyl, indanyl, furanyl, or thienyl,
R² is alkyl or haloalkyl, and
W¹ and W² each independently represents an oxygen or sulfur atom, and
Y is -SO₂R⁹
in which
R⁹ is alkyl, haloalkyl, phenyl, phenyl substituted with a halogen atom, phenyl substituted with alkyl or haloalkyl, or phenyl substituted with alkoxy or haloalkoxy].

29. The Rap1-p30 binding inhibitor according to claim 28, wherein X is alkoxycarbonylalkylcarbonyl, alkenylcarbonyl, alkenylcarbonyl substituted with thienyl, cycloalkylcarbonyl, indanylcarbonyl, furancarbonyl, thiophenecarbonyl, tetrahydronaphthylcarbonyl, or benzoyl unsubstituted or optionally substituted with a halogen atom or haloalkyl, and Y is alkylsulfonyl.

30. The Rap1 and p30 binding inhibitor according to claim 28, wherein X is cycloalkylcarbonyl, furancarbonyl or benzoyl unsubstituted or optionally substituted with halogen, and Y is alkylsulfonyl.

31. The Rap1-p30 binding inhibitor according to claim 28, wherein the compound is selected from the group consisting of
N-(2-ethylsulfonylamino-5-trifluoromethyl-3-pyridyl)cyclohexanecarboxamide,
N-(2-methylsulfonylamino-5-trifluoromethyl-3-pyridyl)-4-fluorobenzamide,
N-(2-isopropylsulfonylamino-5-trifluoromethyl-3-pyridyl)-3-fluorobenzamide,
N-(2-methylsulfonylamino-5-trifluoromethyl-3-pyridyl)-2-furancarboxamide, and
N-(2-isopropylsulfonylamino-5-trifluoromethyl-3-pyridyl)cyclopentanecarboxamide.

32. A Rap1-p30 binding inhibitor comprising an effective amount of N-(2-ethylsulfonylamino-5-trifluoromethyl-3-pyridyl)cyclohexanecarboxamide or a salt thereof.


SEQUENCE LISTING

<110>  Ishihara Sangyo Kaisha, Ltd.

<120>  Regulation of RAPL-Rap1 Interaction

<130>  IS-08PCT

<150>  JP 2002-316892
<151>  2002-10-30

<160>  14

<170>  PatentIn version 3.1

<210>  1
<211>  555
<212>  DNA
<213>  Homo sapiens

<220>
<221>  CDS
<222>  (1)..(555)
<223>  Human Rap1

<400>  1
atg cgt gag tac aag cta gtg gtc ctt ggt tca gga ggc gtt ggg aag      48
Met Arg Glu Tyr Lys Leu Val Val Leu Gly Ser Gly Gly Val Gly Lys
1               5                   10                  15

tct gct ctg aca gtt cag ttt gtt cag gga att ttt gtt gaa aaa tat      96
Ser Ala Leu Thr Val Gln Phe Val Gln Gly Ile Phe Val Glu Lys Tyr
            20                  25                  30

gac cca acg ata gaa gat tcc tac aga aag caa gtt gaa gtc gat tgc      144
Asp Pro Thr Ile Glu Asp Ser Tyr Arg Lys Gln Val Glu Val Asp Cys
        35                  40                  45

caa cag tgt atg ctc gaa atc ctg gat act gca ggg aca gag caa ttt      192
Gln Gln Cys Met Leu Glu Ile Leu Asp Thr Ala Gly Thr Glu Gln Phe
    50                  55                  60

aca gca atg agg gat ttg tat atg aag aac ggc caa ggt ttt gca cta      240
Thr Ala Met Arg Asp Leu Tyr Met Lys Asn Gly Gln Gly Phe Ala Leu
65                  70                  75                  80

gta tat tct att aca gct cag tcc acg ttt aac gac tta cag gac ctg      288
Val Tyr Ser Ile Thr Ala Gln Ser Thr Phe Asn Asp Leu Gln Asp Leu
                85                  90                  95

agg gaa cag att tta cgg gtt aag gac acg gaa gat gtt cca atg att      336
Arg Glu Gln Ile Leu Arg Val Lys Asp Thr Glu Asp Val Pro Met Ile
            100                 105                 110

ttg gtt ggc aat aaa tgt gac ctg gaa gat gag cga gta gtt ggc aaa      384
Leu Val Gly Asn Lys Cys Asp Leu Glu Asp Glu Arg Val Val Gly Lys
        115                 120                 125

gag cag ggc cag aat tta gca aga cag tgg tgt aac tgt gcc ttt tta      432
Glu Gln Gly Gln Asn Leu Ala Arg Gln Trp Cys Asn Cys Ala Phe Leu
    130                 135                 140

gaa tct tct gca aag tca aag atc aat gtt aat gag ata ttt tat gac       480
Glu Ser Ser Ala Lys Ser Lys Ile Asn Val Asn Glu Ile Phe Tyr Asp
145                 150                 155                 160

ctg gtc aga cag ata aat agg aaa aca cca gtg gaa aag aag aag cct       528
Leu Val Arg Gln Ile Asn Arg Lys Thr Pro Val Glu Lys Lys Lys Pro
                165                 170                 175

aaa aag aaa tca tgt ctg ctg ctc tag                                   555
Lys Lys Lys Ser Cys Leu Leu Leu
            180

<210>  2
<211>  184
<212>  PRT
<213>  Homo sapiens

<400>  2

Met Arg Glu Tyr Lys Leu Val Val Leu Gly Ser Gly Gly Val Gly Lys
1               5                   10                  15

Ser Ala Leu Thr Val Gln Phe Val Gln Gly Ile Phe Val Glu Lys Tyr
            20                  25                  30

Asp Pro Thr Ile Glu Asp Ser Tyr Arg Lys Gln Val Glu Val Asp Cys
        35                  40                  45

Gln Gln Cys Met Leu Glu Ile Leu Asp Thr Ala Gly Thr Glu Gln Phe
    50                  55                  60

Thr Ala Met Arg Asp Leu Tyr Met Lys Asn Gly Gln Gly Phe Ala Leu
65                  70                  75                  80

Val Tyr Ser Ile Thr Ala Gln Ser Thr Phe Asn Asp Leu Gln Asp Leu
                85                  90                  95

Arg Glu Gln Ile Leu Arg Val Lys Asp Thr Glu Asp Val Pro Met Ile
            100                 105                 110

Leu Val Gly Asn Lys Cys Asp Leu Glu Asp Glu Arg Val Val Gly Lys
        115                 120                 125

Glu Gln Gly Gln Asn Leu Ala Arg Gln Trp Cys Asn Cys Ala Phe Leu
    130                 135                 140

Glu Ser Ser Ala Lys Ser Lys Ile Asn Val Asn Glu Ile Phe Tyr Asp
145                 150                 155                 160

Leu Val Arg Gln Ile Asn Arg Lys Thr Pro Val Glu Lys Lys Lys Pro
                165                 170                 175

Lys Lys Lys Ser Cys Leu Leu Leu
            180

<210>  3
<211>  798
<212>  DNA
<213>  Homo sapiens

<220>
<221>  CDS
<222>  (1)..(798)
<223>  Human RAPL (or Human p30)

<400>  3
atg acc gtg gac agc agc atg agc agt ggg tac tgc agc ctg gac gag        48
Met Thr Val Asp Ser Ser Met Ser Ser Gly Tyr Cys Ser Leu Asp Glu
1               5                   10                  15

gaa ctg gaa gac tgc ttc ttc act gct aag act acc ttt ttc aga aat        96
Glu Leu Glu Asp Cys Phe Phe Thr Ala Lys Thr Thr Phe Phe Arg Asn
            20                  25                  30

gcg cag agc aaa cat ctt tca aag aat gtc tgt aaa cct gtg gag gaa       144
Ala Gln Ser Lys His Leu Ser Lys Asn Val Cys Lys Pro Val Glu Glu
        35                  40                  45

aca cag cgc ccg ccc aca ctg cag gag atc aag cag aag atc gac agc       192
Thr Gln Arg Pro Pro Thr Leu Gln Glu Ile Lys Gln Lys Ile Asp Ser
    50                  55                  60

tac aac acg cga gag aag aac tgc ctg ggc atg aaa ctg agt gaa gac       240
Tyr Asn Thr Arg Glu Lys Asn Cys Leu Gly Met Lys Leu Ser Glu Asp
65                  70                  75                  80

ggc acc tac acg ggt ttc atc aaa gtg cat ctg aaa ctc cgg cgg cct       288
Gly Thr Tyr Thr Gly Phe Ile Lys Val His Leu Lys Leu Arg Arg Pro
                85                  90                  95

gtg acg gtg cct gct ggg atc cgg ccc cag tcc atc tat gat gcc atc       336
Val Thr Val Pro Ala Gly Ile Arg Pro Gln Ser Ile Tyr Asp Ala Ile
            100                 105                 110

aag gag gtg aac ctg gcg gct acc acg gac aag cgg aca tcc ttc tac       384
Lys Glu Val Asn Leu Ala Ala Thr Thr Asp Lys Arg Thr Ser Phe Tyr
        115                 120                 125

ctg ccc cta gat gcc atc aag cag ctg cac atc agc agc acc acc acc       432
Leu Pro Leu Asp Ala Ile Lys Gln Leu His Ile Ser Ser Thr Thr Thr
    130                 135                 140

gtc agt gag gtc atc cag ggg ctg ctc aag aag ttc atg gtt gtg gac       480
Val Ser Glu Val Ile Gln Gly Leu Leu Lys Lys Phe Met Val Val Asp
145                 150                 155                 160

aat ccc cag aag ttt gca ctt ttt aag cgg ata cac aag gac gga caa       528
Asn Pro Gln Lys Phe Ala Leu Phe Lys Arg Ile His Lys Asp Gly Gln
                165                 170                 175

gtg ctc ttc cag aaa ctc tcc att gct gac cgc ccc ctc tac ctg cgc       576
Val Leu Phe Gln Lys Leu Ser Ile Ala Asp Arg Pro Leu Tyr Leu Arg

180                 185                 190

ctg ctt gct ggg cct gac acg gag gtc ctc agc ttt gtg cta aag gag        624
Leu Leu Ala Gly Pro Asp Thr Glu Val Leu Ser Phe Val Leu Lys Glu
        195                 200                 205

aat gaa act gga gag gta gag tgg gat gcc ttc tcc atc cct gaa ctt        672
Asn Glu Thr Gly Glu Val Glu Trp Asp Ala Phe Ser Ile Pro Glu Leu
    210                 215                 220

cag aac ttc cta aca atc ctg gaa aaa gag gag cag gac aaa atc caa        720
Gln Asn Phe Leu Thr Ile Leu Glu Lys Glu Glu Gln Asp Lys Ile Gln
225                 230                 235                 240

caa gtg caa aag aag tat gac aag ttt agg cag aaa ctg gag gag gcc        768
Gln Val Gln Lys Lys Tyr Asp Lys Phe Arg Gln Lys Leu Glu Glu Ala
                245                 250                 255

tta aga gaa tcc cag ggc aaa cct ggg taa                                798
Leu Arg Glu Ser Gln Gly Lys Pro Gly
            260                 265

<210>  4
<211>  265
<212>  PRT
<213>  Homo sapiens

<400>  4

Met Thr Val Asp Ser Ser Met Ser Ser Gly Tyr Cys Ser Leu Asp Glu
1               5                   10                  15

Glu Leu Glu Asp Cys Phe Phe Thr Ala Lys Thr Thr Phe Phe Arg Asn
            20                  25                  30

Ala Gln Ser Lys His Leu Ser Lys Asn Val Cys Lys Pro Val Glu Glu
        35                  40                  45

Thr Gln Arg Pro Pro Thr Leu Gln Glu Ile Lys Gln Lys Ile Asp Ser
    50                  55                  60

Tyr Asn Thr Arg Glu Lys Asn Cys Leu Gly Met Lys Leu Ser Glu Asp
65                  70                  75                  80

Gly Thr Tyr Thr Gly Phe Ile Lys Val His Leu Lys Leu Arg Arg Pro
                85                  90                  95

Val Thr Val Pro Ala Gly Ile Arg Pro Gln Ser Ile Tyr Asp Ala Ile
            100                 105                 110

Lys Glu Val Asn Leu Ala Ala Thr Thr Asp Lys Arg Thr Ser Phe Tyr
        115                 120                 125

Leu Pro Leu Asp Ala Ile Lys Gln Leu His Ile Ser Ser Thr Thr Thr

130                 135                 140

Val Ser Glu Val Ile Gln Gly Leu Leu Lys Lys Phe Met Val Val Asp
145                 150                 155                 160

Asn Pro Gln Lys Phe Ala Leu Phe Lys Arg Ile His Lys Asp Gly Gln
                165                 170                 175

Val Leu Phe Gln Lys Leu Ser Ile Ala Asp Arg Pro Leu Tyr Leu Arg
            180                 185                 190

Leu Leu Ala Gly Pro Asp Thr Glu Val Leu Ser Phe Val Leu Lys Glu
        195                 200                 205

Asn Glu Thr Gly Glu Val Glu Trp Asp Ala Phe Ser Ile Pro Glu Leu
    210                 215                 220

Gln Asn Phe Leu Thr Ile Leu Glu Lys Glu Glu Gln Asp Lys Ile Gln
225                 230                 235                 240

Gln Val Gln Lys Lys Tyr Asp Lys Phe Arg Gln Lys Leu Glu Glu Ala
                245                 250                 255

Leu Arg Glu Ser Gln Gly Lys Pro Gly
            260                 265

<210>  5
<211>  498
<212>  DNA
<213>  Homo sapiens

<220>
<221>  CDS
<222>  (1)..(498)
<223>  Dominant-Negative Human RAPL

<400>  5
gct ggg atc cgg ccc cag tcc atc tat gat gcc atc aag gag gtg aac       48
Ala Gly Ile Arg Pro Gln Ser Ile Tyr Asp Ala Ile Lys Glu Val Asn
1               5                   10                  15

ctg gcg gct acc acg gac aag cgg aca tcc ttc tac ctg ccc cta gat       96
Leu Ala Ala Thr Thr Asp Lys Arg Thr Ser Phe Tyr Leu Pro Leu Asp
            20                  25                  30

gcc atc aag cag ctg cac atc agc agc acc acc acc gtc agt gag gtc      144
Ala Ile Lys Gln Leu His Ile Ser Ser Thr Thr Thr Val Ser Glu Val
        35                  40                  45

atc cag ggg ctg ctc aag aag ttc atg gtt gtg gac aat ccc cag aag      192
Ile Gln Gly Leu Leu Lys Lys Phe Met Val Val Asp Asn Pro Gln Lys
    50                  55                  60

ttt gca ctt ttt aag cgg ata cac aag gac gga caa gtg ctc ttc cag 240
Phe Ala Leu Phe Lys Arg Ile His Lys Asp Gly Gln Val Leu Phe Gln
65                  70                  75                  80

aaa ctc tcc att gct gac cgc ccc ctc tac ctg cgc ctg ctt gct ggg 288
Lys Leu Ser Ile Ala Asp Arg Pro Leu Tyr Leu Arg Leu Leu Ala Gly
                85                  90                  95

cct gac acg gag gtc ctc agc ttt gtg cta aag gag aat gaa act gga 336
Pro Asp Thr Glu Val Leu Ser Phe Val Leu Lys Glu Asn Glu Thr Gly
            100                 105                 110

gag gta gag tgg gat gcc ttc tcc atc cct gaa ctt cag aac ttc cta 384
Glu Val Glu Trp Asp Ala Phe Ser Ile Pro Glu Leu Gln Asn Phe Leu
        115                 120                 125

aca atc ctg gaa aaa gag gag cag gac aaa atc caa caa gtg caa aag 432
Thr Ile Leu Glu Lys Glu Glu Gln Asp Lys Ile Gln Gln Val Gln Lys
    130                 135                 140

aag tat gac aag ttt agg cag aaa ctg gag gag gcc tta aga gaa tcc 480
Lys Tyr Asp Lys Phe Arg Gln Lys Leu Glu Glu Ala Leu Arg Glu Ser
145                 150                 155                 160

cag ggc aaa cct ggg taa 498
Gln Gly Lys Pro Gly
                165

<210> 6
<211> 165
<212> PRT
<213> Homo sapiens

<400> 6

Ala Gly Ile Arg Pro Gln Ser Ile Tyr Asp Ala Ile Lys Glu Val Asn
1               5                   10                  15

Leu Ala Ala Thr Thr Asp Lys Arg Thr Ser Phe Tyr Leu Pro Leu Asp
            20                  25                  30

Ala Ile Lys Gln Leu His Ile Ser Ser Thr Thr Thr Val Ser Glu Val
        35                  40                  45

Ile Gln Gly Leu Leu Lys Lys Phe Met Val Val Asp Asn Pro Gln Lys
    50                  55                  60

Phe Ala Leu Phe Lys Arg Ile His Lys Asp Gly Gln Val Leu Phe Gln
65                  70                  75                  80

Lys Leu Ser Ile Ala Asp Arg Pro Leu Tyr Leu Arg Leu Leu Ala Gly
                85                  90                  95

Pro Asp Thr Glu Val Leu Ser Phe Val Leu Lys Glu Asn Glu Thr Gly
            100                 105                 110

Glu Val Glu Trp Asp Ala Phe Ser Ile Pro Glu Leu Gln Asn Phe Leu
        115                 120                 125

Thr Ile Leu Glu Lys Glu Glu Gln Asp Lys Ile Gln Gln Val Gln Lys
    130                 135                 140

Lys Tyr Asp Lys Phe Arg Gln Lys Leu Glu Glu Ala Leu Arg Glu Ser
145                 150                 155                 160

Gln Gly Lys Pro Gly
                165

<210>  7
<211>  33
<212>  DNA
<213>  Artificial Sequence

<220>
<223>  Nucleotide Sequence for Myc-tag

<400>  7
atggaacaga aactcatatc ggaggaggat cta                                    33

<210>  8
<211>  11
<212>  PRT
<213>  Artificial Sequence

<220>
<223>  Peptide Sequence for Myc-tag

<400>  8

Met Glu Gln Lys Leu Ile Ser Glu Glu Asp Leu
1               5                   10

<210>  9
<211>  798
<212>  DNA
<213>  Mus musculus

<220>
<221>  CDS
<222>  (1)..(798)
<223>  House Mouse RAPL (Region 104 to 901 of mRNA)

<400>  9
atg acc gtg gac agc agc atg agc agc ggg tac tgc agc ctg gac gag        48
Met Thr Val Asp Ser Ser Met Ser Ser Gly Tyr Cys Ser Leu Asp Glu
1               5                   10                  15

gaa ctg gaa gat tgc ttc ttt acg gct aag acc acc ttc ttc agg aat        96
Glu Leu Glu Asp Cys Phe Phe Thr Ala Lys Thr Thr Phe Phe Arg Asn
            20                  25                  30

ctt cag agc aaa cag cct tca aag aat gtc tgt aag gca gtg gag gag 144
Leu Gln Ser Lys Gln Pro Ser Lys Asn Val Cys Lys Ala Val Glu Glu
        35                  40                  45

aca cag cac ccg ccc acg ata cag gag atc aag cag aag att gac agc 192
Thr Gln His Pro Pro Thr Ile Gln Glu Ile Lys Gln Lys Ile Asp Ser
    50                  55                  60

tat aac agc agg gag aag cac tgc ctg ggc atg aag ctg agt gaa gat 240
Tyr Asn Ser Arg Glu Lys His Cys Leu Gly Met Lys Leu Ser Glu Asp
65                  70                  75                  80

ggc acc tac aca ggt ttc atc aaa gtg cat ttg aag ctc cga cgg cca 288
Gly Thr Tyr Thr Gly Phe Ile Lys Val His Leu Lys Leu Arg Arg Pro
                85                  90                  95

gtg acg gtg ccc gct ggg atc cgg ccc cag tcc atc tat gat gcc att 336
Val Thr Val Pro Ala Gly Ile Arg Pro Gln Ser Ile Tyr Asp Ala Ile
            100                 105                 110

aag gaa gtg aac cct gca gcc acc aca gac aag cgg act tcc ttc tac 384
Lys Glu Val Asn Pro Ala Ala Thr Thr Asp Lys Arg Thr Ser Phe Tyr
        115                 120                 125

ctg cca ctc gat gcc atc aag cag cta cat atc agc agc acc acc acg 432
Leu Pro Leu Asp Ala Ile Lys Gln Leu His Ile Ser Ser Thr Thr Thr
    130                 135                 140

gtt agt gag gtc atc cag ggg ctg ctc aag aag ttc atg gtt gtg gac 480
Val Ser Glu Val Ile Gln Gly Leu Leu Lys Lys Phe Met Val Val Asp
145                 150                 155                 160

aac cca cag aag ttt gca ctt ttt aag cgg ata cac aaa gat gga caa 528
Asn Pro Gln Lys Phe Ala Leu Phe Lys Arg Ile His Lys Asp Gly Gln
                165                 170                 175

gtg ctc ttc cag aaa ctc tcc att gct gac tat cct ctc tac ctt cgt 576
Val Leu Phe Gln Lys Leu Ser Ile Ala Asp Tyr Pro Leu Tyr Leu Arg
            180                 185                 190

ctg ctc gct ggg cct gac acc gat gtt ctc agc ttt gtg cta aag gag 624
Leu Leu Ala Gly Pro Asp Thr Asp Val Leu Ser Phe Val Leu Lys Glu
        195                 200                 205

aat gaa act gga gag gtg gag tgg gat gcc ttt tcc att cct gaa ctc 672
Asn Glu Thr Gly Glu Val Glu Trp Asp Ala Phe Ser Ile Pro Glu Leu
    210                 215                 220

cag aac ttt tta act atc ctg gaa aaa gag gag cag gac aag atc cat 720
Gln Asn Phe Leu Thr Ile Leu Glu Lys Glu Glu Gln Asp Lys Ile His
225                 230                 235                 240

caa ctg caa aag aag tac aac aaa ttc cgt cag aaa ctg gaa gag gca 768
Gln Leu Gln Lys Lys Tyr Asn Lys Phe Arg Gln Lys Leu Glu Glu Ala
                245                 250                 255

tta cga gag tcc caa ggg aag ccg ggg taa 798
Leu Arg Glu Ser Gln Gly Lys Pro Gly
            260                 265

<210> 10
<211> 265

<212>  PRT
<213>  Mus musculus

<400>  10

Met Thr Val Asp Ser Ser Met Ser Ser Gly Tyr Cys Ser Leu Asp Glu
1               5                   10                  15

Glu Leu Glu Asp Cys Phe Phe Thr Ala Lys Thr Thr Phe Phe Arg Asn
            20                  25                  30

Leu Gln Ser Lys Gln Pro Ser Lys Asn Val Cys Lys Ala Val Glu Glu
        35                  40                  45

Thr Gln His Pro Pro Thr Ile Gln Glu Ile Lys Gln Lys Ile Asp Ser
    50                  55                  60

Tyr Asn Ser Arg Glu Lys His Cys Leu Gly Met Lys Leu Ser Glu Asp
65                  70                  75                  80

Gly Thr Tyr Thr Gly Phe Ile Lys Val His Leu Lys Leu Arg Arg Pro
                85                  90                  95

Val Thr Val Pro Ala Gly Ile Arg Pro Gln Ser Ile Tyr Asp Ala Ile
            100                 105                 110

Lys Glu Val Asn Pro Ala Ala Thr Thr Asp Lys Arg Thr Ser Phe Tyr
        115                 120                 125

Leu Pro Leu Asp Ala Ile Lys Gln Leu His Ile Ser Ser Thr Thr Thr
    130                 135                 140

Val Ser Glu Val Ile Gln Gly Leu Leu Lys Lys Phe Met Val Val Asp
145                 150                 155                 160

Asn Pro Gln Lys Phe Ala Leu Phe Lys Arg Ile His Lys Asp Gly Gln
                165                 170                 175

Val Leu Phe Gln Lys Leu Ser Ile Ala Asp Tyr Pro Leu Tyr Leu Arg
            180                 185                 190

Leu Leu Ala Gly Pro Asp Thr Asp Val Leu Ser Phe Val Leu Lys Glu
        195                 200                 205

Asn Glu Thr Gly Glu Val Glu Trp Asp Ala Phe Ser Ile Pro Glu Leu
    210                 215                 220

Gln Asn Phe Leu Thr Ile Leu Glu Lys Glu Glu Gln Asp Lys Ile His
225                 230                 235                 240

Gln Leu Gln Lys Lys Tyr Asn Lys Phe Arg Gln Lys Leu Glu Glu Ala
                245                 250                 255

Leu Arg Glu Ser Gln Gly Lys Pro Gly
            260                 265

<210>  11
<211>  30
<212>  DNA
<213>  Artificial Sequence

<220>
<223>  Oligonucleotide to act as a primer for PCR

<400>  11
atgaccgtgg acagcagcat gagcagcggg                                        30

<210>  12
<211>  30
<212>  DNA
<213>  Artificial Sequence

<220>
<223>  Oligonucleotide to act as a primer for PCR

<400>  12
tatttgtgag ccagggcatt ggccacacca                                        30

<210>  13
<211>  20
<212>  DNA
<213>  Artificial Sequence

<220>
<223>  Oligonucleotide to act as a primer for PCR

<400>  13
   atcaattacg gggtcattag                                                    20

<210>  14
<211>  20
<212>  DNA
<213>  Artificial Sequence

<220>
<223>  Oligonucleotide to act as a primer for PCR

<400>  14
tgtactgcca agtaggaaag                                                   20